# EUROPEAN PATENT SPECIFICATION

(11) **EP 3 256 444 B1**
(45) Date of publication and mention of the grant of the patent: **27.03.2019**
(21) Application number: 16704711.7
(22) Date of filing: 11.02.2016
(51) Int. Cl.: C07C 201/12, C07C 227/04, C07C 335/16

(54) **PROCESS AND INTERMEDIATES FOR THE PREPARATION OF NEP INHIBITORS**
VERFAHREN UND ZWISCHENPRODUKTE ZUR HERSTELLUNG VON NEP-INHIBITOREN
PROCÉDÉ ET INTERMÉDIAIRES POUR LA PREPARATION D'INHIBITEURS DE NEP

(30) Priority: 13.02.2015 EP 15155147
(43) Date of publication of application: 20.12.2017
(73) Proprietor: Novartis AG, 4056 Basel (CH)
(72) Inventor: MARTIN, Benjamin, 4002 Basel (CH); MANDRELLI, Francesca, 4002 Basel (CH); VENTURONI, Francesco, 4002 Basel (CH)
(74) Representative: Larbig, Karen Dorothee
(86) International application number: PCT/IB2016/050725
(87) International publication number: WO 2016/128924

(56) References cited:
- WO-A2-2009/090251

## Description

### FIELD OF THE INVENTION

The present invention relates to a new chemical synthesis route and intermediates useful for the preparation of neprilysin (NEP) inhibitors and their prodrugs, in particular for the NEP inhibitor prodrug sacubitril.

### BACKGROUND OF THE INVENTION

The NEP inhibitor prodrug sacubitril (*N*-(3-carboxyl-1-oxopropyl)-(4*S*)-(p-phenylphenylmethyl)-4-amino-(2*R*)-methyl butanoic acid ethyl ester; IUPAC name 4-{[(1*S*,3*R*)-1-([1,1'-biphenyl]-4-ylmethyl)-4-ethoxy-3-methyl-4-oxobutyl]amino}-4-oxobutanoic acid) is represented by the following formula (A)

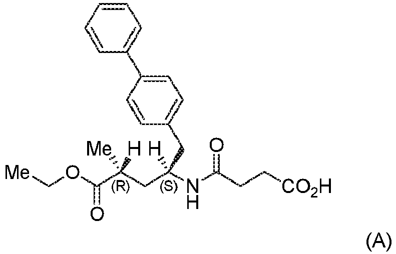

Sacubitril together with valsartan, a known angiotensin receptor blocker (ARB), forms a sodium salt hydrate complex, known as LCZ696, comprising the anionic forms of sacubitril and valsartan, sodium cations and water molecules in the molar ratio of 1:1:3:2.5, respectively (ratio of 6:6:18:15 in the asymmetric unit cell of the solid state crystal), and which is schematically present in formula (B).

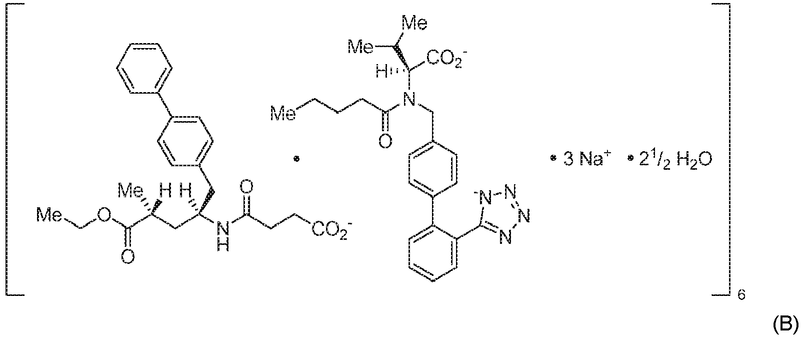

Said complex is also referred to by the following chemical names: Trisodium [3-((1*S*,3*R*)-1-biphenyl-4-ylmethyl-3-ethoxycarbonyl-1-butylcarbamoyl)propionate-(S)-3'-methyl-2'-(pentanoyl{2"-(tetrazol-5-ylate)biphenyl-4'-ylmethyl}amino)butyrate] hemipentahydrate or Octadecasodium hexakis(4-{[(1*S*,3*R*)-1-([1,1'-biphenyl]-4-ylmethyl)-4-ethoxy-3-methyl-4-oxobutyl]amino}-4-oxobutanoate) hexakis(*N*-pentanoyl-*N*-{[2'-(1*H*-tetrazol-1-id-5-yl)[1,1'-biphenyl]-4-yl]methyl)-L-valinate)-water (1/15) (IUPAC nomenclature).

LCZ696 acts as angiotensin receptor neprilysin inhibitor (ARNI) and is therefore useful particularly in the treatment of hypertention or chronic heart failure. Its utility has been confirmed by clinical trials, e.g. in the landmark PARADIGM-HF trial.

Chemical synthesis routes to prepare NEP inhibitors and their prodrugs, in particular sacubitril, and its precursors have been described previously, e.g. in Ksander et al. J. Med. Chem. 1995, 38, pp.1689-1700; in US patent No 5,217,996 and in the international patent applications WO 2008/031567, WO 2008/083967, WO 2009/090251, WO 2010/081410, WO 2011/035569, WO 2011/088797, WO 2012/025501, WO 2012/025502 and WO 2014/032627.

However, there is still a need to design a chemical process for the synthesis of sacubitril which is suitable for industrial scale production under economically and environmentally favorable conditions and provides the drug substance in high chemical purity and with high stereo-chemical selectivity.

### SUMMARY OF THE INVENTION

The invention relates to novel intermediates and process steps and processes for the manufacture of a compound (1), especially (1-a) represented below, and its further use in the manufacture of sacubitril.

In a **first** aspect, the present invention provides the following new compounds:
**A compound of formula (1)**, or a salt thereof 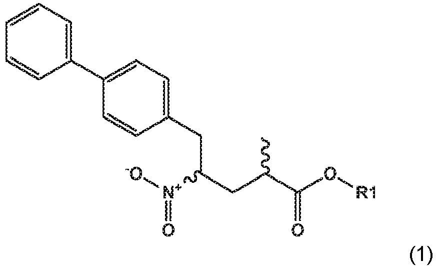 wherein R1 is hydrogen or C₁-C₆-alkyl.
   In one embodiment thereof, the compound of the formula (1) is represented by formula (1-a) with the following stereochemistry 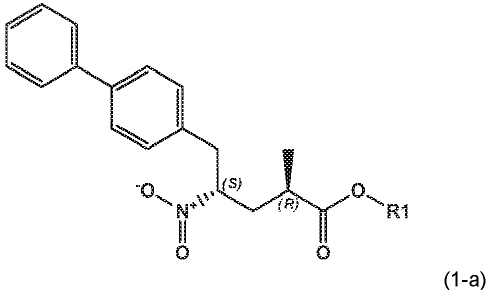 wherein R₁ is hydrogen or C₁-C₆-alkyl, preferably ethyl.
A compound of formula (2) or a salt thereof, 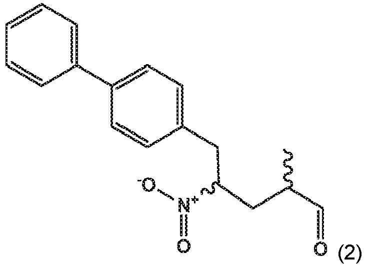 In one embodiment thereof, the compound of the formula (2) is represented by formula (2-a) with the following stereochemistry 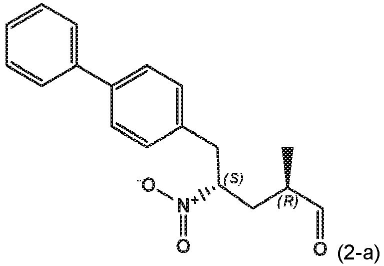
A compound of the formula (7)

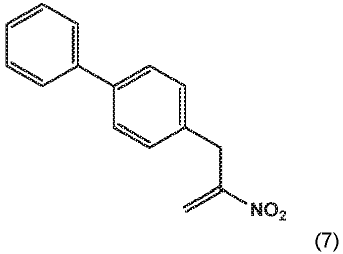

In a **second** aspect, the present invention provides a **new process** for the manufacture of the compound of the formula (8), in particular of formula (8-a), or a salt thereof, as defined herein. This process comprises several steps via novel intermediate compounds and is depicted in the following Schemes 1 to 5, respectively, wherein the process depicted in each SCHEME 1-4 represents a separate embodiment of the invention.

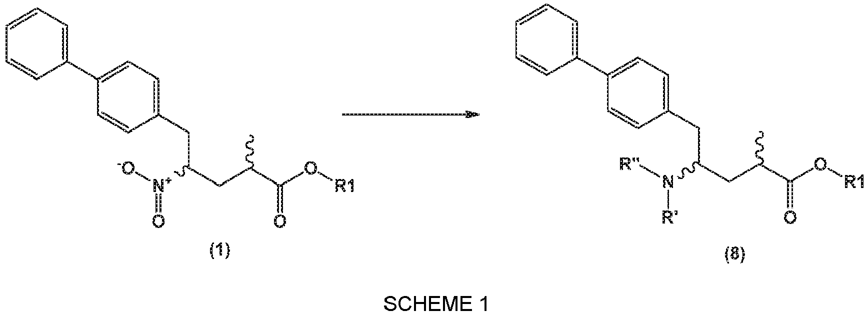

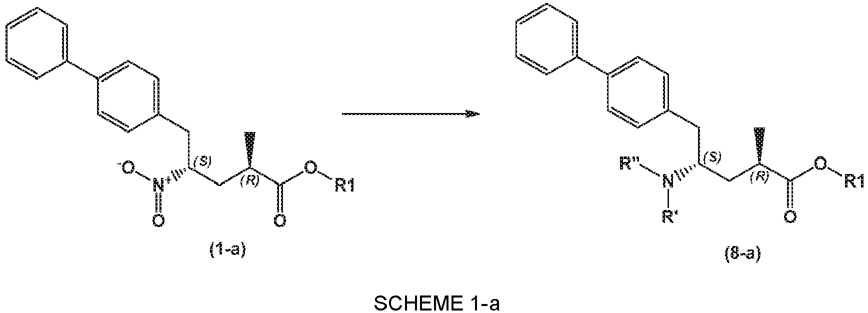

SCHEME 1 and SCHEME 1-a depict the process comprising hydrogenation of the novel intermediate compound of formula (1), preferably of formula (1-a), wherein R1 is hydrogen or C₁-C₆-alkyl, preferably ethyl, into a compound of formula (8), preferably of formula (8-a), wherein R' and R" are independently of each other hydrogen or a nitrogen protecting group, preferably *tert*-butyloxycarbonyl, and R1 is hydrogen or C₁-C₆-alkyl, especially ethyl.

If desired - and not explicitly disclosed in the SCHEME 1 - this can be followed by converting a compound of the formula (8), especially (8-a), wherein each of R' and R" are hydrogen, into a salt, e.g. with an acid. Alternatively, the reduction can take place with parallel introduction of a nitrogen protecting group or the nitrogen protecting group can be added subsequently.

In one embodiment, the compound of formula (1) can be obtained according to the following SCHEMES:

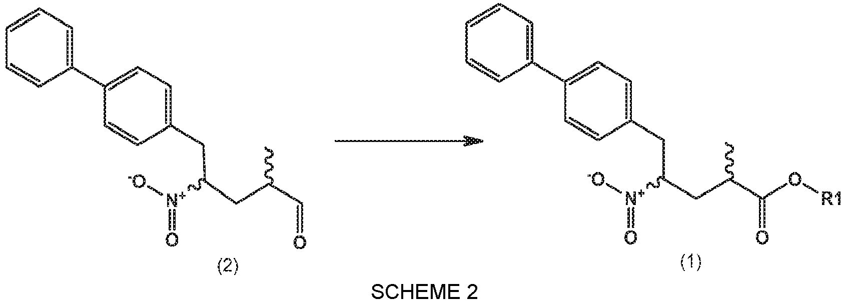

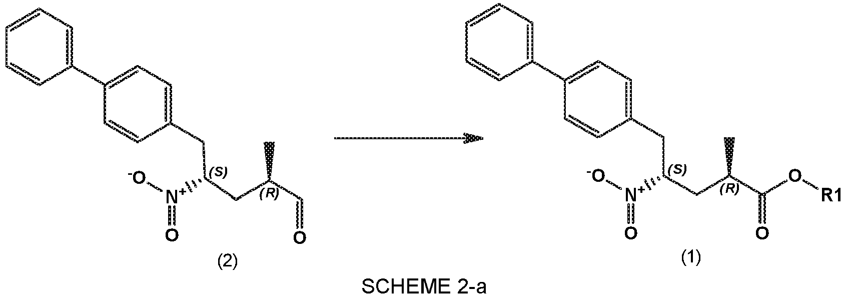

SCHEME 2 and SCHEME 2-a, both depict a process comprising oxidising a compound of the formula (2), preferably of the formula (2-a), preferably under Pinnick oxidation conditions, to obtain a compound of formula (1), preferably of formula (1-a), wherein R1 is hydrogen or C₁-C₆-alkyl, preferably ethyl.

If desired - and not explicitly disclosed in the SCHEME 2 - this can include (i) converting a free compound of the formula (1), preferably of formula (1-a), into its salt, (ii) converting a salt of the compound of the formula (1), preferably of formula (1a), into the free compound; or (iii) converting a salt of a compound of formula (1), preferably of formula (1a), into a different salt thereof; or (simultaneously or separately) esterifiying a compound of formula (1), preferably of formula (1-a), wherein R1 is hydrogen, or a salt or a reactive acid derivative thereof, with a C₁-C₆-aliphatic alcohol, especially ethanol, to yield the compound wherein R1 is C₁-C₆-alkyl, especially ethyl.

In one embodiment, the compound of formula (2) can be obtained according to the following SCHEMES:

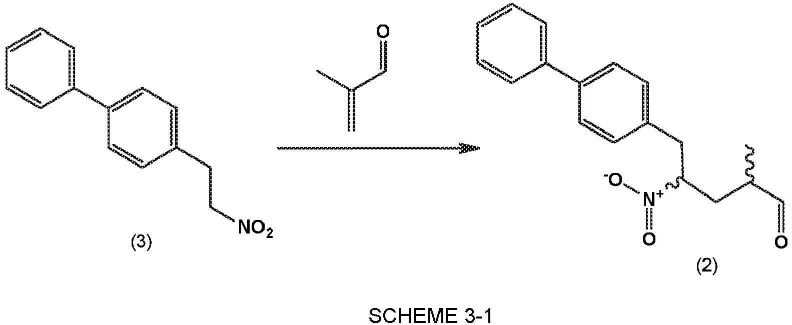

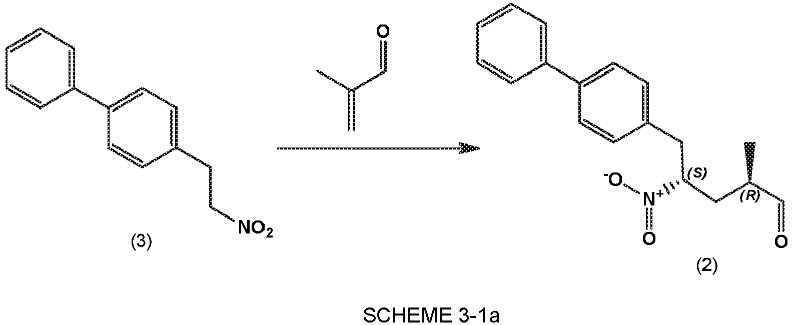

SCHEME 3-1 and SCHEME 3-1a depict a process for the manufacture of a compound of the formula (2), preferably of formula (2-a), said process comprising reacting a compound of formula (3) with methacroleine or a reactive derivative thereof in the presence of an organocatalyst for a Michael reaction.

In an alternative embodiment, the compound of formula (2) can be obtained according to the following SCHEMES:

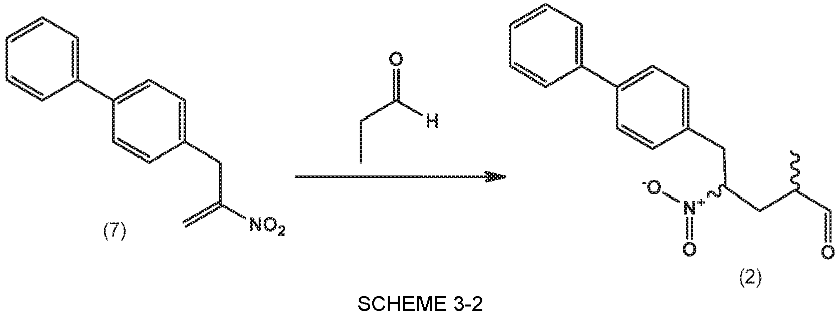

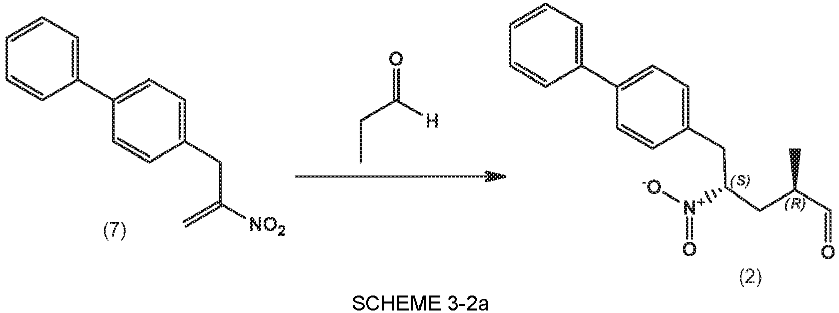

SCHEME 3-2 and SCHEME 3-2a depict a process for the manufacture of a compound of formula (2), preferably of formula (2-a), said process comprising reacting a compound of formula (7) with propionaldehyde or a reactive derivative thereof in the presence of an organocatalyst for Michael reaction.

In a further embodiment, the compound of formula (7) is obtained according to the following SCHEMES:

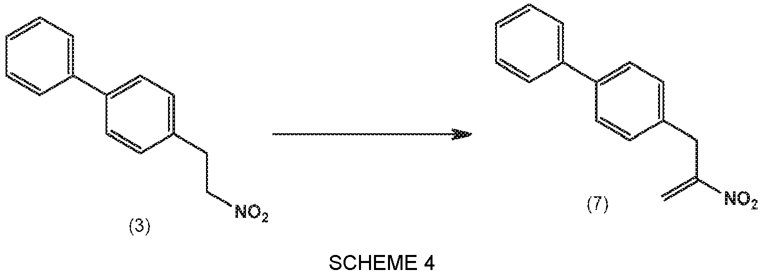

SCHEME 4 depicts a process for the manufacture of a compound of formula (7) comprising reacting a compound of formula (3) with formaldehyde or a reactive derivative thereof.

In one embodiment thereof, the compound of formula (3) is obtained according to the following SCHEME:

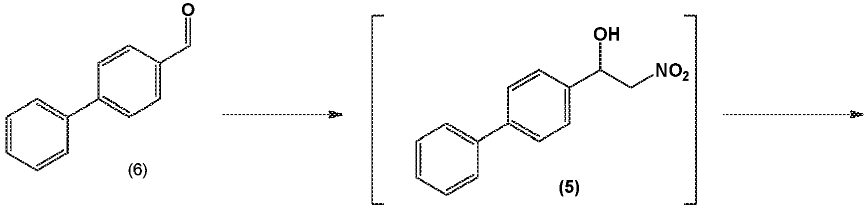

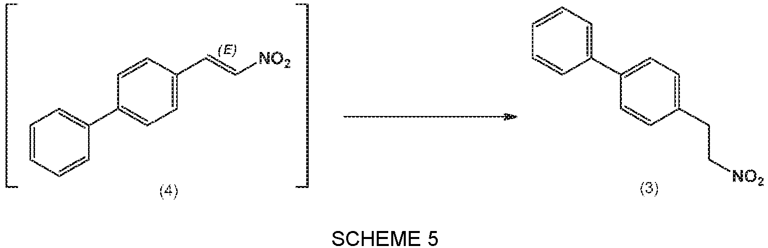

SCHEME 5 depicts a process for the manufacture of a compound of formula (3) said process comprising (a) reacting an aldehyde of formula (6) with nitromethane which leads to the compound of formula (5) or to a mixture of the compounds of formulae (4) and (5), (b) converting the obtained compound of formula (5) into a compound of formula (4) by reacting it with a dehydrating agent, such as an acid anhydride, and (c) hydrogenating the compound of formula (4) or the compound of formula (5) or the mixture of the compounds of formulae (4) and (5), preferably in the presence of a complex hydride, capable of selectively reducing the double bond, to obtain the compound of formula (3).

In one embodiment, the Michael reaction (addition) depicted in SCHEMES 3-1 and 3-2 is performed in the presence of an organocatalyst for Michael reaction, preferably a prolinol and/or thiourea organocatalyst, in particular - then especially for chirally selective synthesis - a chiral prolinol or thiourea organocatalyst.

In one embodiment thereof the catalyst is of one of the formulae (I), (II), (III) or (IV) 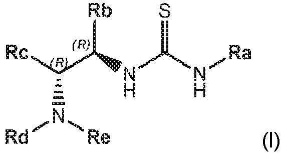 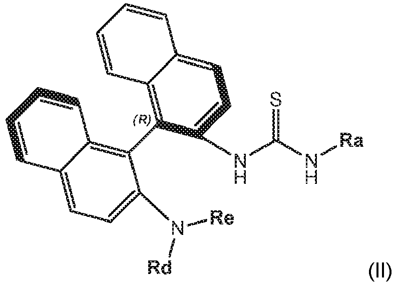 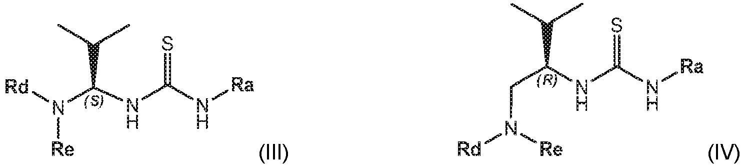 wherein
Ra is C₆-C₁₀-aryl, a heterocyclic group or C₁-C₆-alkyl optionally substituted with C₆-C₁₀-aryl or a heterocyclic group;
Rb and Rc together with the two connecting carbon atoms and the attached nitrogen groups form a chiral scaffold, wherein
   (i) Rb and Rc together with the two connecting carbon atoms form a 5 - 10 membered ring system which is mono or bicyclic and can be saturated, partially unsaturated or unsaturated, or
   (ii) Rb and Rc are each independently selected from hydrogen, C₁-C₇-alkyl, C₆-C₁₀-aryl and a heterocyclic group, and
Rd and Re are independently selected from hydrogen, C₆-C₁₀-aryl, a heterocyclic group, and C₁-C₆-alkyl optionally substituted with one, two or three substituents selected from halogen, hydroxyl, amino, C₆-C₁₀-aryl or a heterocyclic group; or
Rd and Re together with the connecting Nitrogen atom form a group selected from: 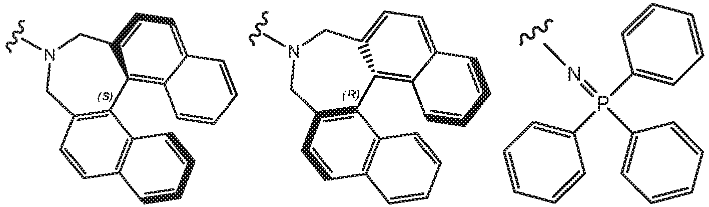 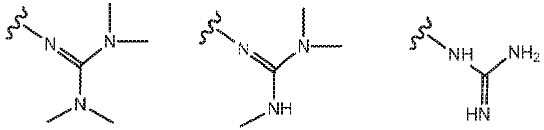 or Rb is selected from hydrogen, C₁-C₇-alkyl, C₆-C₁₀-aryl and a heterocyclic group; and Rc together with the connecting carbon atom and the group -N(Rd)(Re) forms a fused bicyclic 7-9 membered ring system which is optionally substituted with C₁-C₇-alkyl or C₂-C₇-alkenyl;
wherein each heterocyclic group is a mono-, bi- or tricyclic ring system with 5 to 14 ring atoms and 1 to 4 heteroatoms independently selected from N, O, S, S(O) or S(O)₂, and wherein each C₆-C₁₀-aryl or heterocyclic group is optionally substituted by one, two or three residues selected from the group consisting of C₁-C₇-alkyl, hydroxyl, oxo, C₁-C₇-alkoxy, C₂-C₈-alkanoyl-oxy, halogen, nitro, cyano, and CF₃.

In another embodiment thereof the catalyst is of one of the following formulae 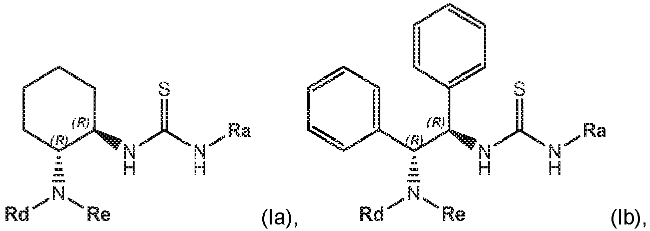 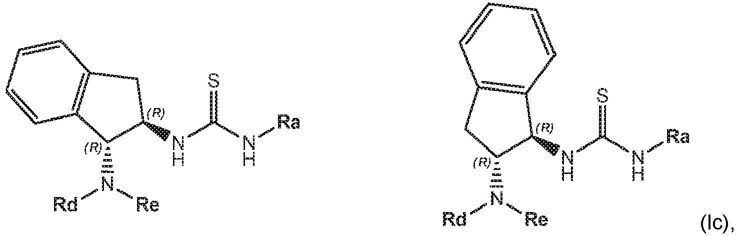 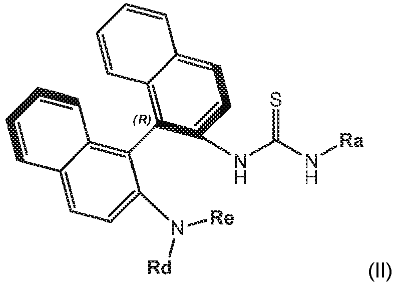 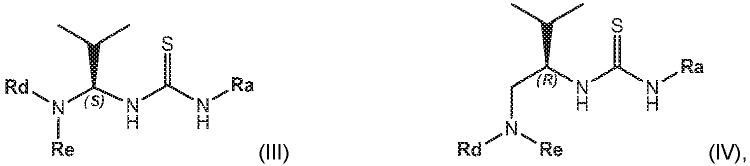 wherein the group -NRdRe forms a group selected from: -NH₂; 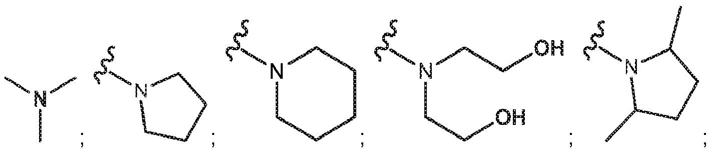 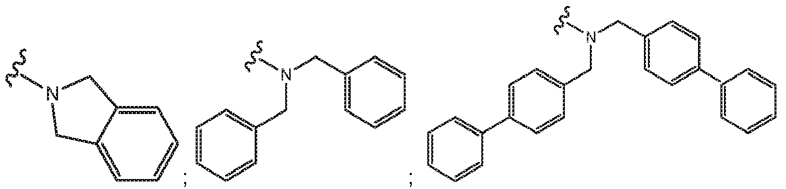 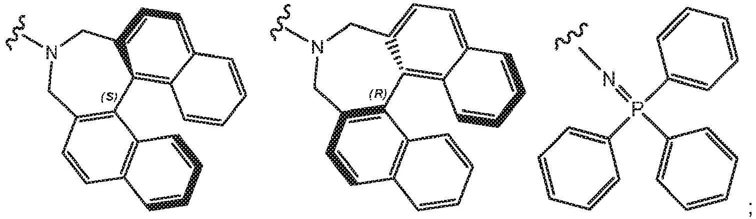 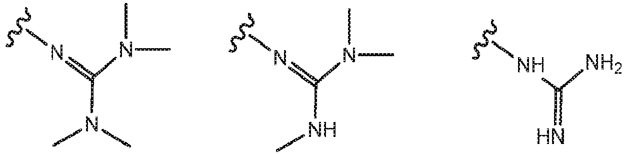 or the catalyst is of the formula (V) 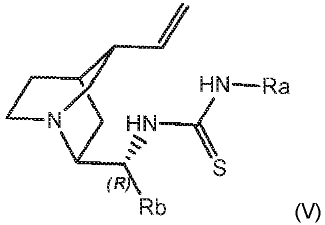 wherein Rb is a quinolone,
and wherein for all formulas Ra is a group

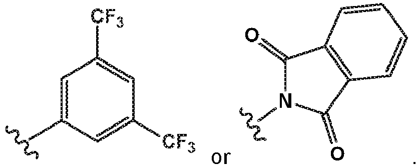

In a **third** aspect, the present invention provides **new organocatalysts** suitable for the manufacture of a compound of formula (2) via Michael addition, as well as to **processes** for their synthesis as described in the Examples or in analogy thereto, which organocatalysts are selected from the group consisting of compounds with the following formulae:

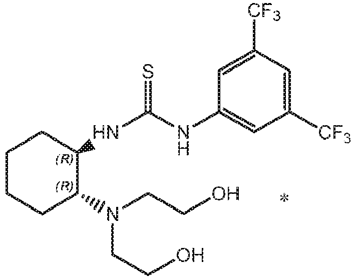

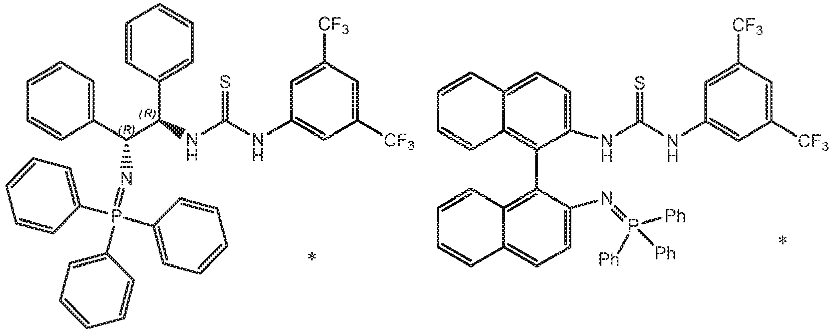

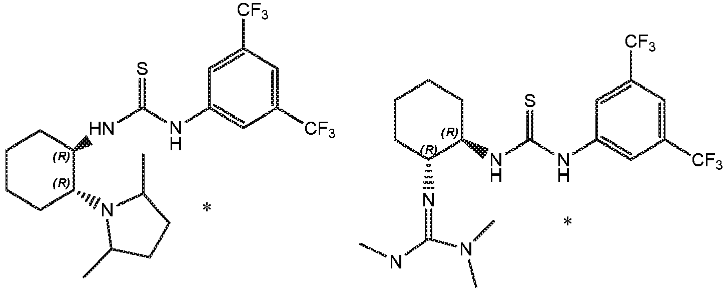

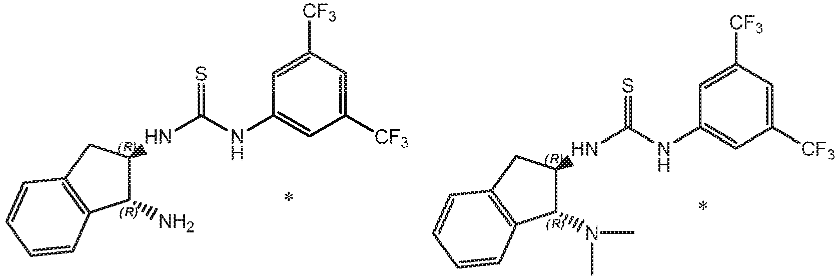

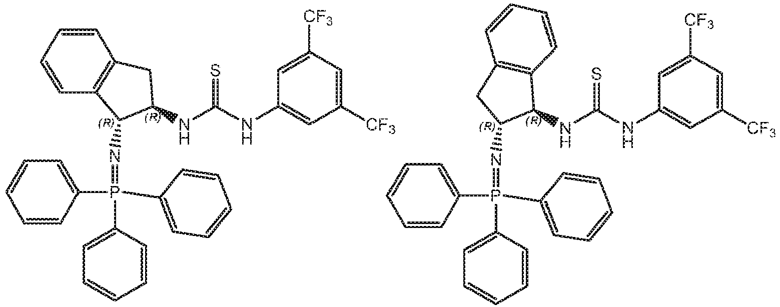

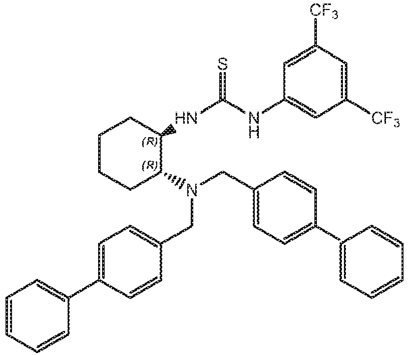

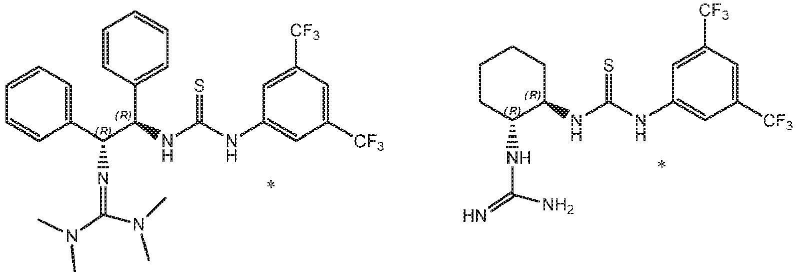

In one embodiment thereof, the catalyst to be used in the process of the invention is selected from the group consisting of those with the following formulae:

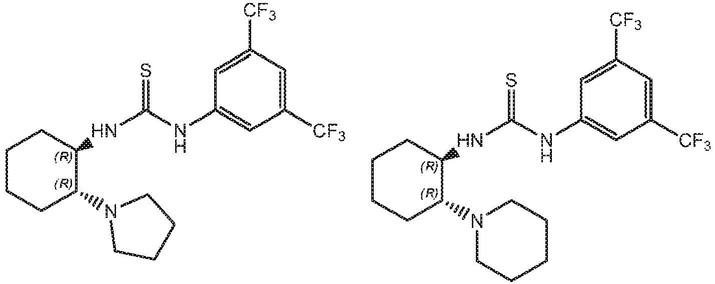

In a fourth aspect, the present invention provides the use of a novel compound of formula (1), (1-a), (2), (2-a) or (7) as depicted above in the manufacture of a compound of formula (10) 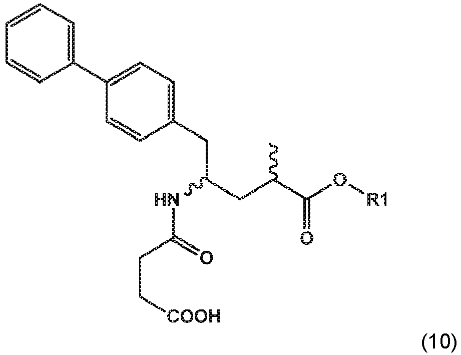 preferably of formula (10-a) 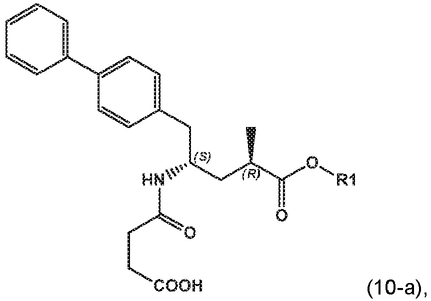 wherein R1 is hydrogen or C₁-C₆-alkyl, preferably ethyl,
preferably in the manufacture of *N*-(3-carboxyl-1-oxopropyl)-(4*S*)-(*p*-phenylphenylmethyl)-4-amino-(2*R*)-methyl butanoic acid, or salts thereof, or *N*-(3-carboxyl-1-oxopropyl)-(4*S*)-(*p-*phenylphenylmethyl)-4-amino-(2*R*)-methyl butanoic acid ethyl ester or salts thereof.

In further embodiments, the invention relates to any one or more of the novel compounds, processes and catalysts represented in the claims which are incorporated here by reference.

The invention also relates to any sequential combination of the process steps described above and below.

In its above mentioned aspects which are also given in more detail below the present invention provides the following advantages: The synthesis route is suitable for industrial scale processing. The synthesis route is economically and environmentally favourable. The compound of formula (1-a) which is an intermediate desired for the synthesis of sacubitril is produced with in high yield and high stereoselectivity.

### DETAILED DESCRIPTION OF THE INVENTION

### General Terms:

The general definitions used above and below, unless defined differently, have the following meanings, where replacement of one or more or all expressions or symbols by the more specific definitions can be made independently for each inventionembodiment and lead to more preferred embodiments:
The compounds of the formula (1) and (2) are a mixture of compounds with configurations *R,R; R,S; S,R* and *SS*, or pure enantiomers/diastereomers, especially of the formula (1-a) or (2-a).

The term "nitrogen protecting group" comprises any group which is capable of reversibly protecting a nitrogen functionality, preferably an amine and/or amide functionality. Preferably the nitrogen protecting group is an amine protecting group and/or an amide protecting group. Suitable nitrogen protecting groups are conventionally used e.g. in peptide chemistry and are described e.g. in the relevant chapters of standard reference works such as J. F. W. McOmie, "Protective Groups in Organic Chemistry", Plenum Press, London and New York 1973, in P. G. M. Wuts and T. W. Greene, "Greene's Protective Groups in Organic Synthesis', fourth edition, Wiley, New Jersey, 2007, and "The Peptides"; volume 3 (editors: E. Gross and J. Meienhofer), Academic Press, London and New York 1981, and "Methoden der organischen Chemie" (Methods of Organic Chemistry), Houben Weyl, fourth edition, volume 15/l, Georg Thieme Verlag, Stuttgart 1974.

Preferred nitrogen protecting groups generally comprise: unsubstituted or substituted C₁-C₆-alkyl, preferably C₁-C₄-alkyl, more preferably C₁-C₂-alkyl, most preferably C₁-alkyl, unsubstituted or substituted C₂₋₄-alkenyl, wherein C₁-C₆-alkyl and C₂₋₄-alkenyl is optionally mono-, di- or tri-substituted by trialkylsilyl-C₁-C₇-alkoxy (e.g. trimethylsilylethoxy), cycloalkyl, aryl, preferably phenyl, or a heterocyclic group, preferably pyrrolidinyl, wherein the cycloalkyl group, the aryl ring or the heterocyclic group is unsubstituted or substituted by one or more, e.g. two or three residues, e.g. selected from the group consisting of C₁-C₇-alkyl, hydroxy, C₁-C₇-alkoxy, C₂-C₈-alkanoyl-oxy, halogen, nitro, cyano, and CF₃; aryl-C₁-C₂-alkoxycarbonyl (preferably phenyl-C₁-C₂-alkoxycarbonyl e.g. benzyloxycarbonyl); C₁₋₁₀-alkenyloxycarbonyl; C₁₋₆-alkylcarbonyl (e.g. acetyl or pivaloyl); C₆₋₁₀-arylcarbonyl; C₁₋₆-alkoxycarbonyl (e.g. *tert*-butoxycarbonyl); C₆₋₁₀-aryl-C₁₋₆-alkoxycarbonyl; allyl or cinnamyl; sulfonyl or sulfenyl; succinimidyl group, silyl, e.g. triarylsilyl or trialkylsilyl (e.g. triethylsilyl).

Examples of preferred nitrogen protecting groups are acetyl, benzyl, cumyl, benzhydryl, trityl, benzyloxycarbonyl (Cbz), 9-fluorenylmethyloxycarbony (Fmoc), benzyloxymethyl (BOM), pivaloyl-oxy-methyl (POM), trichloroethxoycarbonyl (Troc), 1-adamantyloxycarbonyl (Adoc), allyl, allyloxycarbonyl, trimethylsilyl, *tert*-butyl-dimethylsilyl (TBDMS), triethylsilyl (TES), triisopropylsilyl (TIPS), trimethylsilyethoxymethyl (SEM), *tert*-butoxycarbonyl (BOC), *tert*-butyl, 1-methyl-1,1-dimethylbenzyl, (phenyl)methylbenzene, pyrridinyl and pivaloyl. Most preferred nitrogen protecting groups are acetyl, benzyl, benzyloxycarbonyl (Cbz), triethylsilyl (TES), trimethylsilyethoxymethyl (SEM), *tert*-butoxycarbonyl (BOC), pyrrolidinylmethyl and pivaloyl.

Examples of more preferred nitrogen protecting groups are, pivaloyl, pyrrolidinylmethyl, t-butoxycarbonyl, benzyl and silyl groups, particularly silyl groups according to the formula SiRaRbRc, wherein Ra, Rb and Rc are, independently of each other, alkyl or aryl. Preferred examples for Ra, Rb and Rc are methyl, ethyl, isopropyl, t-butyl and phenyl.

Examples of most preferred nitrogen protecting groups are *tert*-butoxycarbonyl (BOC), benzoyl, styryl, 1-butenyl, benzyl, *p*-methoxybenzyl (PMB) and pyrrolidinylmethyl, in particular pivaloyl and *tert*-butoxycarbonyl (BOC).

In one embodiment the term nitrogen protecting group refers to a group which is selected from the group consisting of C₁-C₆-alkyl, which is unsubstituted or mono-, di- or tri-substituted by tri-C₁-C₆-alkylsilylC₁-C₇-alkoxy; C₆-C₁₀-aryl, or a heterocyclic group being a mono-, bi- or tricyclic ring system with 5 to 14 ring atoms and 1 to 4 heteroatoms independently selected from N, O, S, S(O) or S(O)₂, wherein the aryl ring or the heterocyclic group is unsubstituted or substituted by one, two or three residues, selected from the group consisting of C₁-C₇-alkyl, hydroxyl, C₁-C₇-alkoxy, C₂-C₈-alkanoyl-oxy, halogen, nitro, cyano, and CF₃; and C₆-C₁₀-aryl-C₁-C₂-alkoxycarbonyl; C₁-C₁₀-alkenyloxycarbonyl; C₁-C₆-alkylcarbonyl; C₆-C₁₀-arylcarbonyl; C₁-C₆-alkoxycarbonyl; C₆-C₁₀-aryl-C₁-C₆-alkoxycarbonyl; allyl; cinnamyl; sulfonyl; sulfenyl; succinimidyl, and silyl, wherein each silyl group is a SiR11R12R13 group, wherein R11, R12 and R13 are, independently of each other, C₁-C₆-alkyl or C₆-C₁₀-aryl.

Generally, in the present application the term "nitrogen protecting group" comprises any group which is capable of reversibly protecting a amino functionality.

If an embodiment requires the removal of the nitrogen protecting group, as defined above, the removal usually can be carried out by using known methods. Preferably, the nitrogen protecting group, as defined above, is removed by using acidic or basic conditions. Examples for acidic conditions are hydrochloric acid, trifluoroacetic acid, sulphuric acid. Examples of basic conditions are lithium hydroxide, sodium ethoxide. Nucleophiles such as sodium borohydride can be used.

Silyl, as used herein, refers to a group according to the formula -SiR11R12R13, wherein R11, R12 and R13 are, independently of each other, alkyl or aryl. Preferred examples for R11, R12 and R13 are methyl, ethyl, isopropyl, *tert*-butyl, phenyl or phenyl-C₁₋₄-alkyl.

Alkyl is defined as a radical or part of a radical as a straight or branch (one or, if desired and possible, more times) carbon chain, and is especially C₁-C₇-alkyl, preferably C₁-C₄-alkyl.

The term "C₁-C₇-" defines a moiety with up to and including maximally 7, especially up to and including maximally 4, carbon atoms, said moiety being branched (one or more times) or straight-chained and bound via a terminal or a non-terminal carbon.

Cycloalkyl is, for example, C₃-C₇-cycloalkyl and is, for example, cyclopropyl, cyclobutyl, cyclopentyl, cyclohexyl and cycloheptyl. Cyclopentyl and cyclohexyl are preferred.

Alkoxy is, for example, C₁-C₇-alkoxy and is, for example, methoxy, ethoxy, n-propyloxy, isopropyloxy, n-butyloxy, isobutyloxy, sec-butyloxy, *tert*-butyloxy and also includes corresponding pentyloxy, hexyloxy and heptyloxy radicals. C₁-C₄-alkoxy is preferred.

Alkanoyl is, for example, C₂-C₈-alkanoyl and is, for example, acetyl [-C(=O)Me], propionyl, butyryl, isobutyryl or pivaloyl. C₂-C₅-Alkanoyl is preferred, especially acetyl.

Halo or halogen is preferably fluoro, chloro, bromo or iodo, most preferably, chloro, bromo, or iodo.

Halo-alkyl is, for example, halo-C₁-C₇-alkyl and is in particular halo-C₁-C₄-alkyl, such as trifluoromethyl, 1,1,2-trifluoro-2-chloroethyl or chloromethyl. Preferred halo-C₁-C₇-alkyl is trifluoromethyl.

Alkenyl may be linear or branched alkyl containing a double bond and comprising preferably 2 to 12 carbon atoms, 2 to 10 carbon atoms being especially preferred. Particularly preferred is a linear C₂₋ C₇-alkenyl, more preferably C₂-C₄-alkenyl. Some examples of alkyl groups are ethyl and the isomers of propyl, butyl, pentyl, hexyl, heptyl, octyl, nonyl, decyl, undecyl, dodecyl, tetradecyl, hexadecyl, octacyl and eicosyl, each of which containing a double bond. Especially preferred is allyl.

Alkylene is a bivalent radical derived from C₁₋₇-alkyl and is especially C₂-C₇-alkylene or C₂-C₇-alkylene and, optionally, can be interrupted by one or more, e.g. up to three oxygen, NR14 or sulfur, wherein R14 is alkyl, each of which can be unsubstituted or substituted, by one or more substituents independently selected from for example, C₁-C₇-alkyl, C₁-C₇-alkoxy-C₁-C₇-alkyl or C₁-C₇-alkoxy.

Alkenylene is a bivalent radical derived from C₂₋₇-alkenyl and can be interrupted by one or more, e.g. up to three oxygen, NR14 or sulfur, wherein R14 is alkyl, and is unsubstituted or substituted by one or more, e.g. up to three substitutents, preferably independently selected from the substituents mentioned above for alkylene.

Aryl being a radical or part of a radical is, for example C₆₋₁₀-aryl, and is preferably a mono- or polycyclic, especially monocyclic, bicyclic or tricyclic aryl moiety with 6 to 10 carbon atoms, such as phenyl, naphthyl or fluorenyl preferably phenyl, and which can be unsubstituted or substituted,by one or more substituents, independently selected from, e.g. C₁-C₇-alkyl, C₁-C₇-alkoxy-C₁-C₇-alkyl or C₁-C₇-alkoxy.

The term arylalkyl refers to aryl-C₁-C₇-alkyl, wherein aryl is as defined herein and is for example benzyl.

The term carboxyl refers to -CO₂H.

Aryloxy refers to an aryl-O- wherein aryl is as defined above.

Unsubstituted or substituted heterocyclyl is a mono- or polycyclic, preferably a mono-, bi- or tricyclic-, most preferably mono-, unsaturated, partially saturated, saturated or aromatic ring system with preferably 3 to 14 (more preferably 5 to 14) ring atoms and with one or more, preferably one to four, heteroatoms, independently selected from nitrogen, oxygen, sulfur, S(=O)- or S-(=O)₂, and is unsubstituted or substituted by one or more, e.g. up to three substitutents, preferably independently selected from the group consisting of halo, C₁-C₇-alkyl, halo-C₁-C₇-alkyl, C₁-C₇-alkoxy, halo-C₁-C₇-alkoxy, such as trifluoromethoxy and C₁-C₇-alkoxy-C₁-C₇-alkoxy. When the heterocyclyl is an aromatic ring system, it is also referred to as heteroaryl.

Acetyl is -C(=O)C₁-C₇-alkyl, preferably -C(=O)Me.

Sulfonyl is (unsubstituted or substituted) C₁-C₇-alkylsulfonyl, such as methylsulfonyl, (unsubstituted or substituted) phenyl- or naphthyl-C₁-C₇-alkylsulfonyl, such as phenylmethanesulfonyl, or (unsubstituted or substituted) phenyl- or naphthyl-sulfonyl; wherein if more than one substituent is present, e.g. one to three substitutents, the substituents are selected independently from cyano, halo, halo-C₁-C₇-alkyl, halo-C₁-C₇-alkyloxy- and C₁-C₇-alkyloxy. Especially preferred is C₁-C₇-alkylsulfonyl, such as methylsulfonyl, and (phenyl- or naphthyl)-C₁-C₇-alkylsulfonyl, such as phenylmethanesulfonyl.

Sulfenyl is (unsubstituted or substituted) C₆₋₁₀-aryl-C₁-C₇-alkylsulfenyl or (unsubstituted or substituted) C₆₋₁₀-arylsulfenyl, wherein if more than one substituent is present, e.g. one to four substitutents, the substituents are selected independently from nitro, halo, halo-C₁-C₇-alkyl and C₁-C₇-alkyloxy.

Imide refers to a (unsubstituted or substituted) functional group consisting of two acyl groups bound to nitrogen, preferably a cyclic group derived from dicarboxylic acids. Especially preferred is succinimidyl derived from succinic acid or phthalimidyl derived from phthalic acid. The imidyl group may be substituted by one or more substituents independently selected from for example, C₁-C₇-alkyl, C₁-C₇-alkoxy-C₁-C₇-alkyl, C₁-C₇-alkoxy or halo.

Azide refers to a group -N=N⁺=N⁻.

The term "chiral" refers to molecules which have the property of non-superimposability on their mirror image partner, while the term "achiral" refers to molecules which are superimposable on their mirror image partner.

In the formulae of the present application the term " 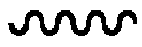 " on a C-sp³ represents a covalent bond, wherein the stereochemistry of the bond is not defined. This means that the term " 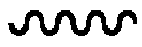 " on a C-sp³ comprises an (*S*) configuration as well as an (*R*) configuration of the respective chiral centre. Furthermore, mixtures, e.g. mixtures of enantiomers such as racemates, are also encompassed by the present invention. Especially preferred are single stereoisomers of the compounds of the formula (1) or (2), especially the specific ones of formula (1-a) and (1-b).

In the formulae of the present application the term " 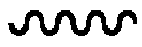 " on a C-sp² represents a covalent bond, wherein the stereochemistry or the geometry of the bond is not defined. This means that the term " 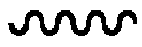 " on a C-sp² comprises a (*Z*) configuration as well as a (*E*) configuration of the respective double bond. Furthermore, mixtures, e.g., mixtures of double bond isomers are also encompassed by the present invention.

The compounds of the present invention can possess one or more asymmetric centers. The preferred absolute configurations are as indicated herein specifically.

In the formulae of the present application the term " 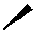 " on a C-sp³ indicates the absolute stereochemistry, either (*R*) or (*S*).

In the formulae of the present application the term " 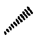 " on a C-sp³ indicates the absolute stereochemistry, either (*R*) or (*S*).

In the formulae of the present application, the term " 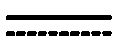 " indicates a C-sp³-C-sp³ bond or a C-sp²-C-sp² bond.

The compounds of the present invention can possess one or more asymmetric centers. The preferred absolute configurations are as indicated herein specifically. However, any possible pure enantiomer, pure diastereoisomer, or mixtures thereof, e.g., mixtures of enantiomers, such as racemates, are encompassed by the present invention.

Stereoisomeric, especially enantiomeric, purity, is where mentioned referring to all diastereomers of the compound taken together (100 %). It is determined by chiral chromatography (examples include HPLC, uPLC and GC) or NMR (with addition of chiral entities and or metals). Specific examples of methods include: chiral HPLC equipped with chiral column Chiralpak ID 4.6mm ø x 250mm, 5 *µ*m (Daicel Corporation, Osaka, Japan) at 25°C; mobil phase Hept:EtOAc:CH₃CN, 90:8:2.

The term "substantially optically pure" compound, as defined herein, refers to a compound obtained by a process according to the invention wherein the compound has an optical purity of at least 70% (ee = enantiomeric excess), more preferably of at least 90% (e.e.) and most preferably at least 95% (ee) or more, such as 100% (ee).

Salts are especially pharmaceutically acceptable salts or generally salts of any of the intermediates mentioned herein, except if salts are excluded for chemical reasons the skilled person will readily understand. They can be formed where salt forming groups, such as basic or acidic groups, are present that can exist in dissociated form at least partially, e.g. in a pH range from 4 to 10 in aqueous solutions, or can be isolated especially in solid, especially crystalline, form.

Such salts are formed, for example, as acid addition salts, preferably with organic or inorganic acids, from compounds or any of the intermediates mentioned herein with a basic nitrogen atom (e.g. imino or amino), especially the pharmaceutically acceptable salts. Suitable inorganic acids are, for example, halogen acids, such as hydrochloric acid, sulfuric acid, or phosphoric acid. Suitable organic acids are, for example, carboxylic, phosphonic, sulfonic or sulfamic acids, for example acetic acid, propionic acid, lactic acid, fumaric acid, succinic acid, citric acid, amino acids, such as glutamic acid or aspartic acid, maleic acid, hydroxymaleic acid, methylmaleic acid, benzoic acid, methane- or ethane-sulfonic acid, ethane-1,2-disulfonic acid, benzenesulfonic acid, 2-naphthalenesulfonic acid, 1,5-naphthalene-disulfonic acid, N-cyclohexylsulfamic acid, *N*-methyl-, *N*-ethyl- or *N*-propyl-sulfamic acid, or other organic protonic acids, such as ascorbic acid.

In the presence of negatively charged radicals, such as carboxy or sulfo, salts may also be formed with bases, e.g. metal or ammonium salts, such as alkali metal or alkaline earth metal salts, for example sodium, potassium, magnesium or calcium salts, or ammonium salts with ammonia or suitable organic amines, such as tertiary monoamines, for example triethylamine or tri(2-hydroxyethyl)amine, or heterocyclic bases, for example *N*-ethyl-piperidine or *N*,*N*'-dimethylpiperazine.

When a basic group and an acid group are present in the same molecule, any of the intermediates mentioned herein may also form internal salts.

For isolation or purification purposes of any of the intermediates mentioned herein it is also possible to use pharmaceutically unacceptable salts, for example picrates or perchlorates.

In view of the close relationship between the compounds and intermediates in free form and in the form of their salts, including those salts that can be used as intermediates, for example in the purification or identification of the compounds or salts thereof, any reference to "compounds", "starting materials" and "intermediates" hereinbefore and hereinafter is to be understood as referring also to one or more salts thereof or a mixture of a corresponding free compound, intermediate or starting material and one or more salts thereof, each of which is intended to include also any solvate or salt of any one or more of these, as appropriate and expedient and if not explicitly mentioned otherwise. Different crystal forms may be obtainable and then are also included.

Where the plural form is used for compounds, starting materials, intermediates, salts, pharmaceutical preparations, diseases, disorders and the like, this intends to mean one (preferred) or more single compound(s), salt(s), pharmaceutical preparation(s), disease(s), disorder(s) or the like, where the singular or the indefinite article ("a", "an") is used, this does not intend to exclude the plural, but only preferably means "one".

The term "pro-drug", as used herein, represents in particular compounds which are transformed *in vivo* to the parent compound, for example, by hydrolysis in blood, for example as described in T. Higuchi and V. Stella, "Pro-drugs as Novel Delivery Systems", volume 14 of the ACS Symposium Series; Edward B. Roche, editor, "Bioreversible Carriers in Drug Design", American Pharmaceutical Association and Pergamon Press, 1987; H Bundgaard, editor, "Design of Prodrugs", Elsevier, 1985; Judkins et al. Synthetic Communications 1996, 26, 4351-4367, and "The Organic Chemistry of Drug Design and Drug Action", second edition, R. B. Silverman (particularly chapter 8, pages 497-557), Elsevier Academic Press, 2004.

Pro-drugs therefore include drugs having a functional group which has been transformed into a reversible derivative thereof. Typically, such prodrugs are transformed to the active drug by hydrolysis. As examples may be mentioned the following:

| ***Functional Group*** | ***Reversible derivative*** |
|---|---|
| *Carboxylic acid* | *Esters, including e.g. alkyl esters* |
| *Alcohol* | *Esters,including e.g. sulfates and phosphates as well as carboxylic acid esters* |
| *Amine* | *Amides, carbamates, imines, enamines,* |
| *Carbonyl (aldehyde,* | *Imines, oximes, acetals*/*ketals, enol esters,* |
| *ketone)* | *oxazolidines and thiazoxolidines* |

Pro-drugs also include compounds convertible to the active drug by an oxidative or reductive reaction. As examples may be mentioned:

### Oxidative activation

- *N*- and *O*-dealkylation
- Oxidative deamination
- *N*-oxidation
- Epoxidation

### Reductive activation

- Azo reduction
- Sulfoxide reduction
- Disulfide reduction
- Bioreductive alkylation
- Nitro reduction

Each of the above described reactions and/or reaction steps can be used individually or in combination in a method to prepare a NEP-inhibitor or a prodrug thereof, such as a NEP inhibitor or pro-drug thereof comprising a γ-amino-δ-biphenyl-α-methylalkanoic acid, or acid ester, such as alkyl ester, backbone. In particular the NEP-inhibitor is *N*-(3-carboxy-1-oxopropyl)-(4*S*)-(*p*-phenylphenylmethyl)-4-amino-(2*R*)-methylbutanoic acid or a salt thereof or a prodrug thereof.

### EMBODIMENTS

The following sections describe the individual process steps as laid out in SCHEMES 1 to 5 above.

### Compounds:

In one embodiment the compound of the formula (1) is represented by formula (1-a) with the following stereochemistry, 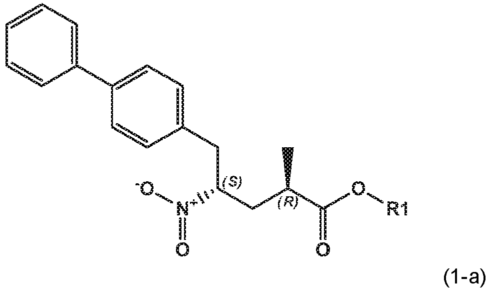 wherein, if compound (1) is present as a mixture of steric forms, the shown steric form is present in at least 60 %, preferably at least 65%, stereoisomeric, especially enantiomeric, purity.

In another embodiment the compound of the formula (2) is represented by formula (2-a) with the following stereochemistry, 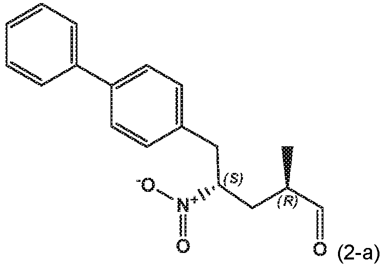 wherein, if compound (2) is present as a mixture of steric forms, the shown steric form is present in at least 60 %, preferably at least 65%, stereoisomeric, especially enantiomeric, purity.

### Michael Addition - Catalysts

In one embodiment, the Michael addition according to any one of SCHEMES 3 is carried out with a organocatalyst selected from the group consisting of those represented by the following formulae:

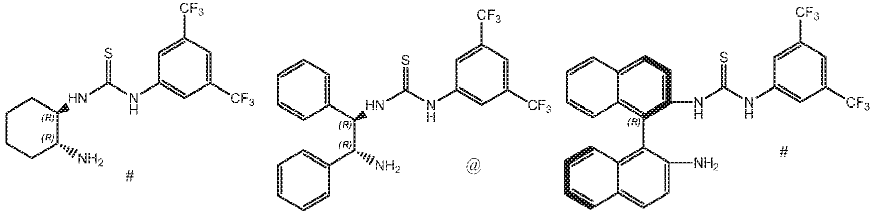

Disclosed in
Left:
(1) Li, De Run; Organic Letters 2010, V12(8), P1756-1759 CAPLUS
(2) Zhang, Xue-jing; Tetrahedron: Asymmetry 2009, V20(12), P1451-1458 CAPLUS
(3) Yu, Feng; Organic & Biomolecular Chemistry 2010, V8(20), P4767-4774 CAPLUS
(4) Fu, Ji-Ya; Tetrahedron Letters 2010, V51(37), P4870-4873 CAPLUS
   Right:
(5) Galzerano, Patrizia; Chemistry - A European Journal 2009, V15(32), P7846-7849, S7846/1-S7846/45 CAPLUS

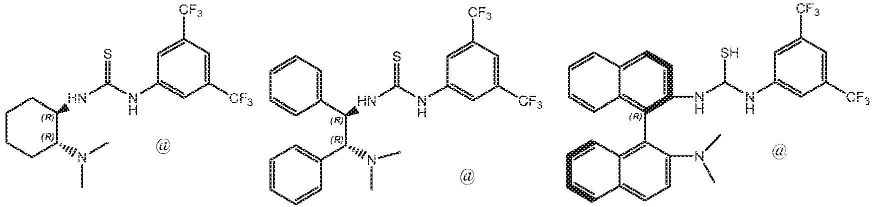

Disclosed by Sigma-Aldrich Corporation, St. Louis, MO, United States of America

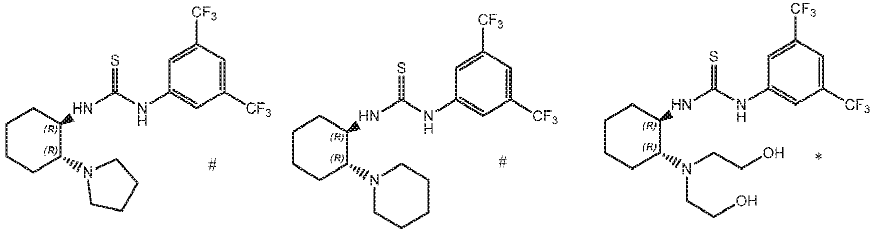

Disclosed in
Left:
(1) Sakamoto, Shota; Inokuma, Tsubasa; Takemoto, Yoshiji From Organic Letters (2011), 13(24), 6374-6377
(2) Bai, Jian-Fei; Wang, Liang-Liang; Peng, Lin; Guo, Yun-Long; Jia, Li-Na; Tian, Fang; He, Guang-Yun; Xu, Xiao-Ying; Wang, Li-Xin, From Journal of Organic Chemistry (2012), 77(6), 2947-2953
(3) Tripathi, Chandra Bhushan; Mukherjee, Santanu, from Organic Letters (2014), 16(12), 3368-3371
   Middle:
(4) Hu, Zhi-Peng; Lou, Chun-Liang; Wang, Jin-Jia; Chen, Chun-Xia; Yan, Ming, from Journal of Organic Chemistry (2011), 76(10), 3797-3804
(5) Sakamoto, Shota; Inokuma, Tsubasa; Takemoto, Yoshiji, from Organic Letters (2011), 13(24), 6374-6377.

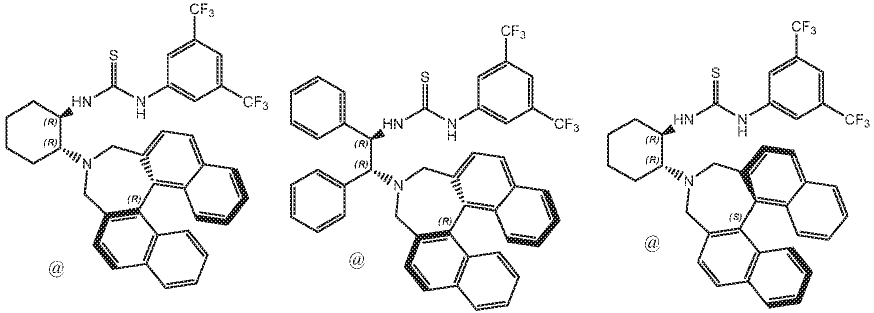

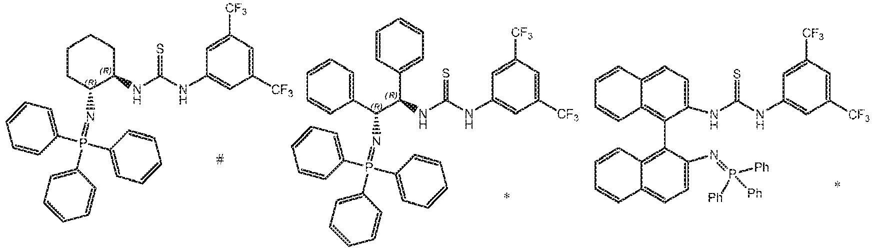

Left & middle compound disclosed in
(1) Nunez, Marta G.; Farley, Alistair J. M.; Dixon, Darren J., from Journal of the American Chemical Society (2013), 135(44), 16348 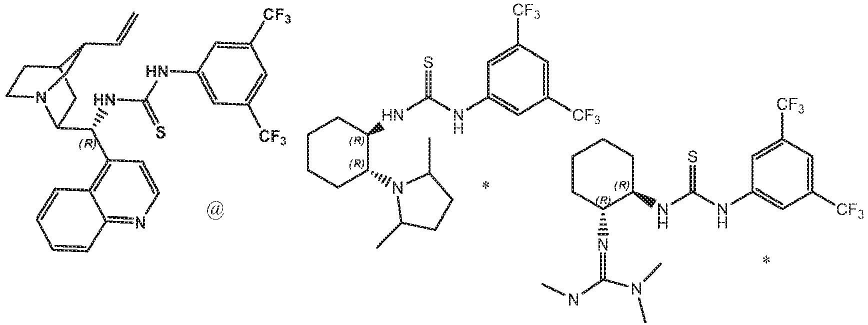
Left compound disclosed in
(1) Sigma-Aldrich Corporation, St. Louis, MO, United States of America 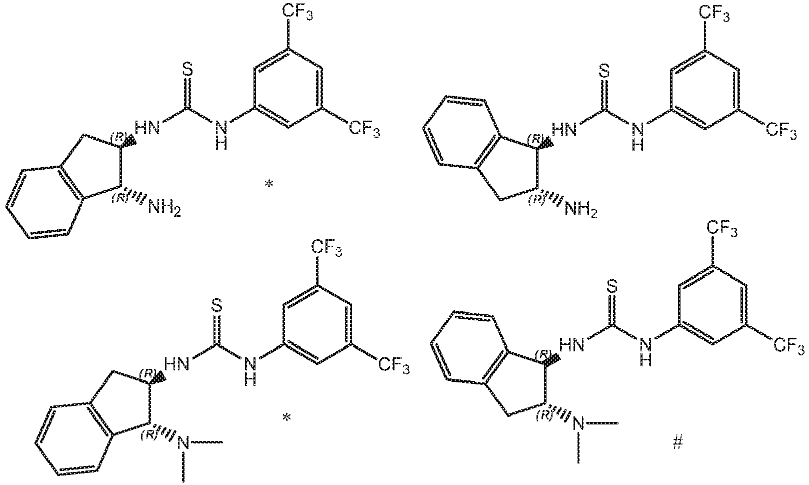
Right Compound disclosed in
(1) Gao, Yaojun; Ren, Qiao; Wu, Hao; Li, Maoguo; Wang, Jian, from Chemical Communications (Cambridge, United Kingdom) (2010), 46(48), 9232-9234
(2) Ren, Qiao; Gao, Yaojun; Wang, Jian, from Chemistry - A European Journal (2010), 16(46), 13594-13598,

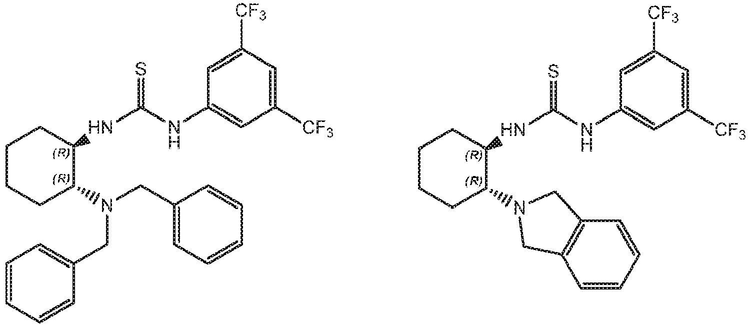

Disclosed in
(1) Berkessel, Albrecht; Seelig, Bianca; Schwengberg, Silke; Hescheler, Juergen; Sachinidis, Agapios, from ChemBioChem (2010), 11(2), 208
(2) Okino, Tomotaka; Hoashi, Yasutaka; Takemoto, Yoshiji, from Journal of the American Chemical Society (2003), 125(42), 12672

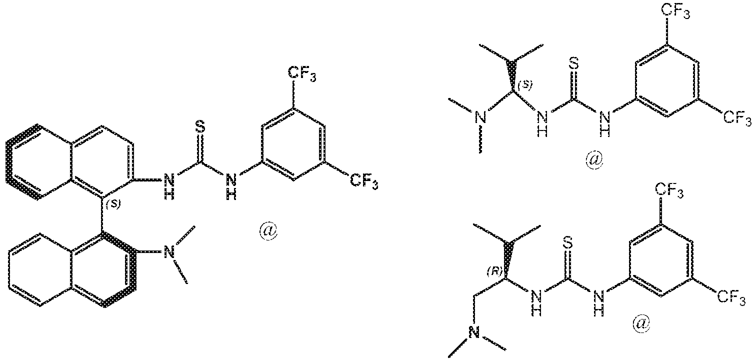

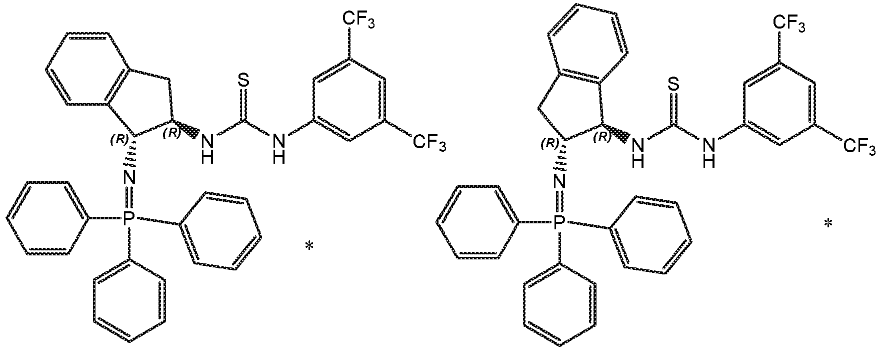

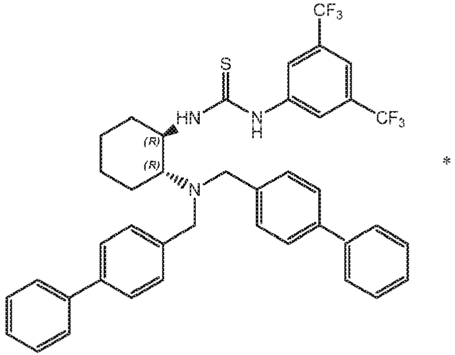

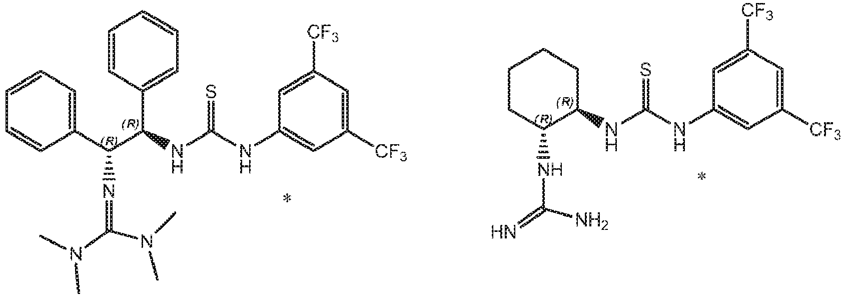

Catalysts commercially available (@), where not otherwise specified, have been both from DAICEL CHIRAL TECHNOLOGIES (CHINA) CO., LTD., Shanghai, People's Republic of China.

These catalysts are either commercially available or amenable according to methods known in the art (see especially the references cited in the preceding scheme, and/or they are novel, (these novel ones are marked with an asterisk * at their lower right side) and thus an invention embodiment and then can be synthesized according to the procedure given in the Examples.

In one embodiment thereof, the organocatalyst is selected from the group consisting of those represented by the following formulae:

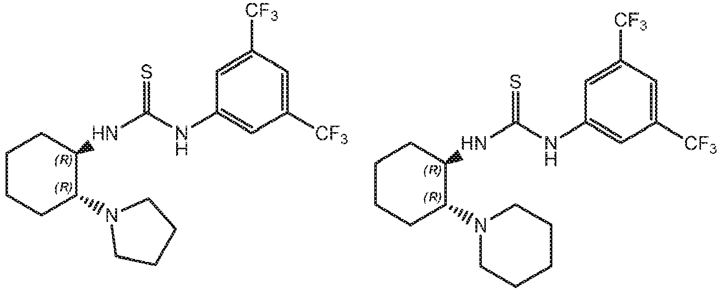

These have the names 1-(3,5-bis(trifluoromethyl)phenyl)-3-((1R,2R)-2-(pyrrolidin-1-yl)cyclohexyl)thiourea and 1-(3,5-bis(trifluoromethyl)phenyl)-3-((1R,2R)-2-(piperidin-1-yl)cyclohexyl)thiourea, respectively.

In another embodiment thereof, the organocatalyst is a prolinol organocatalyst, preferably selected from the group consisting of those with the following formulae: 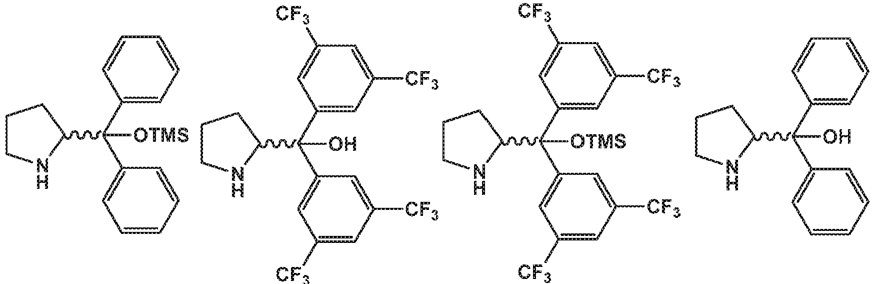 wherein TMS = trimethylsilyl, and which are used in the (R)- or in the (S) configuration, and with the following formulae: 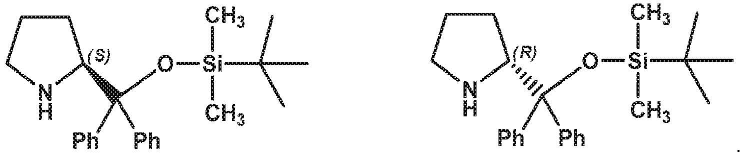 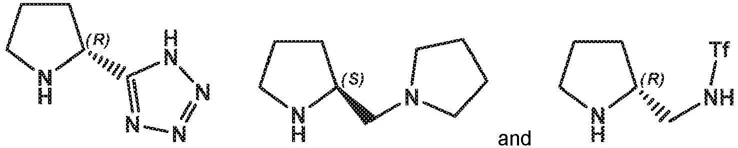 wherein Ph = phenyl and Tf = trifluoromethanesulfonyl.

These catalysts can e.g. be obtained commercially from Sigma-Aldrich Corporation St. Louis, MO, USA.

The proline derivative below is known from the literature 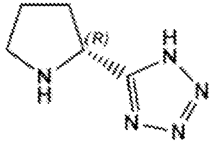 see Cobb, Alexander J. A.; Organic & Biomolecular Chemistry 2005, V3(1), P84-96; and Longbottom, Deborah A.; Franckevicius, Vilius; Ley, Steven V. Chimia (2007), 61(5), 247-256.

### Processes

Wherever room temperature is mentioned in the present disclosure (except in the examples where it refers to 20 to 25 °C), this preferably refers to a temperature of 23 ± 25 °C, e.g. to 23 °C.

Salt conversions (here and anywhere else where mentioned in the present disclosure) can be made using metal (e.g. alkalimetal or earth alkali metal, such as sodium or potassium) salts of organic or inorganic acids, ion exchangers or the like according to methods known in the art.

As solvent for the processes according to the invention, as alternative to those mentioned specifically polar protic solvents (e.g. methanol, ethanol, propanol, butanol), polar aprotic solvents (e.g. tetrahydrofuran (THF), dimethylformamide (DMF), dichloromethane (DCM), acetonitrile (ACN)), or apolar aprotic solvent (e.g. toluene) may be used. Alternatively, mixtures of those solvents/solvent groups or ionic liquids may be used. Preferably, polar solvents are used. More preferably, polar aprotic solvents are used to achieve high yields. A particularly preferred solvent in this regard is THF.

The embodiment depicted in **SCHEME 1 and 1-a** refers to process, wherein a compound of the formula (1), especially of the formula (1-a), is subjected to a hydrogenation reaction to yield a compound of the formula (8), especially of the formula (8-a), which preferably takes place in the presence of a mild hydrogenation catalyst (not affecting the carboxyl or carboxyl ester function), for example by hydrogenation with hydrogen, e.g. at normal or slightly elevated pressure, in an appropriate solvent, such as an alcohol, e.g. methanol or ethanol, with e.g. at temperatures in the range from -10 to 60 °C, such as from 20 to 50 °C.

More specifically, there is provided the process according to **the reaction depicted in SCHEME 1 or 1-a**, wherein the hydrogenation comprises the use of a metal catalyst, preferably a metal catalyst comprising a metal selected from the group of nickel, palladium or platinum, more preferably Raney nickel, even more preferably Raney nickel type 3202.

The process is preferably performed in a hydrogenation reactor. Preferably the catalyst is applied to this process as 50% water slurry.

The process is performed with pressurized hydrogen, preferably the hydrogen is pressurized up to 10, e.g. up to 4 bar.

If a nitrogen protecting group is to be inserted in parallel, the reactions are preferably conducted as described in the general part on nitrogen protecting groups above. For example, to insert a ter-butoxycarbonyl protecting group, Boc₂O is used for this process in excess over sum of compounds of formulas (1) or (1-a, preferably in an excess of 20 - 100 %, more preferably in an excess of 50 ± 10 %.

The process preferably performed at elevated temperatures, preferably from 30 - 70 °C, more preferably from 35 - 50 °C, even more preferably from 40 - 45 °C.

In a specific embodiment, the process is performed with Raney nickel as 50% water slurry, using Boc₂O in excess over sum of compound of formula (1) or (1-a), preferably in an excess of 20 - 100 %, more preferably in an excess of 50 ± 10 %, using pressurized hydrogen, preferably using hydrogen pressurized up to 4 bar, at elevated temperatures, preferably from 30 - 70 °C, more preferably from 35 - 50 °C, even more preferably from 40 - 45 °C.

The Oxidation reaction according to **SCHEME 2 or 2-a** of a compound (aldehyde) of the formula (2), especially (2-a), to yield the compound of formula (1), preferably takes place with an oxidant allowing for selective oxidation of the aldehyde function to a carboxyl (COOH or COO⁻) function. Such oxidants and oxidation conditions are known to the person skilled in the art. Examples of possible oxidants include but are not limited to Oxone (e.g. in DMF, e.g. at room tempareture), with H₅IO₆ in the presence of a catalyst, such as pyridiniumn chroroformate, a solvent, e.g. acetonitrile, e.g. at room temperature; with sodium perborate in the presense of a catalyst such as VO(acac)₂ and hydrogen peroxide, e.g. in a solvent such as acetonitrile or an alcohol, such as methanol or ethanol, e.g. at room temperature; with sodium perborate in acetic acid e.g. at elevated temperature, such as 20 to 70°C; with potassium permanganate in a buffer, especially disodiumhydrogenphosphate buffer, e.g. in a solvent such as methanol; with hydrogen peroxide in the presence of a methyltrioxorhenium catalyst in an ionic liquid, such as [bmim]BF₄ or [bmim]PF₆, e.g. at elevated temperature, such as 30 to 80 °C; or especially under Pinnick oxidation conditions (also known as Lindgren oxidation), using a chlorite salt, e.g. an alkalimetal chlorite, such as sodium chlorite (NaClO₂), preferably in the presence of a buffering substance, such as an alkali metal dihydrogen phosphate, e.g. sodium dihydrogen phosphate, in the presence of a scavenger for the byproduct hypochlorous acid (HOCl), such as an alkene-comprising chemical, e.g. 2-methyl-2-butene or hydrogen peroxide of resorcinol or sulfamic acid, and in a solvent or solvent mixture, e.g. an alcohol, such as butanol, or an ester, such as acetic acid ethyl ester, or a mixture thereof, for example at temperatures in the range from -5 to 80 °C, e.g. at room temperature. Further possible methods for the oxidation are known to the person skilled in the art and can, for example, be derived from Handbook of Reagents for Organic Synthesis, Oxidizing and Reducing Agents Steven D. Burke (Editor), Rick L. Danheiser (Editor) ISBN: 978-0-471-97926-5.

The reaction may be conducted under simultaneous esterification to a compound of the formula (1), especially (1-a), wherein R₁ is C₁-C₆-alkyl, especially ethyl, or by subsequent esterification, in both cases reacting with a C₁-C₆-alkanol, especially ethanol, preferably in the presence of an activator of the carboxyl group in formula I that leads to an (at least intermediary) reactive derivative of a compound of the formula (1), especially (1-a), such as an acid anhydride, e.g. acetic anhydride, or a coupling agent selected from the group consisting of those customary in peptide synthesis, such as aminium compounds, carbodiimides, uranium compounds, and other coupling reagents, for example DCC, DIC, HOBt, HOAt, if appropriate in the presence of a tertiary nitrogen base, such as triethylamine, if required in an appropriate solvent; or by transesterification, e.g. from a corresponding C₁-C₆-alkyl, especially ethyl, ester e.g. of acetic acid; such conditions are known to the person skilled in the art.

In the process according to **SCHEME 3-1 or 3-1a**, the reaction of the compound of the formula (3) with methacroleine or a reactive derivatve thereof, such as an acetal, e.g. the dialkyl acetal, to a compound of the formula (2), especially (2-a), is conducted in the presence of an organocatalyst as described above, preferably one of those described above as being preferred, most especially a chiral prolinol or thiourea catalyst, especially one of those mentioned as preferred above, the catalyst preferably being present in a molar ratio, compared to the compound of the formula (3), of 1 to 50 mol %, e.g. at 5 to 15 mol %; especially in an organic solvent, such as toluene, at temperatures e.g. in the range from -5 to 50 °C, e.g. at 0 to 20 °C. Preferably the organocatalyst is selected so as to achieve the preferred compound of formula (2-a), e.g. a thiourea catalyst of the formula (B) or (C) mentioned above, or a prolinol catalyst selected from those mentioned and represented as specific formulae above. Organic acid or alcohols can be added especially benzoic acid or its derivatives, and/ or catechol derivatives can be added to promote the catalysis of 1 to 50 mol %, e.g. at 5 to 15 mol % catalyst.

According to the reaction as depicted in **SCHEME 3-2 or 3-2a**, as an alternative to the reaction according to SCHEME 3-1 which is starting from the compound of the formula (3), the compound of the formula (2), especially (2-a), is obtained by reacting the compound of the formula (7) with propionaldehyde or a reactive derivative thereof, e.g. an acetal, such as a dialkyl a to a compound of the formula (2), especially (2-a), is conducted in the presence of an organocatalyst as described above, preferably one of those described above as being preferred, most especially a chiral prolinol or thiourea catalyst, especially one of those mentioned as preferred above, the catalyst preferably being present in a molar ratio, compared to the compound of the formula (3), of 1 to 50 mol %, e.g. at 5 to 15 mol %; especially in an organic solvent, such as toluene or dimethylformamide, at temperatures e.g. in the range from -5 to 50 °C, e.g. at 0 to 20 °C. Preferably the organocatalyst is selected so as to achieve the preferred compound of formula (2-a), e.g. a thiourea catalyst of the formula (B) or (C) mentioned above, or a prolinol catalyst selected from those mentioned and represented as specific formulae above. Preferably the reaction takes place in the presence of organic acid or alcohols, especially benzoic acid or its derivatives or catechol derivatives, which can be added to promote the catalysis of 1 to 50 mol %, e.g. at 5 to 15 mol % catalyst.

The reaction as depicted in **SCHEME 4** of a compound of formula (3) with formaldehyde or reactive derivative thereof, such as an acetal, e.g. a dialkylacetal, e.g. dimethoxymethane, dioxolane or 1,3,5-trioxane, to yield a compound of the formula (7) preferably takes place in analogy to or according to a Henry reaction, preferably first reacting the formaldehyde or reactive derivative thereof in the presence of a base, such as an alkalimetal hydroxide, e.g. sodium hydroxide, in an appropriate solvent, e.g. tetrahydrofurane, at a temperature e.g. in the range from -20 to 50 °C, e.g. from -10 to 10 °C, if desired isolation of the reaction product, e.g. by solvent extraction in the organic layer, and then subjecting the (e.g. crude) material to treatment with an acid anhydride, such as trifluoroacetic anhydride, in the presence of a tertiary nitrogen base, such as N,N-diisopropyl-N-ethylamine, in an organic solvent, such as toluene, at a preferred temperature e.g. in the range from -10 to 50 °C, e.g. from 0 to 30 °C.

In the process as depicted in **SCHEME 5** the hydrogenating (hydrogenation) of a compound of the formula (4), a compound of the formula (5) (preferred) or both (e.g. if obtained as a mixture in the process as described above or especially in a preferred version below) to yield the compound of the formula (3) is preferably conducted in the presence of a hydrogenating agent, especially a complex metal hydride, such as an alkalimetal borohydride, e.g. sodium borohydride, preferably in an appropriate solvent liquid at the reaction temperature, such as an organic acid, e.g. acetic acid, an alcohol, such as methanol or ethanol, an organic sulfoxide, such as dimethyl sulfoxide (DMSO), or an ether, such as diethyl ether, or a mixture of two or more thereof, preferably at a temperature in the range from -20 to 50 °C, e.g. -10 to 25 °C.

**SCHEME 5** further embodies the process step wherein, the compound of the formula (5) is reacted with a dehydrating agent to the compound of the formula (4). The dehydrating agent can be, for example, an acid anhydride of an inorganic (such as phosphorous pentoxide) or preferably organic acid (such as acetic anhydride). The reaction is preferably conducted in the presence of a tertiary nitrogen base, such as trimethylamine, and in the presence of an appropriate solvent, such as dichloromethane, at temperatures e.g. in the range from -20 to 50 °C, such as from -10 to 10 °C.

**SCHEME 5** also encompasses the process step wherein the aldehyde of the formula (6) or a reactive derivative thereof, e.g. an acetal, such as a dialkyl acetal, is preferably reacted with nitromethane in an appropriate solvent, such as an acholo, e.g. methanol or ethanol, in the presence of a base, such as an alkalimetal hydroxide, e.g. sodium hydroxide, especially in the form of an aqueous solution of the base, at a preferred temperature in the range from -20 to 50 °C, e.g. from -10 to 10 °C, and thus the compound of the formula (5) alone or in mixture with the compound of the formula (4) is obtained.

### Further Embodiments

The present invention also covers combinations of several reaction steps, e.g.
- a process for the manufacture of a compound of formula (8), preferably of formula (8-a) from a compound of formula (1), preferably of formula (1-a) according to SCHEME 1, wherein the starting compound of formula (1), preferably of formula (1-a), is obtained from a compound of formula (2), preferably of formula (2-a) by a process according to SCHEME 2;
- a process for the manufacture of a compound of formula (1), preferably of formula (1-a) from a compound of formula (2) preferably of formula (2-a) according to SCHEME 2, wherein the starting compound of formula (2), preferably of formula (2-a), is obtained by a process according to SCHEME 3-1 or 3-2;
- a process for the manufacture of a compound of formula (8), preferably of formula (8-a) from a compound of formula (1), preferably of formula (1-a) according to SCHEME 1, wherein the compound of formula (1), preferably of formula (1-a), is obtained from a compound of formula (2), preferably of formula (2-a) by a process according to SCHEME 2, and wherein the starting compound of formula (2), preferably of formula (2-a), is obtained by a process according to SCHEME 3-1 or 3-2;
- a process for the manufacture of a compound of formula (2), preferably of formula (2-a) from a compound of formula (7) according to SCHEME 3-2, wherein the starting compound of formula (7) is obtained by a process according to SCHEME 4;
- a process for the manufacture of a compound of formula (1), preferably of formula (1-a) from a compound of formula (2) according to SCHEME 2, wherein the starting compound of formula (2), preferably of formula (2-a), is obtained from a compound of formula (7) by a process according to SCHEME 3-2, wherein the starting compound of formula (7) is obtained by a process according to SCHEME 4;
- a process for the manufacture of a compound of formula (8), preferably of formula (8-a) from a compound of formula (1), preferably of formula (1-a) according to SCHEME 1, wherein the compound of formula (1), preferably of formula (1-a), is obtained from a compound of formula (2), preferably of formula (2-a) by a process according to SCHEME 2, and wherein the starting compound of formula (2), preferably of formula (2-a), is obtained by a process according to SCHEME 3-2, wherein the starting compound of formula (7) is obtained by a process according to SCHEME 4;
- a process for the manufacture of a compound of formula (7) from a compound of formula (3) according to SCHEME 4, wherein the starting compound of formula (7) is obtained by a process according to SCHEME 5;
- a process for the manufacture of a compound of formula (2) preferably of formula (2-a) from a compound of formula (3) according to SCHEME 3-1, wherein the starting compound of formula (3) is obtained by a process according to SCHEME 5;
- a process for the manufacture of a compound of formula (2), preferably of formula (2-a), from a compound of formula (7) according to SCHEME 3-2, wherein the starting compound of formula (7) is obtained is obtained from a compound of formula (3) by a process according to SCHEME 4, and wherein the starting compound of formula (3) is obtained by a process according to SCHEME 5.

### Follow on reaction of a compound of formula (8) to produce a NEP inhibitor

In another embodiment of the invention the intermediates and the products of the process of the present invention can be used in the synthesis of NEP inhibitors or salts or pro-drugs thereof, in particular they can be used in the synthesis of NEP inhibitors comprising a γ-amino-δ-biphenyl-α-methylalkanoic acid, or acid ester, backbone. NEP inhibitors or pro-drugs thereof comprising a γ-amino-δ-biphenyl-α-methylalkanoic acid, or acid ester, backbone include, for example, the NEP inhibitor pro-drug *N*-(3-carboxy-1-oxopropyl)-(4*S*)-(*p*-phenylphenylmethyl)-4-amino-(2*R*)-methylbutanoic acid ethyl ester and the corresponding NEP inhibitor *N*-(3-carboxy-1-oxopropyl)-(4*S*)-(*p*-phenylphenylmethyl)-4-amino-(2*R*)-methylbutanoic acid.

The term "NEP inhibitor" describes a compound which inhibits the activity of the enzyme neutral endopeptidase (NEP, EC 3.4.24.11).

Compounds of formula (8) or salts thereof, preferably of formula (8-a), or salts thereof, as described herein above can be further reacted to a NEP inhibitor or salts or prodrugs thereof, in particular to the NEP inhibitor prodrug *N*-(3-carboxy-1-oxopropyl)-(4*S*)-(*p-*phenylphenyl-methyl)-4-amino-(2*R*)-methylbutanoic acid ethyl ester or the corresponding NEP inhibitor *N*-(3-carboxy-1-oxopropyl)-(4*S*)-(*p*-phenylphenylmethyl)-4-amino-(2*R*)-methylbutanoic acid as described by Ksander et al. in J. Med. Chem. 1995, 38, 1689-1700, or as described in WO 2008/31567.

In a preferred embodiment of the invention a compound according to formula (8), preferably of formula (8-a), or salt thereof, is further reacted to obtain the NEP inhibitor pro-drug of formula (10) 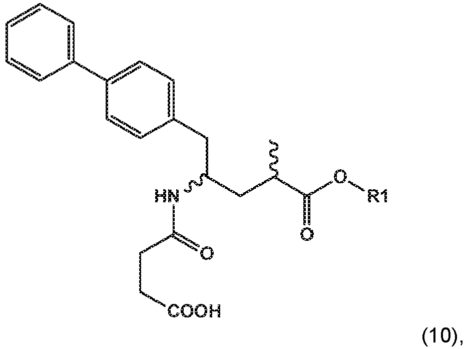 preferably of formula (10-a) 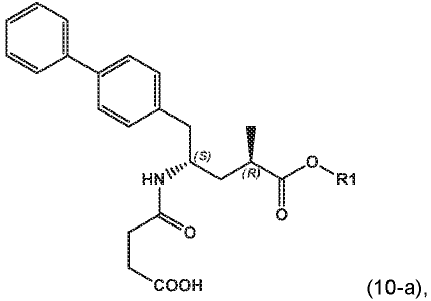 wherein R1 is hydrogen or C₁-C₆-alkyl, preferably ethyl, by reaction with succinic acid or a derivative thereof, preferably succinic acid anhydride.

Deprotection of the nitrogen functionality, i.e. removal of the Boc group, - if necessary - reintroduction of the ethyl ester group, and subsequent coupling with succinic anhydride delivers the desired NEP inhibitor prodrug compound. Optionally, the ester can be saponified to the free acid providing the NEP inhibitor drug compound.

In one embodiment, the compound of formula (10-a) is *N*-(3-carboxy-1-oxopropyl)-(4*S*)-(*p-*phenylphenylmethyl)-4-amino-(2*R*)-methylbutanoic acid ethyl ester (known in the art as AHU377) or a salt thereof.

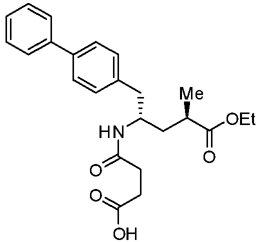

The NEP inhibitor pro-drug *N*-(3-carboxy-1-oxopropyl)-(4*S*)-(*p*-phenylphenylmethyl)-4-amino-(2R)-methylbutanoic acid ethyl ester optionally is further reacted to obtain the NEP inhibitor *N*-(3-carboxy-1-oxopropyl)-(4*S*)-(*p*-phenylphenylmethyl)-4-amino-(2*R*)-methylbutanoic acid.

For example, this conversion can be performed using the Boc protected amino acid e.g. according to scheme Z below where the Boc corresponds to R' and R" is H in formula (8-a) and R1 is H and the aminoethyl ester (upper right formula in scheme Z below with R' = H, R" = H, R1 = Et). If R1 is ethyl, the reaction works identically. If R1 is a different alkyl group, then transesterification or saponification is needed before.

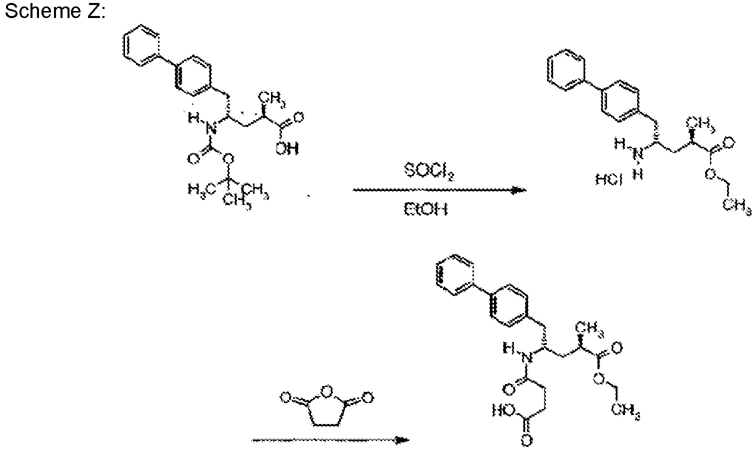

### Examples

The following examples serve to illustrate the invention without limiting the scope thereof, while they on the other hand represent preferred embodiments of the reaction steps, intermediates and/or the process of the present invention.

### ABBREVIATIONS:

- δ: chemical shift
- µl: microlitre
- Ac: acetyl
- ACNL: acetonitrile
- AcOH: acetic acid
- Ac₂O: acetic acid anhydride
- Bn: benzyl
- Boc: *tert*-butoxycarbonyl
- BOC₂O: di-*tert*-butyl carbonate
- Brine: saturated (at room temperature) sodium chloride solution in water
- BuOH: n-butanol
- Cbz: benzyl carbamate
- Cbz-Cl: benzyl chloroformate
- DBU: 1,8-Diazabicycloundec-7-ene
- DCM: dichloromethane/ methylenechloride
- de: diastereomeric excess
- DIPEA: diisopropylethylamine
- DMAP: 4-(dimethylamino)pyridine
- DMF: *N*,*N*-dimethylformamide
- DMPU: 1,3-dimethyl-3,4,5,6-tetrahydro-2(1*H*)-pyrimidinone
- DMSO: dimethylsulfoxide
- ee: enantiomeric excess
- ES: electrospray
- ESI: electrospray ionisation
- Et: ethyl
- Et₂O: diethyl ether
- Et₃N: triethanolamine
- EtOH: ethanol
- EtOAc: ethyl acetate
- GC: gas chromathography
- h: hour(s)
- Hep: heptane(s)
- Hex: hexane(s)
- HNMR: proton nuclear magnetic resonance
- HOBt: 1-hydroxybenzotriazole
- HPLC: high performance liquid chromatography
- i-Pr: isopropyl
- IPA or iPrOAc: isopropyl acetate
- iPr₂O: isopropyl alcohol
- IR: infra red
- KHMDS: potassium bis(trimethylsilyl)amide
- L: litre
- LC-MS: liquid chromatography-mass spectrometry
- LDA: lithium diisopropylamide
- LHMDS: lithium bis(trimethylsilyl)amide
- M: molarity
- m/e: mass-to-charge ratio
- Me: methyl
- MeOH: methanol
- mg: milligram
- min: minute(s)
- mL: millilitre
- mmol(s): millimole(s)
- mol(s): mole(s)
- MS: mass spectrometry
- NaHMDS: sodium bis(trimethylsilyl)amide
- nm: nanometre
- NMR: nuclear magnetic resonance
- Pd/C: palladium on carbon
- Ph: phenyl
- PHCH₃: toluene
- Piv: pivaloyl
- Piv-Cl: pivaloyl chloride
- ppm: parts per million
- psi: pounds per square inch
- RP: reverse phase
- RT: room temperature
- rt: retention time
- SEM: 2-(trimethylsilyl)ethoxymethyl
- SEM-Cl: (2-chloromethoxyethyl)-trimethylsilane
- SM: starting material
- TEMPO: (2,2,6,6-tetramethyl-piperidin-1-yl)oxidanyl
- TES: triethylsilyl
- Tf: triflyl, rifluoromethanesulfonyl
- TFA: trifluoroacetic acid
- TFAA: trifluoroacetic acid anhydride
- THF: tetrahydrofuran
- TLC: thin layer chromatography
- TMEDA: *N*,*N*,*N*',*N*'-tetramethylethylenediamine
- TMS: trimethylsilyl
- t_{R}: retention time
- Ts: tosyl
- TsO: tosylate
- uPLC: Ultra Performance Liquid Chromatography

In quoting NMR data, the following abbreviations are used: s, singlet; d, doublet; t, triplet; q, quartet; quint., quintet; m, multiplet.

Note that in the Examples the numbers of compounds, such as 1 or 2, are different and separate from the compounds represented by numbers in parenthesis above and in the claims, such as (1) or (2), just to clarify paradigmatically.

### Example 1: Synthesis of (2R,4S)-4-amino-5-([1,1'-biphenyl)-4-yl)-2-methyl-pentanoic acid chlorohydrate

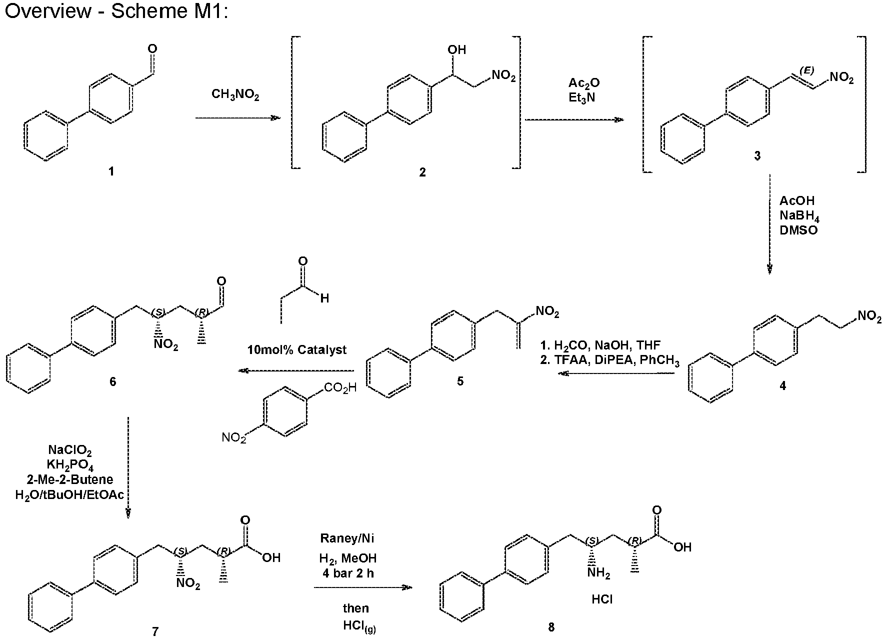

### Example of catalyst includes (catalysts commercially available or reported in literature)

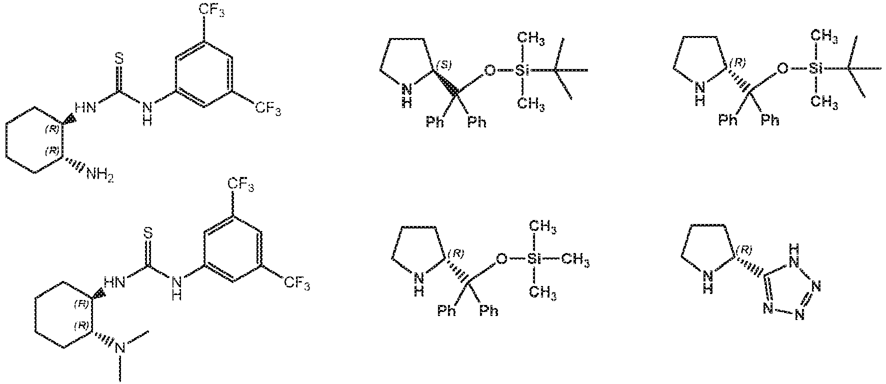

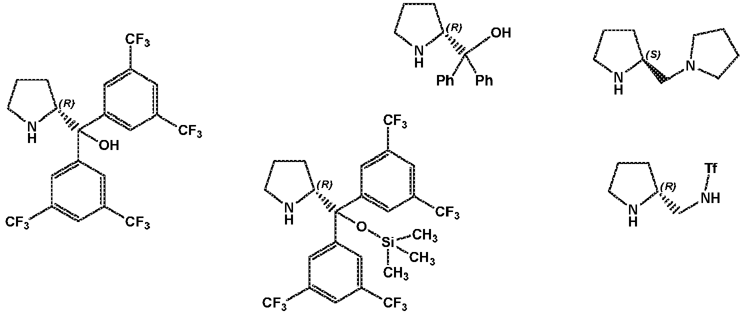

### a) 1-(biphenyl-4-yl)-2-nitroethanol (Compound 2) from biphenyl-4-carbaldehyde (2)

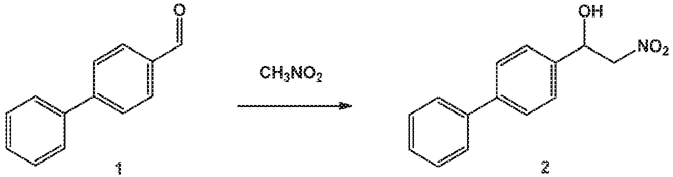

To a solution of 4-biphenyl carboxaldehyde (Sigma-Aldrich Corporation St. Louis, MO, USA) (25 g, 137 mmol) and nitromethane (7.3 mL, 137 mmol) in methanol (125 mL), sodium hydroxide (6.25 g, 164 mmol) in water (21 mL) was added dropwise by maintaining the internal temperature at 0 °C. The reaction mixture was stirred at 0° C for 5 h. The progress of the reaction was monitored by HPLC. The reaction mixture was diluted with ice-cold water (150 mL) and stirred for 15 min at 0 °C. The resultant reaction mixture was acidified with 6 N HCI (250 mL) at 0 °C and stirred for 30 min. The yellow solid precipitated out was filtered, washed with water and dried under vacuum to get a yellow solid (23 g). Analysis revealed that the isolated material is a mixture of compounds 2 and 3. This material has been used for the next step without any further purification.

### b) 4-[2-nitroethenyl]biphenyl (Compound 3) from 1-(biphenyl-4-yl)-2-nitroethanol (3)

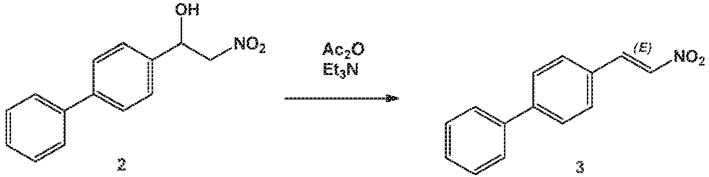

To a solution of the above mixture (23 g, 94.5 mmol, considered as 1 eq.) in dichloromethane (230 mL) acetic anhydride (18 mL, 189 mmol) and triethylamine (26 mL, 189 mmol) were added at 0° C. The reaction mixture was stirred at room temperature for 16 h. The progress of the reaction was monitored by TLC. The reaction mixture was diluted with ice-cold water (100 mL) at 0 °C and concentrated under reduced pressure to remove dichloromethane completely. The brown solid was precipitated out from the resultant aqueous layer. It was washed with excess of water, filtered and dried under *vacuum* to get compound 3 as a yellow solid (18 g), used for the next step without further purification.

### c) 4-(2-nitroethyl)biphenyl (Compound 4) from 4-[2-nitroethenyl]biphenyl (4)

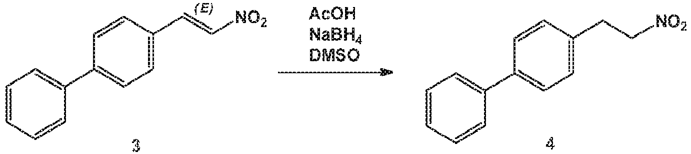

To a solution of compound 3 (10 g, 44.4 mmol) in dimethylsulfoxide (35 mL) was added acetic acid (5 mL, 88.8 mmol) followed by sodium borohydride (0.51 g, 13.3 mmol) portionwise (3 lots) at 0 °C and stirred for 90 min. The progress of the reaction was monitored by TLC. The reaction mixture was quenched with ice-cold water (50 mL) at 0 °C and extracted with dichloromethane (2 x 100 mL). The combined organic layers were washed with water (2 x 100 mL) and saturated brine solution (100 mL), dried over anhydrous Na₂SO₄ and concentrated under reduced pressure. The crude material was purified by column chromatography on silica-gel using 6 % ethyl acetate in hexane as an eluent to afford compound 4 as a pale yellow solid. Yield: 6 g (26.4 mmol, 34 %, over 3 steps).
¹H-NMR (300 MHz, CDCl₃) *δ* 1.58 (s, 1 H), 3.37 (t, 2 H, J = 8 Hz), 4.65 (t, 2 H, J = 7.5 Hz), 7.263-7.384 (m, 2H), 7.423 (m, 1H), 7.448-7.472 (m, 2 H), 7.55-7.59 (m, 2 H).
¹³C-NMR (100 MHz, CDCl₃) *δ* 33.0, 76.14, 127.0, 127.4, 127.6, 128.8, 129.0, 134.7, 140.3, 140.5

### d) 4-(2-nitroallyl)-1,1'-biphenyl (5)

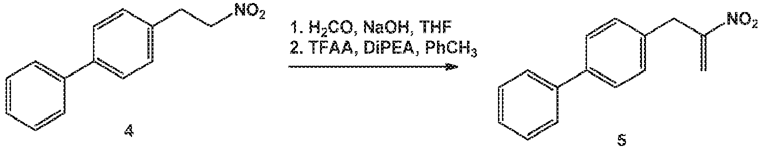

To a solution of compound 4 (10 g, 44.0 mmol) in THF (100 mL) a solution of NaOH (10%, w/w) was added, followed by a 37% aqueous solution of formaldeyde (37.5 g, 46.2 mmol) added dropwise at 0 °C. The resulting mixture was stirred at room temperature and the progress of the reaction was monitored by TLC. Brine was added and the organic layer was separated, dried over anhydrous Na₂SO₄ and concentrated under reduced pressure. The crude material was dissolved (6g of target compound estimated) in toluene and N-ethyl-N-isopropylpropan-2-amine (diisopropylamine) was added (13.2 ml, 76.96 mmol). To the resulting stirring solution 2,2,2-trifluoroacetic anhydride (3.73 g, 26.8 mmol) was added dropwise at 0 °C and the mixture stirred for 3h at room temperature. the resulting solution was purified by flash chromatography (gradient Hex → Hex:EtOAc, 8:2, v/v) to obtain 5.8 g (24.24 mmol, 55%) of compound 5
¹H-NMR (300 MHz, CDCl₃) *δ* 3.94 (s, 2 H), 5.53 (psd, 1 H), 6.56 (psd, 1 H), 7.30-7.38 (m, 3H), 7.44-7.47 (t, 2 H), 7.57-7.60 (m, 4 H).
¹³C-NMR (100 MHz, CDCl₃) *δ* 36.1, 119.0, 127.1, 127.4, 127.6, 128.8, 129.4, 134.5, 140.3, 140.6, 157.5.

### e) (2R,4S)-5-([1,1'-biphenyl]-4-yl)-2-methyl-4-nitropentanal (5)

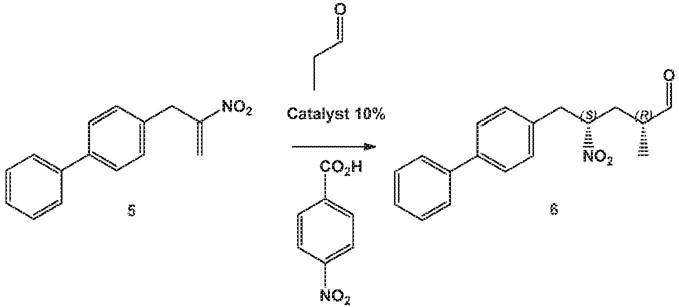

Screening conditions: To a solution of 4-(2-nitroallyl)-1,1'-biphenyl (5) (50 mg, 0.21 mmol) in toluene or DMF (1 ml), propionaldehyde (37 ∟l, 0.522 mmol) 4-nitrobenzoic acid (8.8 mg, 0.0525 mmol) and catalyst (0.021 mmol, 10mol %) were added at 10 °C. The mixture was stirred at 10 °C for 48 h.

By using (S)-(-)-α,α-Diphenyl-2-pyrrolidinemethanol tert-butyldimethylsilyl ether as catalyst a mixture of isomers enriched in compound 6 (96 % solution yield; 33% (2*R*,4*S*); 28% (2*S*,4*R*); 20% (2*S*,4*S*); 17% (2*R*,4*R*)), was obtained.

### f) Compound 6 as 2R4S isomer

¹H-NMR (400 MHz, CDCl₃) *δ* 1.19 (d, 3H, J = 7.5 Hz), 1.98-2.06 (m, 1H), 2.21 (ddd, 1H, J₁ = 3.3, J₂ = 9.8, J₃ = 14.8 Hz), 2.43-2.53 (m, 1H), 3.12 (dd, 1H, J₁ = 5.5, J₂ = 14.3 Hz), 3.31 (dd, 1H, J₁ = 8.8, J₂ = 14.3 Hz), 4.89-4.96 (m, 1H), 7.25 (psd, 2H), 7.33-7.37 (m, 1H), 7.44 (pst, 2H), 7.53-7.59 (m, 4H), 9.56 (s, 1 H).
¹³C-NMR (100 MHz, CDCl₃) δ 14.2, 33.9, 40.0, 42.9, 87.8, 126.9, 127.3, 127.5, 128.7, 129.2, 133.9, 140.4, 140.4, 202.3.
LC-MS: [MH₃O]+ = 315.2 m/z
Chiral HPLC , 9.00 (2R,4S), 10.17 (2S,4S) 12.65 (2R,4R), 14.89 min (2S,4R); Column Chiralpak ID 4.6mm ø x 250mm, 5 *µ*m Daicel Corporation, Osaka, Japan
25°C; Hept:EtOAc:CH3CN, 90:8:2

### g) (2R,4S)-5-([1,1'-biphenyl]-4-yl)-2-methyl-4-nitropentanoic acid

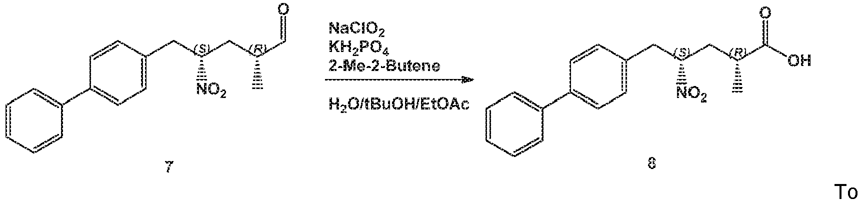

a solution of compound 6 (492 mg, 1.72 mmol) in a mixture of EtOAc/tBuOH/H₂O (1:2:1.5 v/v/v, 20 ml) was added 2-methyl-2-butene (361 mg, 5.15 mmol). The resulting mix was stirred for 1 min and KH₂PO₄ (750 mg, 5.15 mmol) and NaClO₄ (311 mg, 3.44 mmol) were added. The mixture was stirred 2 h at room temperature. HCl 3 M (25 ml) was added and the product was extracted with EtOAc (3*30 ml) and the combined organic phases were filtered on a pad of MgSO₄, and dried in *vacuum,* to obtain 508 mg (1.62 mmol, crude yield 94%) of acid 7. Compound 7 was consider pure enough and has been used for the next step without any further purification.
Compound 7 as 2R4S isomer
¹H-NMR (400 MHz, CDCl₃) *δ* 1.27 (d, 3H, J = 7.5 Hz), 2.12-2.22 (m, 2H), 2.51-2.57 (m, 1H), 3.12 (dd, 1H, J₁ = 5.5, J₂ = 14.3 Hz), 3.30 (dd, 1H, J₁ = 8.8, J₂ = 14.3 Hz), 4.92-4.97 (m, 1H), 7.25 (psd, 2H), 7.34-7.37 (m, 1H), 7.44 (pst, 2H), 7.53-7.59 (m, 4H).
¹³C-NMR (100 MHz, CDCl₃) δ 18.1, 36.0, 36.7, 40.1, 88.0, 127.0, 127.3, 127.5, 128.8, 129.3, 130.8, 140.0, 179.3.

### h) (2R,4S)-4-amino-5-([1,1'-biphenyl]-4-yl)-2-methyl-pentanoic acid chlorohydrate

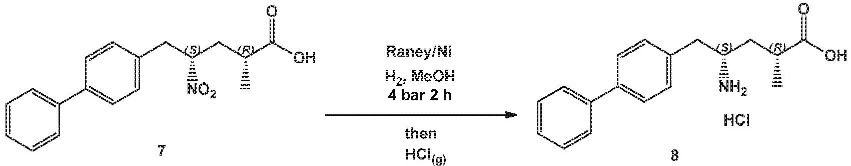

Compound 6 (considered 508 mg, 1.62 mol) was dissolved in MeOH (5 ml), and Nickel Raney (30 mg) was added (washed with MeOH 4 time). The hydrogenation was carried out in endeavour biotage equipment (4 bar, 35 °C, 2 h) and the mixture was filtered on a pad of celite and washed with MeOH. To this solution was bubbled HCl_{(g)}, in turn generated in a separated flask from NaCl and H₂SO₄. After 10 min the mixture was dried in *vacuum,* washed with DCM/Hexane 1:4 (v/v), to obtain (475 mg, 1.48 mmol) of solid in 92 % yield; Analytical data have been compared and found consistent with those reported into PCT Int. Appl. WO 2008/083967.

### Example 2: Alternative synthesis of (2R,4S)-4-amino-5-([1,1'-biphenyl]-4-yl)-2-methyl-pentanoic acid chlorohydrate

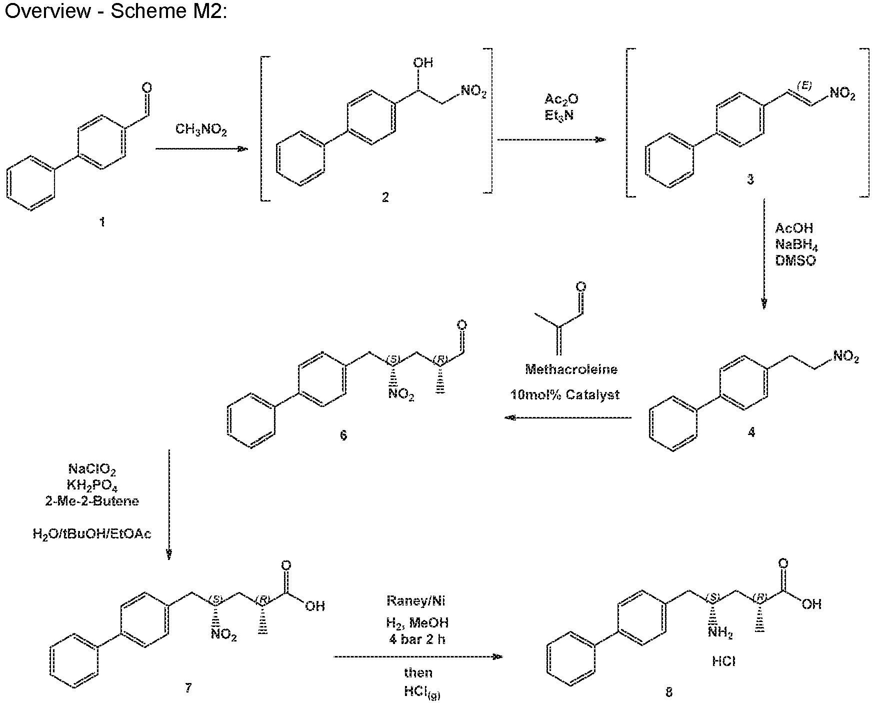

Examples of catalysts are:

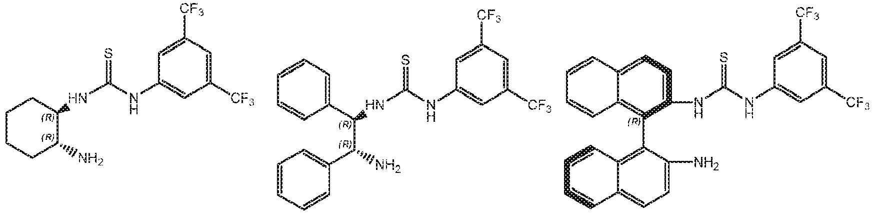

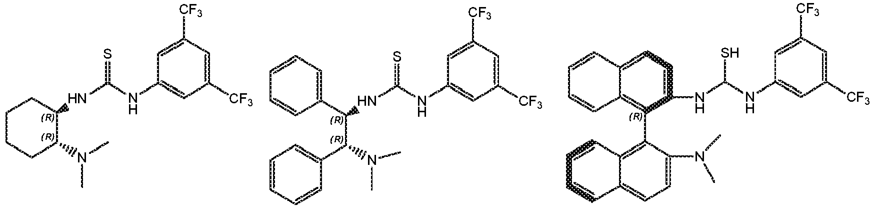

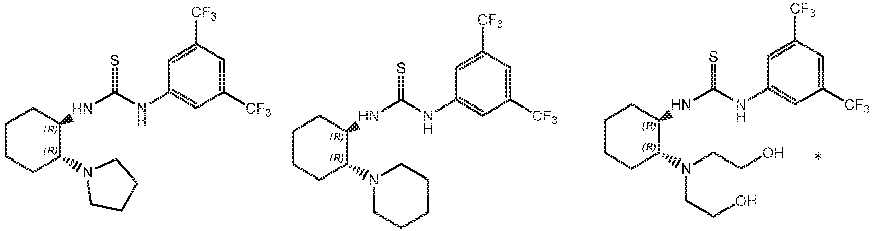

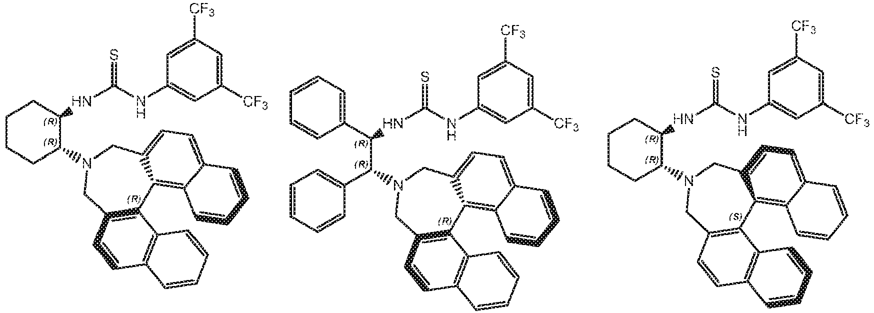

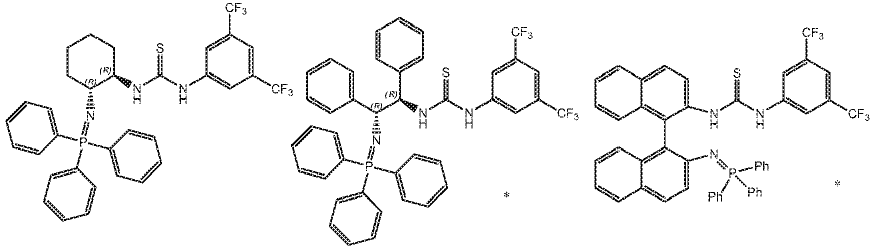

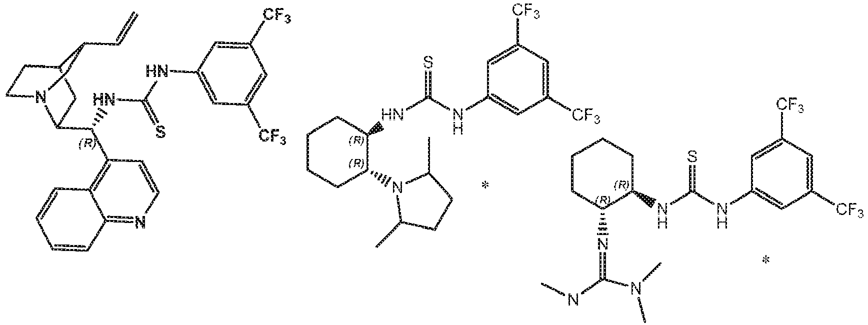

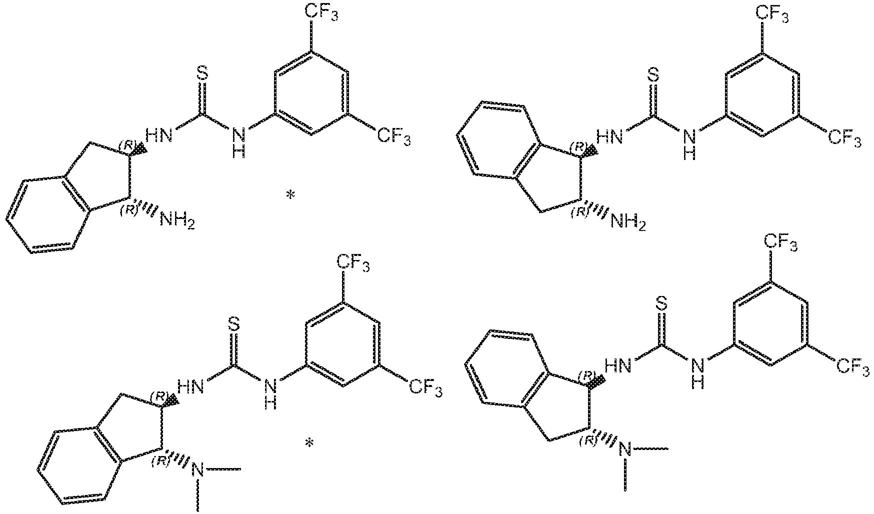

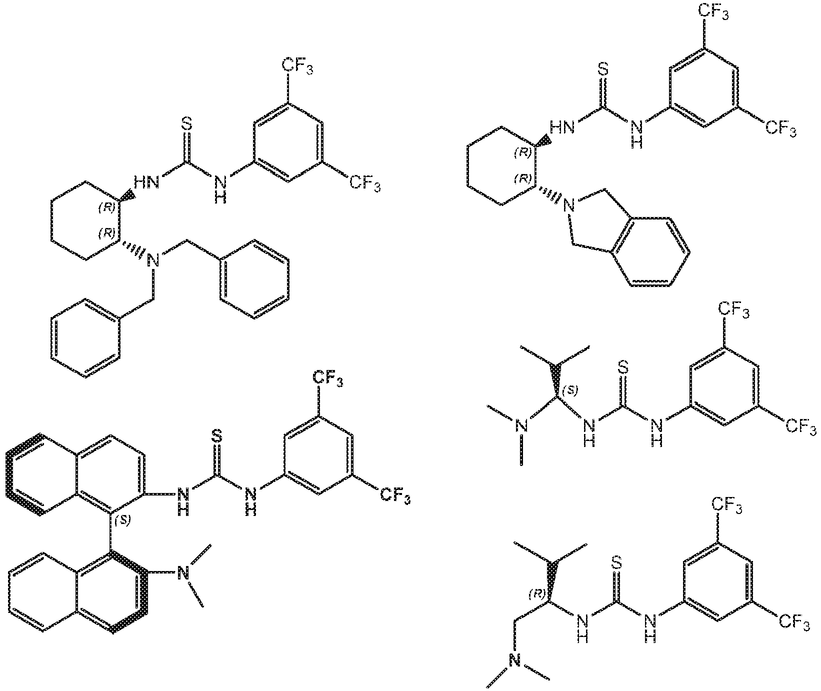

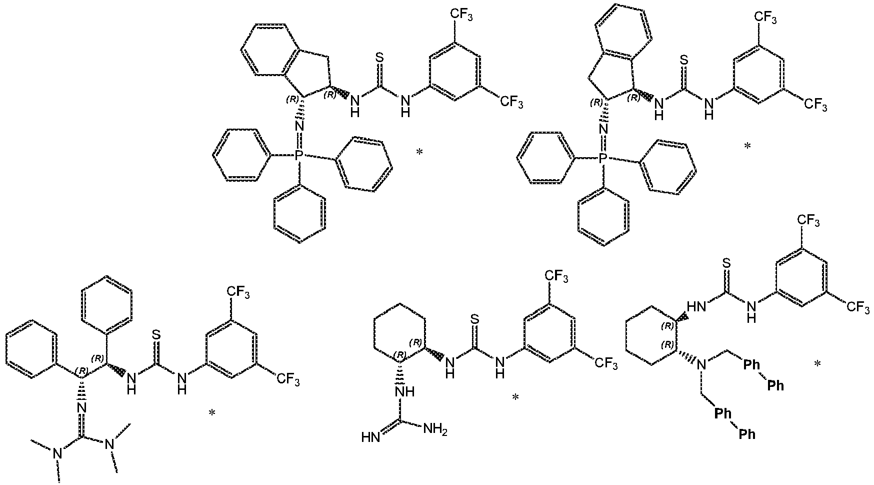

### a) 4-(2-nitroethyl)biphenyl (Compound 4)

This compound is synthesized as described in Example 1.

### b) (2R,4S)-5-([1,1'-biphenyl]-4-yl)-2-methyl-4-nitropentanal (5)

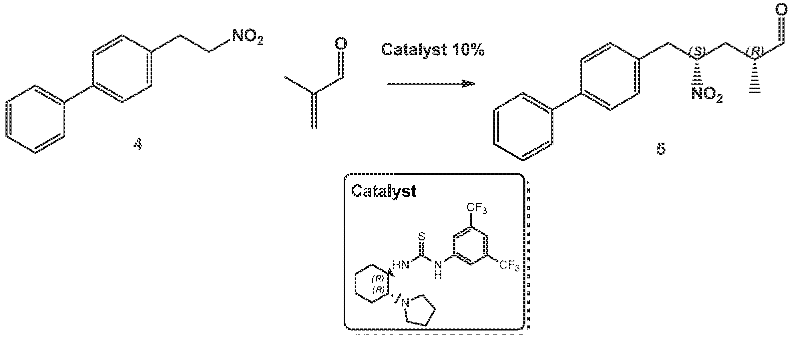

To a solution of 4-(2-nitroethyl)-1,1'-biphenyl (600 mg, 2.64 mmol) in toluene 6 ml, methacroleine (462.6 mg, 6.6 mmol) and catalyst (116 mg, 0.264 mmol, 10mol %) were added. The solution was stirred 8 h at 10 °C. The crude material was purified by column chromatography on silica-gel using 20 % ethyl acetate in heptane to afford 492 mg (1.76 mmol, 65 % yield) of a mixture of isomers enriched in compound **5** (65 % yield; 59% (2R,4S); 17% (2S,4R); 15% (2S,4S); 8% (2R,4R)), as white solid.
Compound **5** as 2R4S isomer
¹H-NMR (400 MHz, CDCl₃) *δ* 1.19 (d, 3H, J = 7.5 Hz), 1.98-2.06 (m, 1H), 2.21 (ddd, 1H, J₁ = 3.3, J₂ = 9.8, J₃ = 14.8 Hz), 2.43-2.53 (m, 1H), 3.12 (dd, 1H, J₁ = 5.5, J₂ = 14.3 Hz), 3.31 (dd, 1H, J₁ = 8.8, J₂ = 14.3 Hz), 4.89-4.96 (m, 1H), 7.25 (psd, 2H), 7.33-7.37 (m, 1H), 7.44 (pst, 2H), 7.53-7.59 (m, 4H), 9.56 (s, 1 H).
¹³C-NMR (100 MHz, CDCl₃) δ 14.2, 33.9, 40.0, 42.9, 87.8, 126.9, 127.3, 127.5, 128.7, 129.2, 133.9, 140.4, 140.4, 202.3.
LC-MS: [MH₃O]⁺ = 315.2 m/z
Chiral HPLC: 9.00 (2R,4S), 10.17 (2S,4S) 12.65 (2R,4R), 14.89 min (2S,4R); Column Chiralpak ID (Daicel Corporation, Osaka, Japan) 4.6mm ø x 250mm, 5 *µ*m;
25°C; Hept:EtOAc:CH3CN, 90:8:2

### c) (2R,4S)-4-amino-5-([1,1'-biphenyl]-4-yl)-2-methyl-pentanoic acid chlorohydrate

(2*R*,4*S*)-5-([1,1'-biphenyl]-4-yl)-2-methyl-4-nitropentanoic acid 7 and (2R,4S)-4-amino-5-([1,1'-biphenyl]-4-yl)-2-methyl-pentanoic acid chlorohydrate 8 were then synthesized as described in Example 1, step i) and step h).

Analytical data have been compared and found consistent with those reported into PCT Int. Appl. WO 2008/083967.

### Enantiomer characterization:

X-ray data available for the ester analogues:

| Compound | Structure | X-RAY | HPLC rt (min)*^{a}* |
|---|---|---|---|
| **E1** | 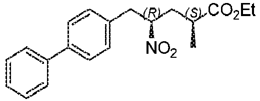 | confirmed | 9.6 |
| **E2** | 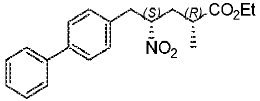 | confirmed | 5.8 |
| **E3** | 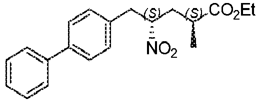 | NO X-ray availlable | 6.4 |
| **E4** | 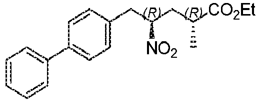 | confirmed | 13.3 |

| | | | |
|---|---|---|---|
| *^{a}*heptanes/IPA/EtOH 85:10:5 (v/v/v), flow: 1 ml/min, isocratic, column: chiralcel OD-H, 4.6 x 250 mm | | | |

### Example 3: Preparation of the nitroester isomers

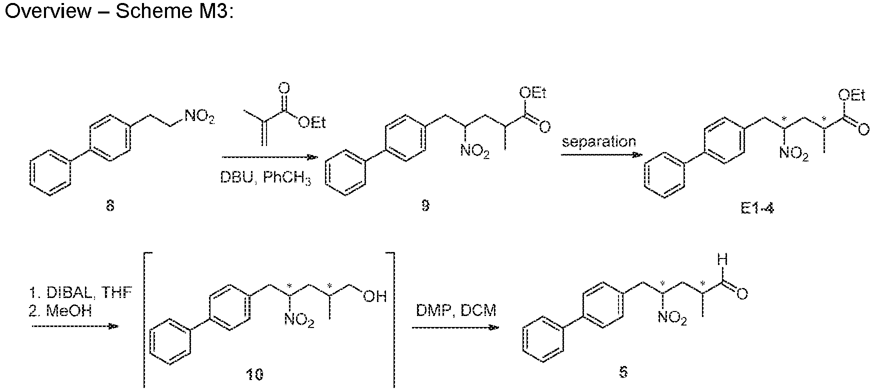

### Ethyl 5-([1,1'-biphenyl]-4-yl)-2-methyl-4-nitropentanoate (9)

To a solution of compound **8** and ethyl methacrylate (1.3 equiv.) in toluene, DBU (1 equiv.) was added dropwise and the mixture was stirred at room temperature for 48h. NH₄Cl₍ₛₛ₎ was added and the mix stirred for 5 min. The phases were separated ant the aqueous was extracted with EtOAc. The combined organic phases were dried over MgSO₄ and the solvent was removed under *vacuum* and submitted to flash chromatography.

The fractions containing the four isomers of compound **9** were further purified by preparative chromatography, (Thar SFC200) (Waters Corporation, Milford, MA, United States of America) , mobile phase: A: scCO2 ; B: MeOH, flow: 200 g/min, conditions: 5% B isocratic column: chiralpak AD-H, 50 x 250 mm **P** (Daicel Corporation, Osaka, Japan). The enantio-enriched compounds **E1**, **E2**, **E3** and **E4** have also been tested for their chiral purity with chiral HPLC, mobile phase: heptanes/IPA/EtOH 85:10:5 (v/v/v), flow: 1 ml/min, conditions: isocratic column: chiralcel OD-H, 4.6 x 250 mm (Daicel Corporation, Osaka, Japan) (rt= **E1**: 9.6 min, **E2**: 5.8 min, **E3**: 6.4 min, **E4**: 13.3 min).

The enantio-enriched isomers **E1-4** have been individually reacted with 5 equivalents of DIBAL 1M solution in THF at 0 °C. After 1 h methanol was added at the same temperature and 15 w% brine solution was added. After the phase separation the organic phase was concentrated in *vacuum.* The residue was dissolved in DCM and Dess-Martin periodinane (1.1 equiv., DMP) was added. After 45 min hexane was added and the solid filtered off. The mix was filtered on a pad of silica affording the corresponding aldehydes **5.**

The conditions for obtaining the X-ray data are as follows:

| | | |
|---|---|---|
| Temperature | 100(2) K | |
| Wavelength | 1.54178 Å | |
| Crystal system | Orthorhombic | |
| Space group | P212121 | |
| Unit cell dimensions | a = 5.448(2) Å | ∟ = 90° |
| | b = 8.286(2) Å | ∟ = 90° |
| | c = 40.777(12) Å | ∟ = 90° |
| Volume | 1840.8(10) Å³ | |
| Z | 4 | |
| Density (calculated) | 1.232 g/cm³ | |
| Absorption coefficient | 0.696 mm⁻¹ | |
| F(000) | 728 | |
| Crystal size | 0.21 x 0.11 x 0.04 mm³ | |
| Theta range for data collection | 2.17 to 66.57° | |
| Index ranges | -6<=h<=5, -9<=k<=9, -48<=l<=48 | |
| Reflections collected | 53449 | |
| Independent reflections | 3246 [R(int) = 0.0429] | |
| Completeness to theta = 66.57° | 99.5 % | |
| Absorption correction | Semi-empirical from equivalents | |
| Max. and min. transmission | 0.9727 and 0.8676 | |
| Refinement method | Full-matrix least-squares on F² | |
| Data / restraints / parameters | 3246 / 0 / 229 | |
| Goodness-of-fit on F² | 1.112 | |
| Final R indices [l>2sigma(l)] | R1 = 0.0324, wR2 = 0.0826 | |
| R indices (all data) | R1 = 0.0332, wR2 = 0.0831 | |
| Absolute structure parameter | 0.0(2) | |
| Extinction coefficient | 0.0042(3) | |
| Largest diff. peak and hole | 0.166 and -0.158 e.Å⁻³ | |

### Example 4: Manufacture of AHU377 (sacubitril)

The compound is prepared from the compound of the formula (8-a) given above as described in Ksander (Med. Chem. 1995, 38, 1689-1700; compound 18 in the publication corresponds to formula (8-a) and is converted to AHU377 and salts thereof).

Alternatively, AHU377 can be manufactured as described in WO 2008/083967.

### Example 5: Synthesis of thiourea catalysts:

The following catalysts were synthesized as described below:

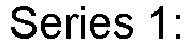

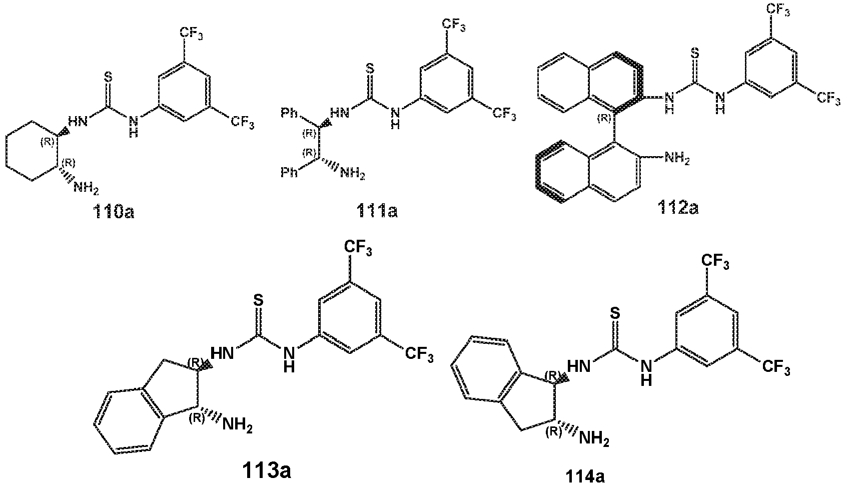

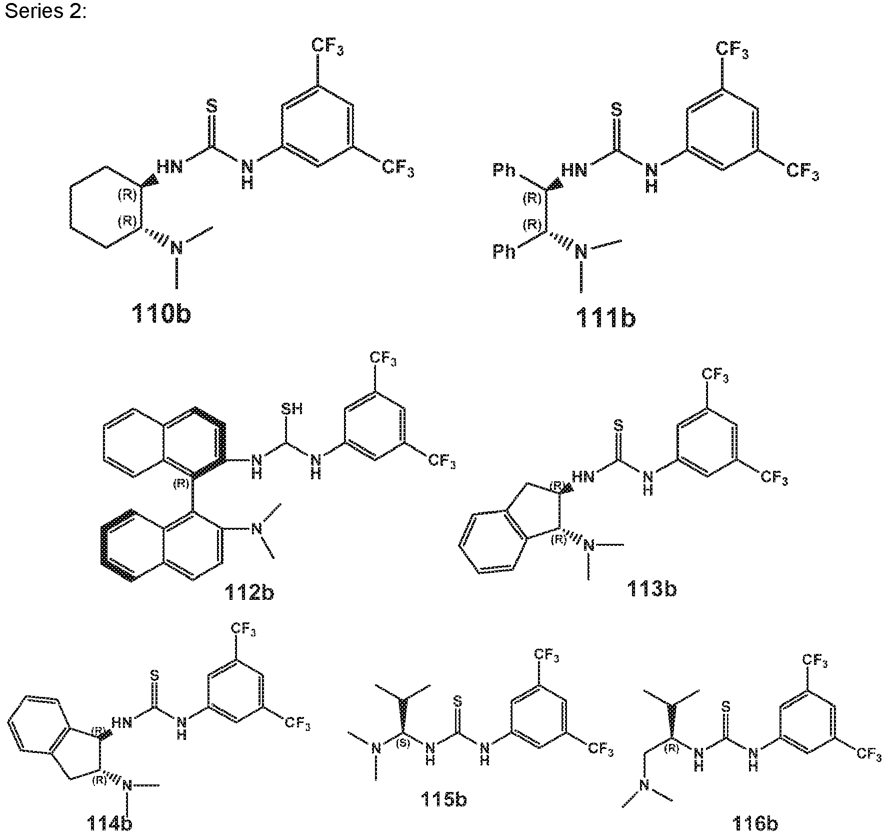

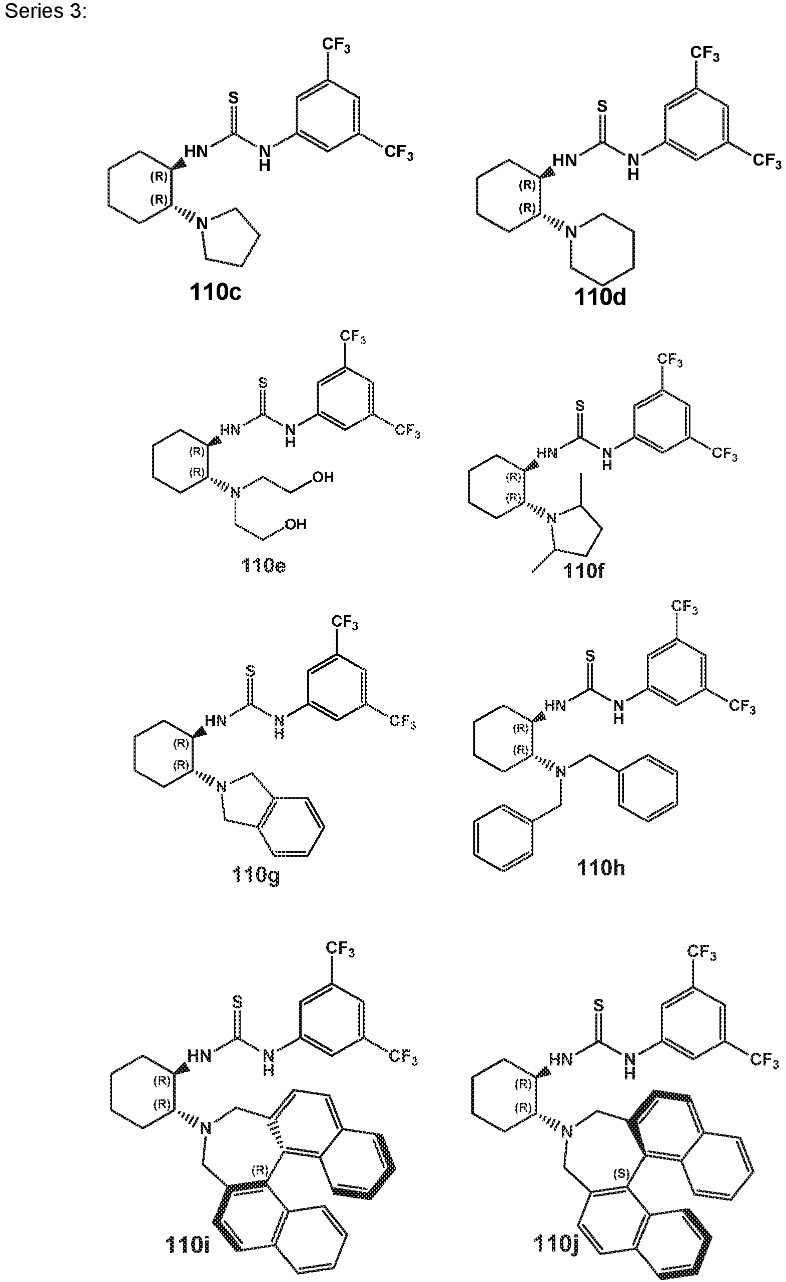

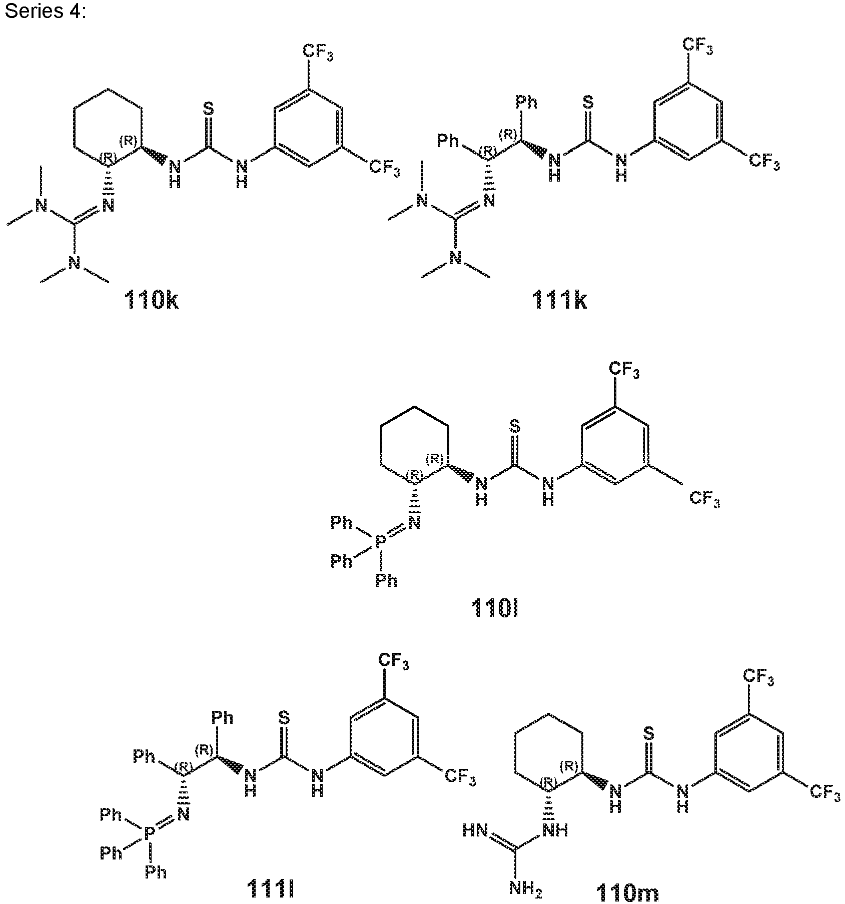

The members of series 1 can be prepared as follows:

Compounds **110a**, **111a** (Scheme A) and, **112a** (Scheme B), were prepared by reacting a THF solution of the chiral amine with an equimolar amount of 3,5-bis(trifluoromethyl)phenyl isothiocyanate (**119**). This is followed by chromatography purification of target products **110a**, **111a** and **112a** obtained in good to excellent yield.

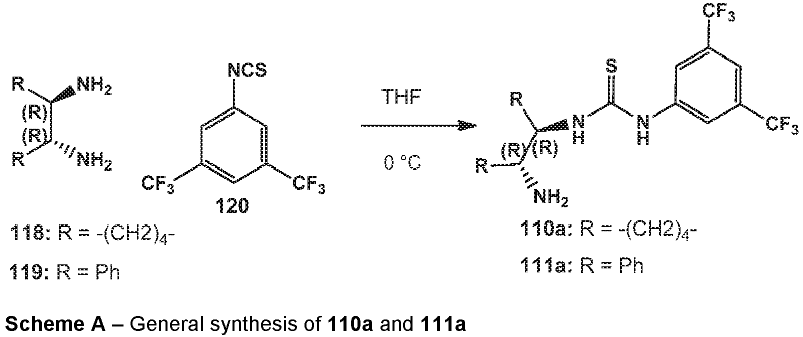

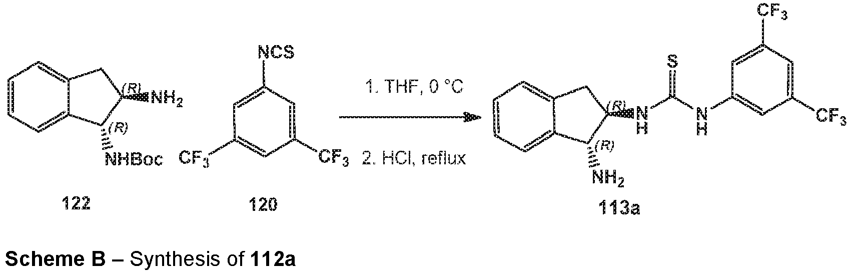

Compounds **113a** and **114a**, can easily be prepared from the common precursor **122**. **113a** obtained by reacting **122** with isothiocyanate **120**, followed by acid hydrolysis of the *tert*-butyl carbonate protecting group and basic work-up (Scheme C).

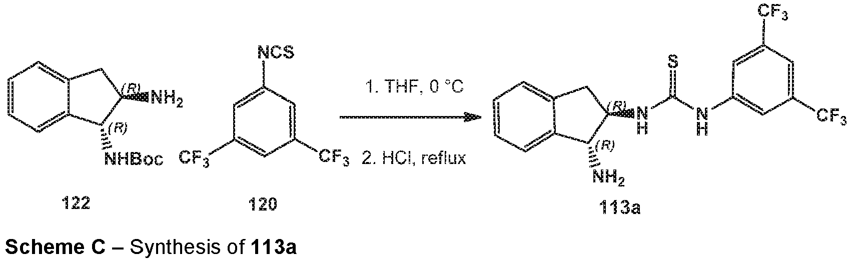

Compound **114a**, was prepared from **122** by acetylation of the free amine and hydrolysis of the Boc residue. The compound **123** thus prepared, was treated with isothiocyanate **120.** Catalyst **114a** was obtained from following acid hydrolysis and chromatography purification (Scheme D).

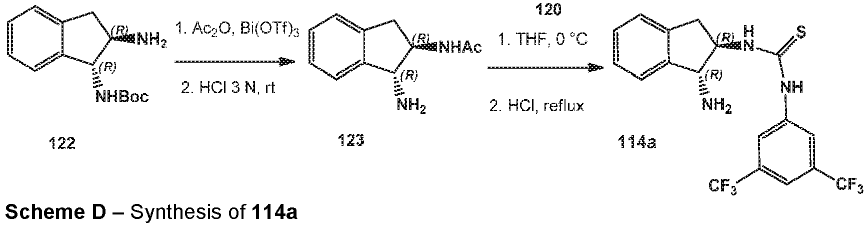

The synthesis of the catalysts belonging to series 2 is reported in Schemes E and F. The monoprotected chiral diamines **124** and **126** were initially submitted to reductive amination by treatment with formaldehyde and sodium cyanoborohydride. Compounds **125** and **127** thus obtained were deprotected by acid hydrolysis, while the thiourea function was introduced by reaction with isothiocyanate **120** using THF as solvent. The corresponding derivatives **111b** and **112b** were obtained after flash chromatography in good yield (Scheme 17).

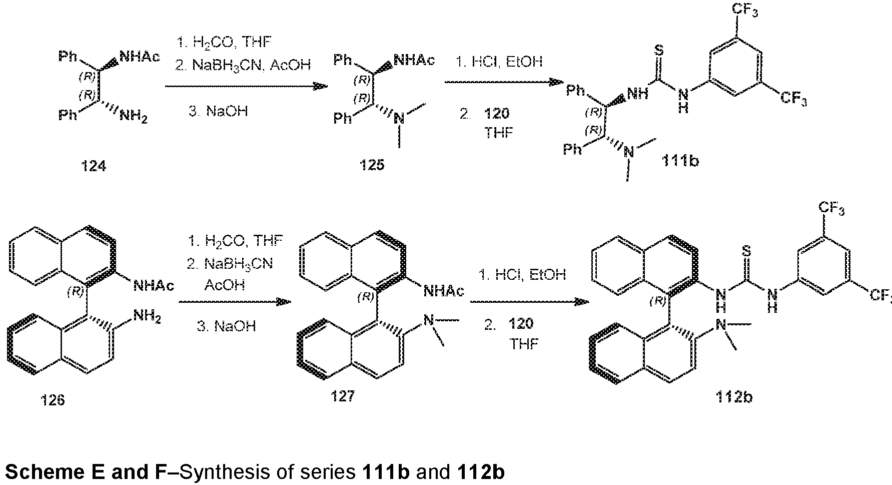

The synthesis of **114b** was performed by a similar procedure, while **113b** required a different approach. First, it was reacted with **120** in THF, and subsequently the Boc protecting group was removed by acid hydrolysis with HCl 6 N. The intermediate **128** was submitted to reductive amination by treatment with formaldehyde and sodium cyanoborohydride to obtain the product **113b** in good overall yield (Scheme G).

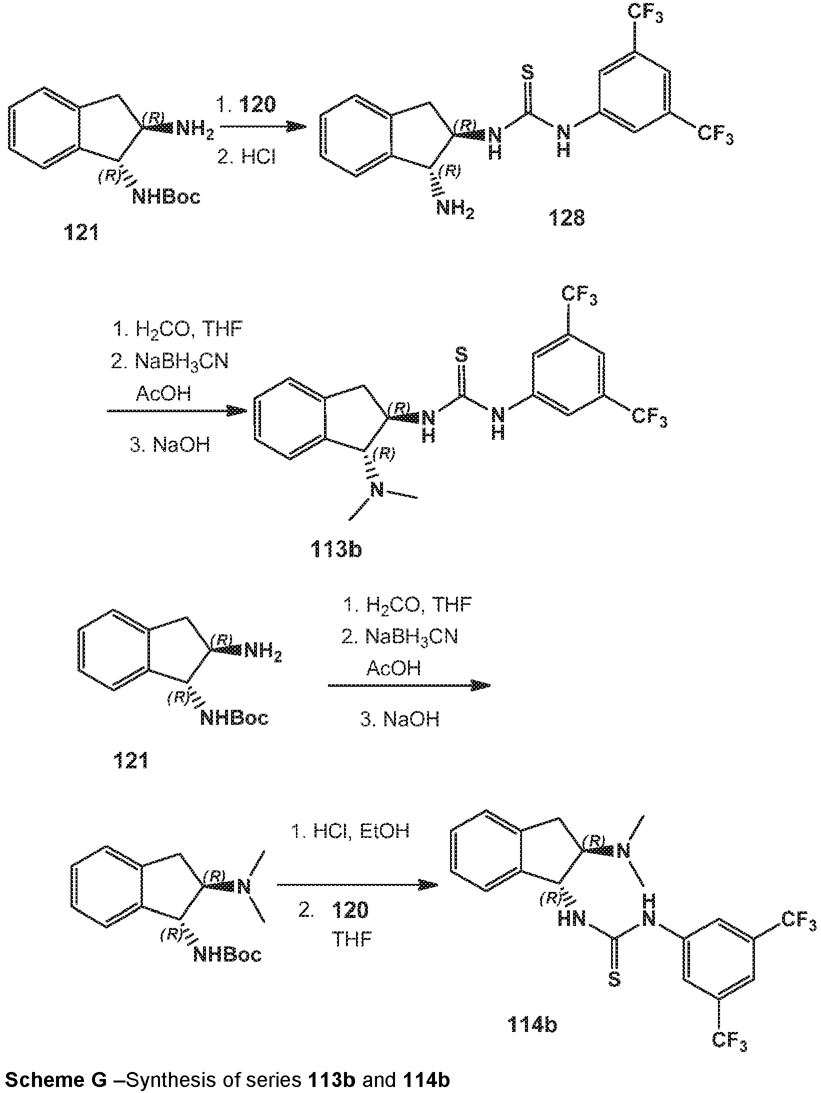

### Series 3

This series was designed combining the chiral scaffold **110** with the tertiary amines corresponding to **c-j**. The scaffold **110** and different residues on the basic nitrogen allowed us to explore a small range of pKₐ values (pKₐ ∼10), the influence of steric bulk and potential additional interaction with the substrates during the formation of the intermediate complex (e.g. hydrogen bounds).

The preparation of this series is reported in Scheme H. Starting from the benzylic protected chiral diamine **129**, the first step involves of the alkylation of the free amine with the alkyl halide in water/organic mixture at 120 °C. This is followed by deprotection of the protecting group with HCI 6N at 120 °C, leading to compound **130**. Both reactions were performed in a microwave apparatus. The last step is the reaction with isothiocyanate **120**. The pure catalysts **110c-g** were obtained by precipitation from an hexane/EtOAc mixture.

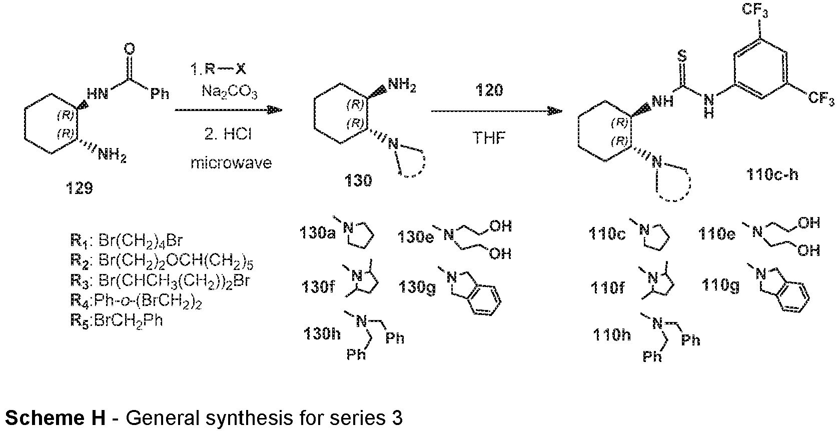

### Series 4

The last series were generated combining the chiral scaffold **110** and **111** with two different moieties: guanidine and iminophosphorane. Regarding the preparation of these compounds, the corresponding primary amines belonging to series 1 were used as starting materials. The guanidine derivatives **110k** and **111k** were synthesized from the reaction of **110a** and **111a** respectively, with N-[chloro(dimethylamino)methylene]-N-methylmethanaminium chloride (**131**) in the presence of triethylamine. Compound **131** was in turn generated by reacting tetramethylurea and oxalyl chloride under Vilsmeyer's conditions (Scheme I).

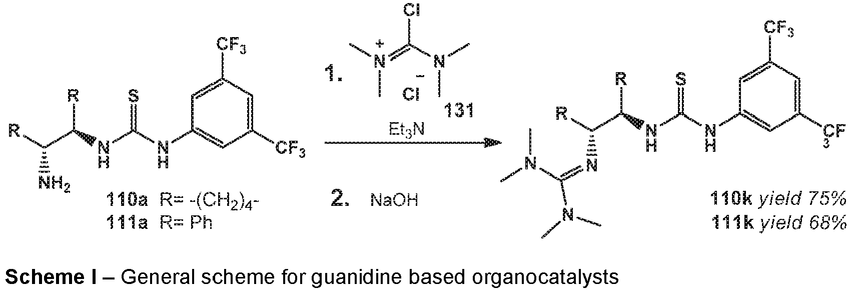

The iminophosphorane-bases **110l** and **111l** were synthesized by Staudinger reaction by employing the commercially available diazo-transfer reagent **132** and triphenylphosphine affording **110l** and **111l** respectively after filtration of the precipitated product from the reaction mixture (Scheme K).

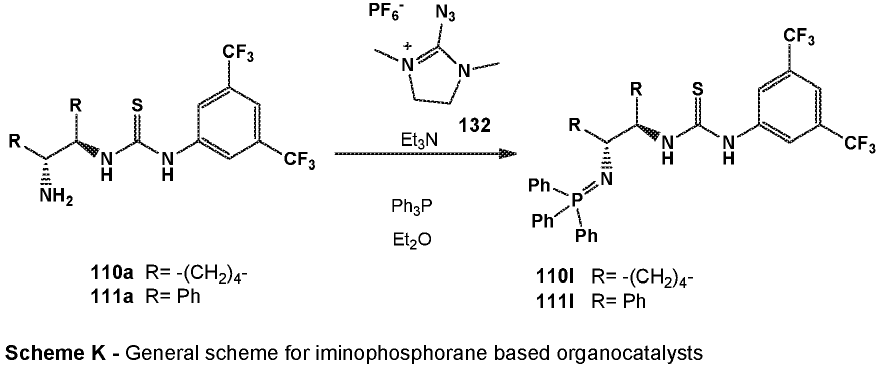

The second type of guanidine-based catalyst **110m** was synthesized by reacting the commercially available 1,3-bis(tert-butoxycarbonyl)-2-methyl-2-thiopseudourea (**133**) with derivative **110a** followed by deprotection of the Boc group (Scheme L).

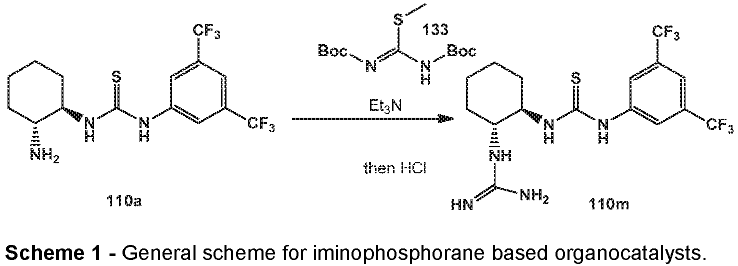

In the following, details for the synthesis of catalysts are provided:

### General methods

All used commercial chemicals were supplied by Sigma-Aldrich, Merck or Fluka, and all these reagents were used without further purification. All reaction were monitored by analytical thin layer chromatography (TLC), performed on Merck silica gel 60 F254 precoated aluminum or glass sheets (0.2 mm) and visualized with UV irradiation (254 nm). NMR were recorded on 400 MHz instruments. Chemical shift are given in δ (ppm) relative to TMS, multiplicity (s = singlet, d = doublet, t = triplet, q = quartet, m = multiplet and br = broad), relative integral, and coupling constants J (Hz). General laboratory equipment and materials like Rotavapor (Büchi), flash-chromatography systems (Biotage). Analytical high performance liquid chromatography (HPLC) and Analytic reverse phase high-performance liquid chromatography (RP-HPLC) were carried out on Agylent instruments, using chiral or RP-18 columns (see Appendix A for HPLC methods). LC-MS were performed in Acquity UPLC / ESI MS.

### Synthesis of the catalysts

### 1. General procedure for 110a, 111a and 112a

Isothiocyanato-3,5-bis(trifluoromethyl)benzene (2.95 mmol, 1 eq) was added via syringe pump (0,5 ml/min) to a stirred solution of the (1R,2R)-cyclohexane-1,2-diamine (2.95 mmol, 1 eq) in dry THF (15 mL) over a period of 30 min at 0 °C, the reaction was stirred for additional 15 h at room temperature. The solvent was removed and the residue was purified by flash chromatography on biotage apparatus.

### 110a- 1-((1R,2R)-2-aminocyclohexyl)-3-(3,5-bis(trifluoromethyl)phenyl)thiourea

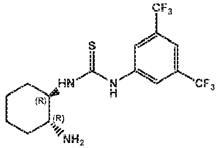

Prepared according to the general procedure 1. Purification by flash column chromatography on silica gel (DCM:MeOH, from 100:0 to 93:7, v/v) to afford 900 mg of pure product. (Purity estimated by HPLC ≥ 99%). The product was obtained as yellow solid in 80% yield
¹H NMR (400 MHz, CDCl₃): d (ppm) δ 1.25-1.34 (m, 4 H), 1.76-1.81 (m, 2 H), 1.95 (m, 1 H), 1.97-2.03 (m, 1 H), 2.07 (m, 1 H), 2.66-2.73 (m, 1 H), 3.39 (m, 1 H), 6.63 (s, 1 H), 7.58 (s, 1 H), 8.02 (s, 2H).
¹³C NMR (100 MHz, CDCl₃): (ppm) δ 24.3, 31.7, 59.4, 118.6, 121.3, 124, 132.2-133.2, 138.6, 180.4.
LCMS (ESI): exact mass calculated for [M+H]+ (C15H18F6N3S) requires m/z 386.11, found m/z 386.2.

### 111a -1-((1R,2R)-2-amino-1,2-diphenylethyl)-3-(3,5-bis(trifluoromethyl)phenyl)thiourea

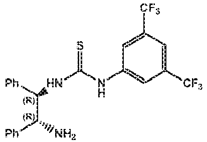

Prepared according to the general procedure 1. Purification by flash column chromatography on silica gel (DCM:MeOH, from 100:0 to 93:7, v/v) to afford 490 mg of pure product (Purity estimated by HPLC ≥ 95%). The product was obtained in 90% yield, yellow solid.
¹H NMR (400 MHz, DMSO): d (ppm) δ 4.38 (d, 1 H, J= 8 Hz), 5.50 (d, 1 H, J= 8 Hz), 7.20-7.45 (m, 10 H), 7.71 (s, 1 H), 8.32 (s, 2 H), 10.58 (s, 1 H).
¹³C NMR (100 MHz, DMSO): (ppm) δ 59.9, 63.7, 116.3, 121.5, 122.3, 125.0, 127.3, 127.4, 127.5, 128.4, 128.6, 130.5, 141.4, 142.5, 143.4, 180.6.
LCMS (ESI): exact mass calculated for [M+H]+ (C23H19F6N3S) requires m/z 483.47, found m/z 484.2.

### 112a - (R)-1-(2'-amino-[1,1'-binaphthalen]-2-yl)-3-(3,5-bis(trifluoromethyl)phenyl)thiourea

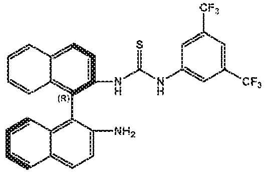

Prepared according to the general procedure 1. Purification by flash column chromatography on silica gel apparatus (Hex:EtOAc, from 100:0 to 75:25, v/v) to afford 990 mg of pure product (Purity estimated by HPLC ≥ 93%). The product was obtained in 68% yield, yellow solid.
¹H NMR (400 MHz, CDCl₃): d (ppm) δ 6.87 (dd, 1 H, J₁= 1.1 Hz, J₂= 8.3 Hz), 7.04 (d, 1 H,J= 8.8 Hz), 7.16 (ddd, 1 H, J₁= 1.5 Hz, J₂= 6.8 Hz, J₃= 8.3 Hz), 7.23 (ddd,1 H, J₁= 1.3 Hz, J₂= 6.8 Hz, J₁= 8.1 Hz), 7.39-7.35 (m, 2 H), 7.59 - 7.53 (m, 3H), 7.62 (s, 1H), 7.72 (d, 2H, J = 3.0 Hz), 7.81 - 7.74 (m, 2H), 8.00 (dt, 1H, J₁= 1.0 Hz, J₂= 8.3 Hz), 8.13 - 8.05 (m, 2H).
¹³C NMR (100 MHz, CDCl₃): (ppm) δ 76.7, 77.0, 77.2, 77.3, 11.9, 118.2, 119.7, 121.4, 122.9, 123.0, 124.1, 124.7, 124.9, 126.1, 126.9, 127.4, 127.5, 128.2, 128.4, 128.5, 129.7, 130.4, 132.2 (q), 132.7, 132.9, 133.2, 133.8, 138.7, 141.9, 180.1.
LCMS (ESI): exact mass calculated for [M+H]+ (C29H19F6N3S) requires m/z 555.2, found m/z 556.2.

### 110d - 1-(3,5-bis(trifluoromethyl)phenyl)-3-((1R,2R)-2-(piperidin-1-yl)cyclohexyl)thiourea

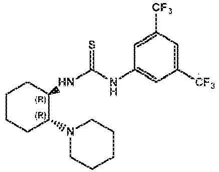

Prepared according to the general procedure 1. Purification by flash column chromatography on silica gel (DCM:MeOH, from 100:0 to 9:1, v/v) to afford 900 mg of pure product. (Purity estimated by HPLC ≥ 97%). The product was obtained as yellow solid in 76% yield.
¹H NMR (400 MHz, CDCl₃): d (ppm) δ 1.49 - 1.07 (m, 10H), 1.78 (d, 1H, J = 8.1 Hz), 1.85 (d, 1H, J= 8.1 Hz), 1.95 (d, 1H, J= 8.1 Hz), 2.36 (td, 3H, J₁ = 3.9 Hz, J₂= 11.1 Hz), 2.62 (t, 2H, J = 8.2 Hz), 2.73 (s, 1H), 3.75 (td, 1H, J₁ = 4.0 Hz, J₂= 10.5 Hz), 7.73 (s, 1H), 7.84 (s, 2H).
¹³C NMR (100 MHz, CDCl₃): (ppm) δ 32.6, 49.6, 56.3, 68.9, 118.8, 119.0, 119.1, 119.2, 121.5, 124.3, 124.6, 127.0, 132.6 (q), 139.7, 182.
LCMS (ESI): exact mass calculated for [M+H]+ (C20H25F6N3S) requires m/z 453.5, found m/z 454.3.

### 113a - 1-((1R,2R)-1-amino-2,3-dihydro-1H-inden-2-yl)-3-(3,5-bis(trifluoromethyl)phenyl) thiourea

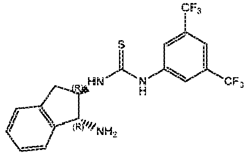

To a solution of 890 mg of **121** in 15 ml THF, a solution of isothiocyanate (1.069 g, 3.94 mmol) in 5 ml of THF at 0°C, was added drop-wise via syringe pump (0.5 ml/min), stirring 5 h (until room temperature). The crude was concentrated under reduced pressure. It was suspended on 5 ml H2O, HCl 6 N (10 ml) was added and stirred overnight. The suspension was dissolved, heating (reflux), stirred 1 h. White crystals were formed from the solution at room temperature, and they were filtered. iPr₂O (10 ml) were added to remove lipophilic impurities, and the solid was filtered. Satured solution of sodium bicarbonate (20 ml) and ethyl acetate were added, stirred 3 h. Acqueous phase was than extracted with EtOAc (2*20), and the organic phases were collected and dried with MgSO₄ and concentrated under reduced pressure. 1,15 g of pure product were obtained (69% yield), analized by NMR, HPLC and LC-MS.
¹H NMR (400 MHz, CDCl₃): d (ppm) δ 2.98 (dd, 1H, J₁=10.1 Hz, J₂= 15.3), 3.43 (dd, 1H, J₁=15.1 Hz, J₂= 8.3 Hz), 4.32 - 4.17 (m, 1H), 4.41 (d, 1H, J = 7.9 Hz), 6.96 (s, 1H), 7.45 - 7.22 (m, 4H), 7.65 (s, 1H), 8.19 (s, 2H), 12.72 (s, 1H),
¹³C NMR (100 MHz, CDCl₃): (ppm) δ 36.5, 64.5, 65.3, 121.8, 123.0, 123.5, 124.8, 128.1, 128.7, 131.7 (q), 137.9, 141.7, 143.1, 182.6.
LCMS (ESI): exact mass calculated for [M+H]+ (C20H25F6N3S) requires m/z 419.4, found m/z 420.2.

### 115a - 1-((1R,2R)-2-amino-2,3-dihydro-1H-inden-1-yl)-3-(3,5-bis(trifluoromethyl)phenyl) thiourea

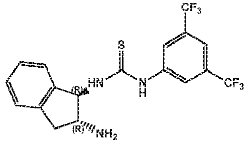

To a solution of **121** (1,0 g, 4.03 mmol) in 10 ml of dichloromethane, 0.62 ml (0.669 mg, 6.5 mmol) acetic anhydride and bismuth triflate (118 mg, 0.2 mmol) were added.

The solution turn from red to brown, stirred for 2 h. HPLC showed the formation of a product, quantitative. HCl 3N was added and stirred overnight. NaOH 2N (30 ml) was added to the aqueous phase which extracted with EtOAc (3*40 ml). The organic phase was dried with MgSO4, filtered and concentrated under reduced pressure. 800 mg of product were obtained as yellow solid. It has been used directly for the step 2 and the product from step 1 was dissolved on 15 ml of THF. A solution of isothiocyanate (1.092 mg, 4.03 mmol) in 5 ml of THF was added drop wise and stirred 2 h. The reaction mixture was concentrated under reduced pressure, then HCl 6 N (5 ml) and EtOH (10 ml) were added to the brownish solid. The suspension was dissolved at reflux, 24 h, with magnetic stirring. The reaction was monitored by HPLC. The solution was cooled at rt. and small crystals were formed. The crystals were filtered to obtain a yellow solid. The solid was dissolved in NaOH (50 ml), and the solution was extracted with EtOAc (50*3), dried with MgSO4 and concentrated under reduced pressure to obtain 710 mg of white powder (overall yield 42%). Product was analyzed by HPLC, NMR and LC-MS.
¹H NMR (400 MHz, CDCl₃): d (ppm) δ 2.69 (dd, 1H, J₁= 9.3 Hz, J₂= 15.6), 3.40 (dd, 1H, J₁= 8.2 Hz, J₂= 15.8Hz), 3.67 (q, 1H, J = 8.5 Hz), 5.06 - 4.85 (m, 1H), 6.67 (s, 1H), 7.16 - 7.08 (m, 1H), 7.41 - 7.22 (m, 2H), 7.55 (s, 1H), 8.09 (s, 2H), 12.37 (s, 1H).
¹³C NMR (100 MHz, CDCl₃): (ppm) δ 41.9, 62.9, 67.3, 117.9, 123.0, 123.3, 124.9, 128.03, 129.3, 131.8, 138.3, 139.8, 141.8, 182.6.
LCMS (ESI): exact mass calculated for [M+H]+ (C18H15F6N3S) requires m/z 419.3, found m/z 420.1.

### 2. General procedure for 111b and 112b

SM (3.93 mmol, 1 eq) was suspended in CH₃CN (20 ml), stirred 15 min, and aqueous HCOH (19.6 mmol, 5 eq) was added and stirred 15 min. The suspension became a solution and NaCNBH₃ (7.86 mmol, 2 eq) was added, stirring 5 h. AcOH (2 ml) was added and after 2 h of magnetic stirring the solution was diluite with 2% MeOH-DCM (50 ml),washed with NaOH 1 N (3*40 ml) and dried by MgSO₄ and concentrated under reduced pressure. A brownish oil was obtained and it was used directly for the step 2. It was dissolved in EtOH (15 ml) and HCl 6 N (5 ml) was added in a microwave vial (20 ml). The solution was heated at 120°C for 3 h. NaOH 2 N (40 ml) was added and the solution was extracted with EtOAc (3*30 ml), dried by MgSO₄ and concentrated under reduced pressure to obtain a brownish oil which was employed directly for the Step 3. To a solution of crude in THF (20 ml), under nitrogen atmosphere, and stirring 10 min as suspension, isothiocyanate (3.93 mmol, 1 eq) was added. After stirring 1 h, a yellow solution was obtained. The reaction mixture was concentrated under reduced pressure, to obtain a yellow oil.

### 111b - 1-(3,5-bis(trifluoromethyl)phenyl)-3-((1R,2R)-2-(dimethylamino)-1,2-diphenylethyl) thiourea

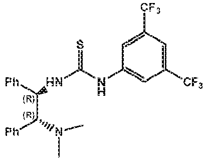

Prepared according to the general procedure 2. The crude was dissolve in EtOAc:Hex (20 ml), 2:3, v/v, stirring 1 h, until the formation of a fine precipitate which has been filtered. The liquid phase was purified by flash chromatography (biotage apparatus) (Hex:DCM from 100:0 to 8:2) to obtain 695 mg of product **111b** (overall yield 36%). The reaction product was analyzed by HPLC (≥99%), NMR and LC-MS
¹H NMR (400 MHz, CDCl₃): d (ppm) δ 2.13(s, 1 H), 3.76 (d, 1H, J = 10.9 Hz), 5.20 (s, 2H), 5.34 (s, 1H), 6.99 (dd, 2H, J₁= 2.4 Hz, J₂= 6.7 Hz), 7.05 (s, 5H), 7.17 - 7.14 (m, 2H), 7.59 (s, 1H), 7.66 (s, 2H), 8.35 (s, 2H).
¹³C NMR (100 MHz, CDCl₃): (ppm) δ 40.5, 53.4, 59.4, 74.0, 119.0, 121.6, 123.6, 124.3, 127.8, 128.6, 129.9, 131.3, 132.4, 139.1, 180.5.
LCMS (ESI): exact mass calculated for [M+H]+ (C25H23F6N3S) requires m/z 511.5, found m/z 513.2.

### 112b - (R)-1-(3,5-bis(trifluoromethyl)phenyl)-3-(2'-(dimethylamino)-[1,1'-binaphthalen]-2-yl) thiourea

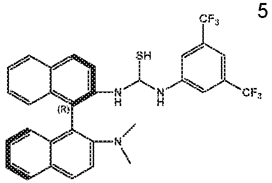

Prepared according to the general procedure2. Hex:DCM from 100:0 to 8:2 to obtain 702 mg of product C2 (overall yield 36%).The reaction product was analyzed by HPLC (≥97%), NMR and LC-MS
¹H NMR (400 MHz, CDCl₃): d (ppm) δ 2.47 (s, 6H), 5.21 (s, 2H), 6.82 (d, 1H, J = 8.5 Hz), 7.02 (ddd, 1H, J₁= 1.4 Hz, J₂= 6.6 Hz, J₃= 8.3 Hz), 7.23 - 7.13 (m, 2H), 7.34 - 7.25 (m, 2H), 7.51 - 7.38 (m, 5H), 7.64 (d, 1H, J = 8.7 Hz), 7.75 (d, 1H, J = 8.1 Hz), 7.90 (t, 2H, J = 8.0 Hz), 7.99 (d, 1H, J = 8.6 Hz), δ 8.27 (s, 1H).
¹³C NMR (100 MHz, CDCl₃): (ppm) δ 43.1, 52.4, 117.8, 117.9, 120.5, 121.9, 122.9, 123.0, 123.2, 123.9, 125.8, 126.2, 126.5, 127.4, 127.5, 128.9, 129.0, 129.5, 130.2, 130.6, 130.9, 131.0, 131.2, 131.9, 132.2, 132.3, 133.0, 138.6, 149.0, 178.8.
LCMS (ESI): exact mass calculated for [M+H]+ (C31H23F6N3S) requires m/z 583.59, found m/z 585.2.

### 113b - 1-(3,5-bis(trifluoromethyl)phenyl)-3-((1R,2R)-1-(dimethylamino)-2,3-dihydro-1H-inden-2-yl)thiourea

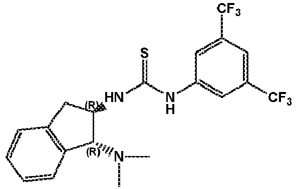

To a solution of SM (4.0 g, 16 mmol) in THF (50 ml), a solution of isothiocyanate (4.8 g, 17.7 mmol) in THF (10 ml) at 0°C was added drop-wise *via* syringe pump (0.5 ml/min). The solution was stirred 30 min util room temperature was reached. The crude was concentrated under reduced pressure and employed directly for the step 2. The crude from was suspended on H₂O (20 ml) and dissolved at reflux. HCl 6 N (10 ml) was added stirring overnight. White crystals were formed from the solution at room temperature, and they were filtered. NaOH 2 N (30 ml) solution and EtOAc (20 ml) were added; the biphasic system was stirred 30 min. Acqueous phase was than extracted with EtOAc (3*20 ml) and all organic phases were dried with MgSO₄ and concentrated under reduced pressure. 6,450 g of pure product were obtained The solid obtained from step 2 was suspended on CH₃CN (100 ml) and stirred 15 min. Aqueous HCOH (37%) (2.42 g, 80,5 mmol) was added, stirred 15 min and the suspension became a solution. NaCNBH₃ (2.025 g, 32.2 mmol)were added, stirres 6 h, and AcOH (5 ml) was added drop-wise and stirred 20 min. The reaction was dilute with 2% MeOH-DCM (100 ml), washed with NaOH 1 N (3*50 ml), dried by MgSO₄ and concentrated under reduced pressure. A yellowish solid (5.68 g) were obtained; it was dissolved on MeOH (10 ml) and precipitated as yellowish product (1,958 g, Purity ≥ 97%; 2,562 g ≥ 75% and 1.130 g ≥ 40% (by HPLC)).
¹H NMR (400 MHz, CDCl₃): d (ppm) δ 2.67 (s, 6H), 2.90 (dd, 1H, J₁=9.3 Hz, J₂₌ 15.7 Hz), 3.47 (dd, 1H, J₁=8.8 Hz, J₂₌ 15.7 Hz), 4.50 - 4.36 (m, 1H), 4.52 (d, 1H, J = 7.4 Hz), 6.83 - 6.68 (m, 1H), 7.45 - 7.28 (m, 4H), 7.65 (s, 1H), 8.12 (s, 2H), 13.05 (s, 1H).
¹³C NMR (100 MHz, CDCl₃): (ppm) δ 36.7, 41.4, 60.0, 117.9, 121.8, 123.1, 124.5, 125.5, 127.2, 128.8, 131.7, 136.9, 139.3, 141.9, 182.6
LCMS (ESI): exact mass calculated for [M+H]+ (C20H19F6N3S) requires m/z 447.4, found m/z 448.2.

### 114b - 1-(3,5-bis(trifluoromethyl)phenyl)-3-((1R,2R)-2-(dimethylamino)-2,3-dihydro-1H-inden-1-yl)thiourea

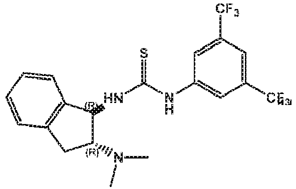

To a solution of SM (1.5 g, 6.04 mmol) in CH3CN (30 ml), acqueous formaldehyde (37%) (3 ml) was added at room temperature, stirring 15 min. NaBH3CN (760 mg, 12.08 mmol) was added to the solution and it was stirred 2 h. AcOH (2 ml) was added and the solution was stirred for 2 h. Afterwards, the reaction was diluted with 2% MeOH-DCM (80 ml), washed with NaOH 1 N (3*50) and dried by MgSO4 and concentrated under reduced pressure. Brownish oil was obtained and it was employed directly for the step 2. The crude from step 1 was dissolved in EtOH (10 ml) and HCl 6 N (5 ml) was added in a microwave vial (20 ml). The solution was heated at 120°C for 45 minutes. NaOH 2 N (40 ml) was added and the solution was extracted with EtOAc (25 ml *3), dried by MgSO4 and concentrated under reduced pressure to obtain a brownish oil which was employed directly for the Step 3. To a solution of crude from step 2 in THF (10 ml), under nitrogen atmosphere, and stirring 10 min as suspension, isothiocyanate (1.64 g, 6.04 mmol) was added. After stirring 30 min, a yellow solution was obtained. The reaction mixture was concentrated under reduced pressure, and purified by flash chromatography on biotage apparatus (Biotage EU Customer Service, Uppsala, Sweden) (DCM:MeOH from 100:0 to 98:2). 1.5 g of pure product were obtained as white powder (overall yield 56%)

The reaction product was analyzed by HPLC (≥97%), NMR and LC-MS
¹H NMR (400 MHz, CDCl₃): d (ppm) δ 2.45 (s, 6H), 3.11 - 2.89 (m, 2H), 3.69 (q, 1H, J = 8.5 Hz), 5.14 (dd, 1H, J₁= 4.5 Hz, J₂= 7.6 Hz), 6.59 (d, 1H, J = 4.0 Hz), 7.20 - 7.23 (m, 2H), 7.26 - 7.29 (m, 2H), 7.40 - 7.32 (m, 1H), 7.55 (t, 1H, J = 1.5 Hz), 8.03 (d, 2H, J = 1.7 Hz), 12.71 (s, 1H).
¹³C NMR (100 MHz, CDCl₃): (ppm) δ.25.5, 40.9, 53.4, 62.6, 75.6, 117.8, 122.8, 123.5, 125.7, 128.0, 129.5, 131.7, 137.6, 139.9, 142.0, 182.5.

### 3. General procedure for series 3

**128** (4.58 mmol, 1 eq), **R-X**(11.9 mmol, 2.5 eq), K₂CO₃ (11.9 mmol, 2.5 eq) and water (18 ml) were added in a microwave vial. The reaction tube was placed in a microwave synthesis system and operated at 120°C for 35 min. After completion of the reaction (monitored by HPLC and LC-MS), the organic portion was extracted into EtOAc (3*20 ml) and the solvent was removed under reduced pressure to obtain a yellowish solid, which was employed directly for the step 2 The crude and HCl 6 N (10 ml) were added in a microwave vial (10-20 ml). The reaction tube was placed in a microwave synthesis system and operated at 150 °C for 1 h. After completion of the reaction (monitored by HPLC and LC-MS), the organic portion was extracted into DCM (3*10 ml) (HPLC showed the presence of benzoic acid). The organic phase was wash with HCl 2 N (3*10 ml) and the aqueous phase (3*15 ml) was concentrate under reduced pressure and washed with MeOH, to obtain yellowish oil, which was used directly for the step 3. To the crude from step 2, THF (30 ml) and Et3N (1.5 ml) were added to obtain a white suspension and let stir for 30 min. 3,5-bis-trifluoromethyl-isothiocianate (4.58 mmol, 1 eq) in THF (15 ml) was added drop-wise and stirred 2 h. The reaction was monitored by HPLC and LC-MS. The reaction mixture was filtered to remove triethylammonium salts; the organic phase was washed with NaOH 1 N (30 ml) and concentrated under reduced pressure to obtain a brownish oil. The crude was purified by flash chromatography on Biotage apparatus.

### 110c - 1-(3,5-bis(trifluoromethyl)phenyl)-3-((1R,2R)-2-(pyrrolidin-1-yl)cyclohexyl)thiourea

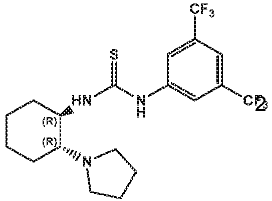

Prepared according to the general procedure 3. Purification by flash column chromatography on silica gel apparatus, DCM:MeOH from 100:0 to 95:5, to obtain 921 mg of final product. Overall yield 46%. (Purity ≥ 98%, estimated by HPLC). The reaction has been monitored by NMR and LC-MS.
¹H NMR (400 MHz, DMSO-*d₆*): d (ppm) δ 1.37 - 1.14 (m, 4H), 1.53 - 1.38 (m, 1H), 1.70 - 1.57 (m, 1H), 1.85 - 1.70 (m, 5H), 1.97 - 1.87 (m, 1H), 2.14 - 2.02 (m, 1H), 2.89 (s, 4H), 4.39 (s, 1H), 7.72 (s, 1H), 8.27 (s, 2H), 8.50 (s, 1H), 10.51 (s, 1H). ¹³C NMR (100 MHz, DMSO-*d₆*): (ppm) δ 34.5, 47.2, 53.3, 118.4, 119.1, 119.2, 119.4, 121.7, 125.2, 127.1, 127.5, 132.4, 138.6, 182.
LCMS (ESI): exact mass calculated for [M+H]+ (C19H23F6N3S) requires m/z 439.5, found m/z 441.2.

### 110e - 1-((1R,2R)-2-(bis(2-hydroxyethyl)amino)cyclohexyl)-3-(3,5-bis(trifluoromethyl) phenyl)thiourea

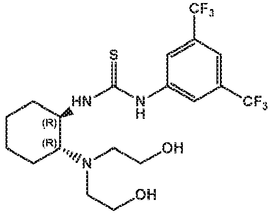

Prepared according to the general procedure 3. Purification by flash column chromatography on silica gel apparatus Hep:EtOAc from100:0 to 8:2, to obtain 633 mg of final product. Overall yield 30% (Purity ≥ 95%, estimated by HPLC). The reaction has been monitored by NMR and LC-MS.
¹H NMR (400 MHz, DMSO-*d₆*): d (ppm) δ 1.30 - 1.04 (m, 4H), 1.67 - 1.55 (m, 1H), 1.80 - 1.68 (m, 1H), 1.92 - 1.82 (m, 1H), 2.30 (s, 1H), 2.74 - 2.56 (m, 3H), 3.47 - 3.33 (m, 4H), 4.26 (s, 2H), 7.73 (s, 1H), 7.83 (s, 1H), 8.27 (s, 2H), 10.00 (s, 1H).
¹³C NMR (100 MHz, DMSO-*d₆*): (ppm) δ 24.5, 25.5, 32.5, 53.4, 55.6, 61.0, 116.4, 122.4, 125.1, 130.7, 142.5, 179.8.
LCMS (ESI): exact mass calculated for [M+H]+ (C19H25F6N3O2S) requires m/z 473.2, found m/z 474.3.

### 110f - 1-(3,5-bis(trifluoromethyl)phenyl)-3-((1R,2R)-2-(2,5-dimethylpyrrolidin-1-yl)cyclohexyl) thiourea

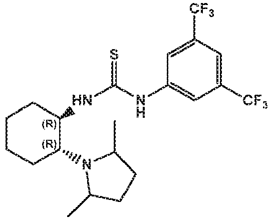

Prepared according to the general procedure 3. Purification by flash column chromatography on silica gel apparatus,DCM:MeOH from 100:0 to 95:5, to obtain 921 mg of final product. Overall yield 46%. Overall yield 30% (Purity ≥ 98%, estimated by HPLC). The reaction has been monitored by NMR and LC-MS.
¹H NMR (400 MHz, DMSO-*d₆*): d (ppm) δ 0.89 (s, 3 H), 0.91 (s, 3 H), 1,04 - 1,23 (m, 4 H), 1.26 - 1.42 (m, 3 H), 1.71 - 1.82 (m, 4 H), 2.02 (m, 1 H), 2.75 (dt, J₁=7.5 Hz, J₂₌ 28.2 Hz, 1 H), 2.82 - 2.91 (m, 1 H), 3.19 (m, 2 H), 3.96 (dt, J₁=7.5 Hz, J₂₌ 26.9 Hz, 1 H), 7.74 (s, 3 H)
¹³C NMR (100 MHz, DMSO-*d₆*): (ppm) δ 19.9, 24.7, 26.0, 27.3, 31.1, 32.8, 57.0, 58.9, 120.3, 122.1, 124, 9, 125.0, 133.9, 139.5, 181.5.

### 110g - 1-(3,5-bis(trifluoromethyl)phenyl)-3-((1R,2R)-2-(isoindolin-2-yl)cyclohexyl)thiourea

Prepared according to the general procedure 3. Purification by flash column chromatography on silica gel DCM:MeOH from100:0 to 95:5, to obtain 521 mg of final product.

Overall yield 24% (Purity ≥ 96%, estimated by HPLC). The reaction has been monitored by NMR and LC-MS. 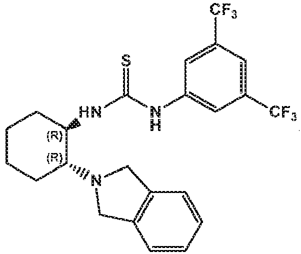 ¹H NMR (400 MHz, DMSO-*d₆*): d (ppm) δ 1.40 - 1.13 (m, 5H), 1.55 - 1.40 (m, 1H), 1.71 - 1.58 (m, 1H), 1.77 (d, J = 9.1 Hz, 1H), 1.90 (d, 1H), 2.18 (d, J = 97.5 Hz, 1H), 3.03 - 2.77 (m, 1H), 4.23 - 3.91 (m, 4H), 4.33 (s, 1H), 7.22 (ddd, J₁=3.2 Hz, J₂₌ 5.6 Hz, J₃₌ 22.4, 4H), 7.67 (s, 1H), 8.17 (s, 2H), 8.29 (d, J = 7.8 Hz, 1H), 10.03 (s, 1H).
¹³C NMR (100 MHz, CDCl₃): (ppm) δ 23.8, 27.1, 25.7 32.7, 53.6, 55.3, 61.4, 119.9, 121.1, 127.5, 128.6, 132.1, 133.2, 139.9, 180.7.
LCMS (ESI): exact mass calculated for [M+H]+ (C23H23F6N3S) requires m/z 487.5, found m/z 489.2.

### 110h- 1-(3,5-bis(trifluoromethyl)phenyl)-3-((1R,2R)-2-(dibenzylamino)cyclohexyl)thiourea

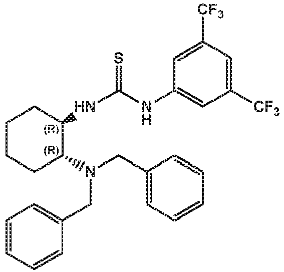

Prepared according to the general procedure 3. Purification by flash column chromatography on silica gel Hep:EtOAc from 100:0 to 8:2, to obtain 988 mg of final product. Overall yield 39% (Purity ≥ 95%, estimated by HPLC). The reaction has been monitored by NMR and LC-MS.
¹H NMR (400 MHz, CDCl₃): d (ppm) δ 0.88 (qd, 1H, J₁=3.9 Hz, J₂= 13.3 Hz), 1.08 (qt, 1H, J₁= 3.6 Hz, J₂= 13.1 Hz), 1.34 - 1.23 (m, 2H), 1.63 (d, 1H, J = 13.4, 3.2 Hz), 1.79 (d, 1H, J = 12.0 Hz), 2.04 (d, 1H, J = 12.2 Hz), 2.33 (td, 1 H, J₁=3.3 Hz, J₂= 11.3 Hz), 2.55 (d, 1H, J = 11.8 Hz), 3.25 (d, 2H, J = 13.1 Hz), 3.68 (d, 2H, J = 13.2 Hz), 4.13 - 3.99 (m, 1H), 6.16-6.00 (m, 1H), 7.04 - 6.94 (m, 4H), 7.15 - 7.04 (m, 6H), 7.61 (s, 2H), 7.65 (s, 1H),
¹³C NMR (100 MHz, CDCl₃): (ppm) δ 23.2, 24.5, 25.3 32.3, 53.3, 55.8, 61.0, 119.3, 121.4, 124.0, 127.2, 128.4, 129.8, 132.5, 132.8, 133.5, 139.5, 180.7.
LCMS (ESI): exact mass calculated for [M+H]+ (C29H29F6N3S) requires m/z 565.8, found m/z 568.3.

### 4. General procedure for 110k and 111k

**110a** (5 mmol, 1 eq) was dissolved in ACNL (10 ml) and stirred 15 min. Reagent **130** (1.2 eq) was dissolved in ACNL (12 ml) and added drop-wise at -10 °C, stirring for 3 h. NaOH (2 eq) in water (20 ml), and EtOAc (30 ml) were added and stirred 10 min. The organic phase was dried over MgSO₄ and concentrated under reduced pressure.

### 130 - N-(chloro(dimethylamino)methylene)-N-methylmethanaminium chloride

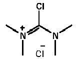

The synthesis of the guanidine-based organocatalysts was performed preparing first the reagent **130** under Vilsmeyer's condition, and it was then employed in synthesis of A12 and B12. To a solution of tetramethylurea (0.71 mmol, 1 eq) in Et₂O (5 ml), stirred 5 min, oxalylcloride (3.55 mmol, 5 eq) dissolved in Et₂O (5 ml) was added drop-wise. The solution was stirred overnight and the product precipitated as white solid. The solid has been washed with Et2O (3*15ml), without getting dry.

### 110k - 1-((1R,2R)-2-((bis(dimethylamino)methylene)amino)cyclohexyl)-3-(3,5-bis (trifluoromethyl)phenyl)thiourea

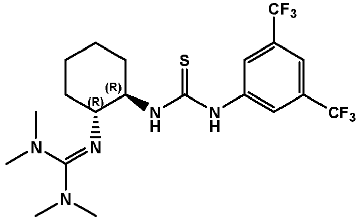

Prepared according to the general procedure 4. The crude was purified by RP-flash chromatography (H₂O:ACNL, from 8:2 to 1:1) To obtain 262 mg of **110k** in 78% yield (purity> 93% estimated by HPLC)
¹H NMR (400 MHz, CDCl₃): d (ppm) δ 1.25-1.34 (m, 4 H), 1.76-1.81 (m, 2 H), 1.95 (m, 1 H), 1.97-2.03 (m, 1 H), 2.07 (m, 1 H), 2.72 (m, 12 H), 3.39 (m, 1 H), 6.63 (s, 1 H), 7.58 (s, 1 H), 8.02 (s, 2H).
¹³C NMR (100 MHz, CDCl₃): (ppm) δ 24.3, 31.7, 39.2, 59.4, 118.6, 121.3, 124, 132.2-133.2, 138.6, 180.4.

### 111k - 1-((1R,2R)-2-((bis(dimethylamino)methylene)amino)-1,2-diphenylethyl)-3-(3,5-bis (trifluoromethyl)phenyl)thiourea

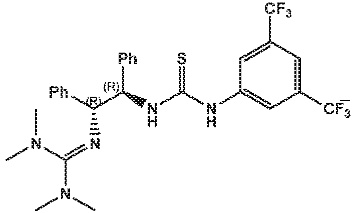

Prepared according to the general procedure 4.

The crude was purified by RP-flash chromatography (H₂O:ACNL, from 8:2 to 1:1) To obtain 149 mg of **110k** in 63% yield (purity> 96% estimated by HPLC)
¹H NMR (400 MHz, DMSO): d (ppm) δ 2.65 (s, 12 H), 4.38 (d, 1 H, J= 8 Hz), 5.50 (d, 1 H, J= 8 Hz), 7.20-7.45 (m, 10 H), 7.71 (s, 1 H), 8.32 (s, 2 H), 10.58 (s, 1 H).
¹³C NMR (100 MHz, DMSO): (ppm) δ 34.4, 59.9, 63.7, 116.3, 121.5, 122.3, 125.0, 127.3, 127.4, 127.5, 128.4, 128.6, 130.5, 141.4, 142.5, 143.4, 180.6.

### 5. General procedure 110l and 111l

To a solution of 110a (0.4 mmol, 1 eq) dissolved in DCM (20 ml) 2-azido-4,5-dihydro-1,3-dimethyl-1H-imidazolium hexafluorophosphate (0.46 mmol, 1.15 eq) and triethylamine (2 mmol, 5 eq) were added and the solution was stirred 30 min. The reaction was quenched with NaHCO₃ (20 ml), then extracted with DCM (3*20 ml), washed with water, satured brine, and dried over MgSO₄. The organic phase has been concentrated, without reaching dryness. Et₂O (5 ml) was added and a solution was formed. Triphenylphosphine (0.4 mmol, 1 eq) was dissolved in Et₂O (5 ml) and added drop-wise to the reaction. The solid formed was dissolved in MeOH and purified by SFC.

### 110l 1-(3,5-bis(trifluoromethyl)phenyl)-3-((1R,2R)-2-((triphenylphosphoranylidene)amino) cyclohexyl)thiourea

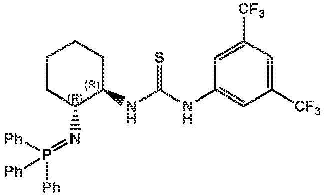

Prepared according to the general procedure 5.
¹H NMR (400 MHz, CDCl₃): d (ppm) δ 1.08 - 0.90 (m, 1H), 1.40 - 1.15 (m, 4H), 1.59 - 1.41 (m, 3H), 1.75 - 1.63 (m, 1H), 2.11 - 1.98 (m, 1H), 2.94 - 2.79 (m, 1H), 3.84 - 3.67 (m, 1H), 7.38 - 7.31 (m, 1H), 7.76 - 7.43 (m, 18H),
¹³C NMR (100 MHz, CDCl₃): (ppm) δ 24.2, 25.0, 32.8, 37.5, 62.1, 66.0, 115.6, 121.9, 124.6, 128.4, 130.9, 131.9, 132.9, 144.0, 183.2
LCMS (ESI): exact mass calculated for [M+H]+ (C33H30F6N3PS) requires m/z 645.6, found m/z 646.5.

### 110l - 1-(3,5-bis(trifluoromethyl)phenyl)-3-((1R,2R)-1,2-diphenyl-2-((triphenylphosphorany lidene)amino)ethyl)thiourea

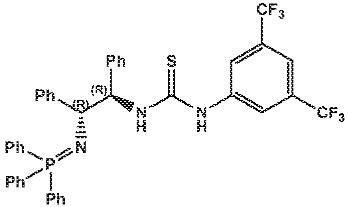

Prepared according to the general procedure 5.
¹H NMR (400 MHz, CDCl₃): d (ppm) δ 4.43 (dd, 1H, J₁= 8.8 Hz, J₂= 15.7 Hz), 5.06 (d, 1H, J = 8.6 Hz), 7.01 - 6.84 (m, 7H), 7.22 - 7.05 (m, 5H), 7.63 - 7.37 (m, 25H), 8.23 (s, 1H)
¹³C NMR (100 MHz, CDCl₃): (ppm) δ 60.7, 69.2, 116.5, 123.2, 123.7, 124.6, 125.6, 126.4, 128.4, 128.6, 128.7, 129.2, 131.9, 133.0, 140.4, 144.1, 157.2, 178.2.
LCMS (ESI): exact mass calculated for [M+H]+ (C41H32F6N3PS) requires m/z 747.7, found m/z 748.9.

### 110m - 1-(3,5-bis(trifluoromethyl)phenyl)-3-((1R,2R)-2-guanidinocyclohexyl)thiourea

SM (1g, 2.595 mmol) and DIPEA (0.671 g, 5.19 mmol) were dissolved in DCM (25 ml), stirred 10 min. 1,3-Bis(tert-butoxycarbonyl)-2-methyl-2-thiopseudourea (1.5g, 5.19 mmol) were dissolved in DCM (25 ml) and added drop-wise to the mixture and stirred 16 h, reflux. The crude has been washed with water, dried with MgSO4, and concentrated under reduced pressure. The intermediate 1 was dissolved in EtOH (15 ml) and HCl 6 N (10 ml) were added, stirring 3 h, reflux. NaOH 2 N (60ml) was added and stir 10 min. The mixture was extracted 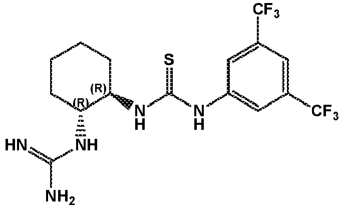 with EtOAc (3*40 ml), washed with brine, dried with MgSO4 and concentrated under reduced pressure.

The mixture was purified by flash chromatography to obtain 935 mg of product in 84% overall yield (Purity estimated by HPLC > 97%).
¹H NMR (400 MHz, CDCl₃): d (ppm) δ 1.06-1.18 (m, 1 H), 1.26-1.42 (m, 2 H), 1.50-1.65 (m, 5 H), 3.7-3.75 (m, 1 H), 3.82-3.89 (m, 1 H), 7.95 (s, 1 H), 8.01 (s, 2 H)
¹³C NMR (100 MHz, CDCl₃): (ppm) δ 29.3, 29.8, 55.5, 56.9, 118.9, 119.9, 121.6, 122.6, 124.3, 126.9, 131.9, 139.6, 156.7, 179.3.
LCMS (ESI): exact mass calculated for [M+H]+ (C16H19F6N5S) requires m/z 427.4, found m/z 429.1.

## Claims

1. A compound of formula (1), or a salt thereof 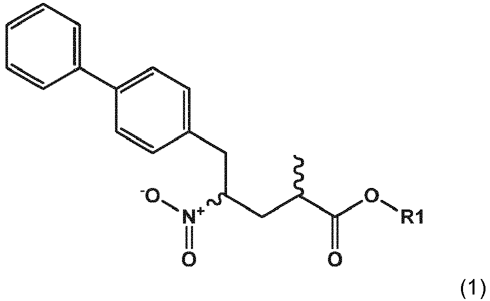 wherein R1 is hydrogen or C₁-C₆-alkyl.

2. A compound of formula (2) or a salt thereof,

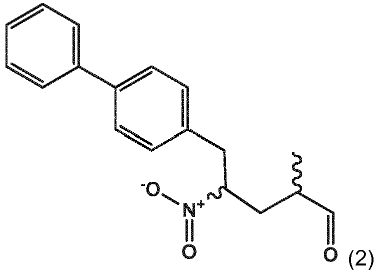

3. A compound of the formula (7),

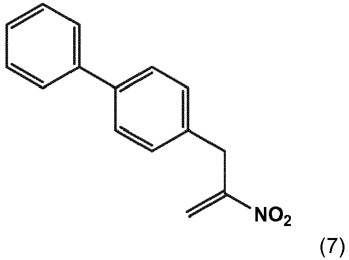

4. A process for the manufacture of the compound of the formula (1), or a salt thereof, 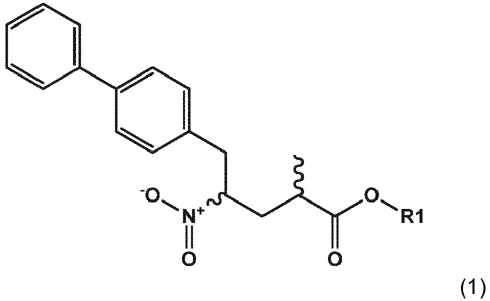 wherein R₁ is hydrogen, or C₁-C₆-alkyl,
said process comprising oxidizing a compound of the formula (2) or a salt thereof 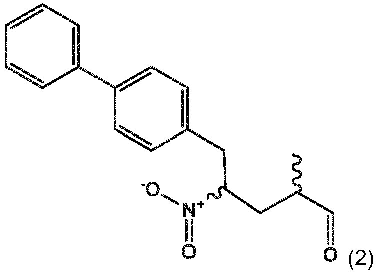 and optionally comprising the step of simultaneously or separately esterifiying the obtained compound of formula (1), or a salt thereof, wherein R1 is hydrogen, with a C₁-C₆-aliphatic alcohol, to yield the compound of formula (1) wherein R1 is C₁-C₆-alkyl.

5. A process for the manufacture of a compound of formula (2), 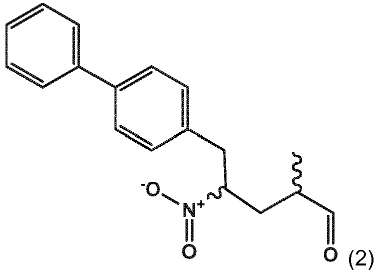 comprising
(i) reacting a compound of the formula (3) 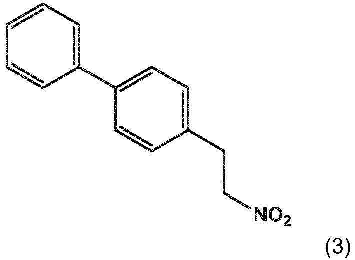 with methacroleine or a reactive derivative thereof in the presence of an organocatalyst for a Michael reaction, or
(ii) reacting of a compound of the formula (7), 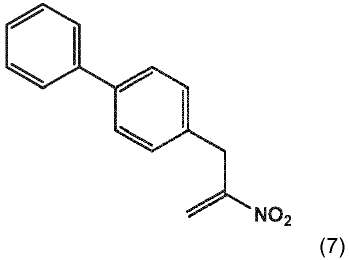 with propionaldehyde or a reactive derivative thereof in the presence of an organocatalyst for Michael reaction.

6. A process according to claim 5, wherein the organocatalyst is a chiral prolinol or a chiral thiourea organocatalyst.

7. A process for the manufacture of a compound of the formula (7), 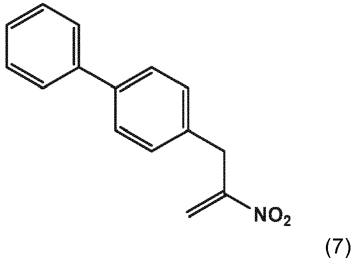 comprising reacting a compound of the formula (3) 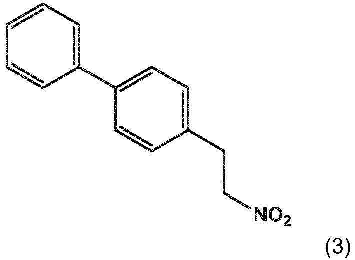 with formaldehyde or a reactive derivative thereof.

8. A process according to claim 5 or 6, wherein the compound of formula (7) is manufactured according to the process of claim 7.

9. A process according to any one of claims 5 to 8, wherein the compound of the formula (3) 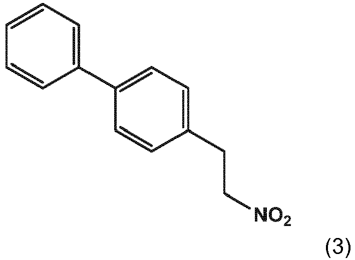 is obtained by a process comprising
(a) reacting an aldehyde compound of the formula (6), 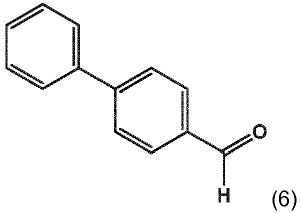 or a reactive derivative thereof, with nitromethane, to obtain a compound of formula (5), formula (4) or both 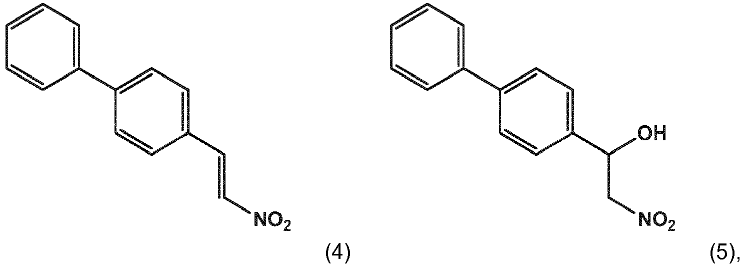
(b) reacting the obtained compound of the formula (5) with a dehydrating agent, preferably an acid anhydride, to obtain the compound of formula (4) 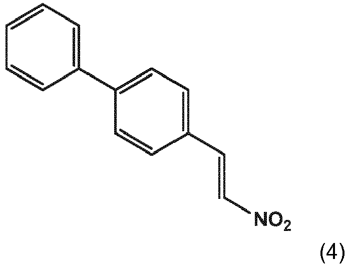 and
(c) hydrogenating a compound of the formula (4) and/or a compound of the formula (5) preferably in the presence of a complex hydride capable of selectively reducing the double bond.

10. A process for the manufacture of a compound of formula (1) according to claim 4, wherein the starting compound of formula (2) is obtained by a process according to any one of claims 5 to 6, 8 or 9.

11. A process for the manufacture of a compound of the formula (8), 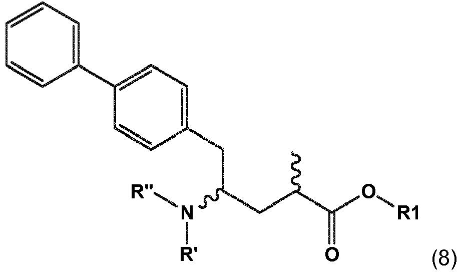 wherein R' and R" are independently of each other hydrogen or a nitrogen protecting group, and R1 is hydrogen or C₁-C₆-alkyl, comprising hydrogenating a compound of the formula (1), or a salt thereof, 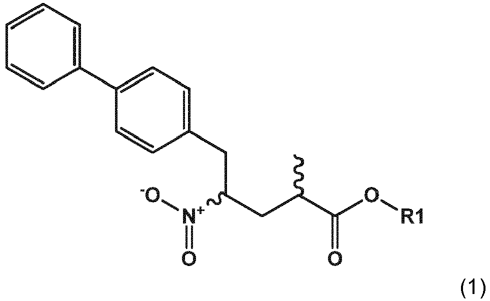 wherein R1 is hydrogen or C₁-C₆-alkyl,
optionally including or followed by the introduction of a nitrogen protecting group.

12. A process according to claim 11, wherein the compound of formula (1), preferably of formula (1-a) is obtained by a process according to claim 4 or 10.

13. A process according to claim 11 or 12, wherein the obtained compound of formula (8) is further reacted to a compound of formula (10) 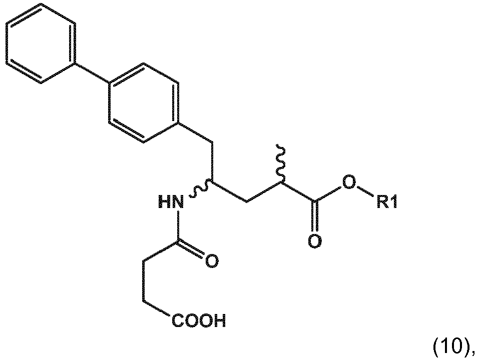 wherein R1 is hydrogen or C₁-C₆-alkyl, by reaction with succinic acid or a derivative thereof.

14. An organocatalyst selected from the group consisting of those with the following formulae:

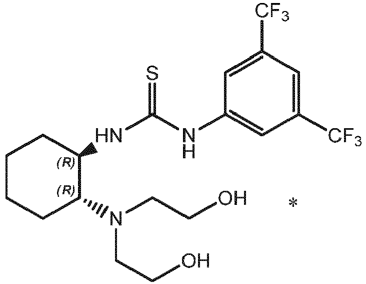

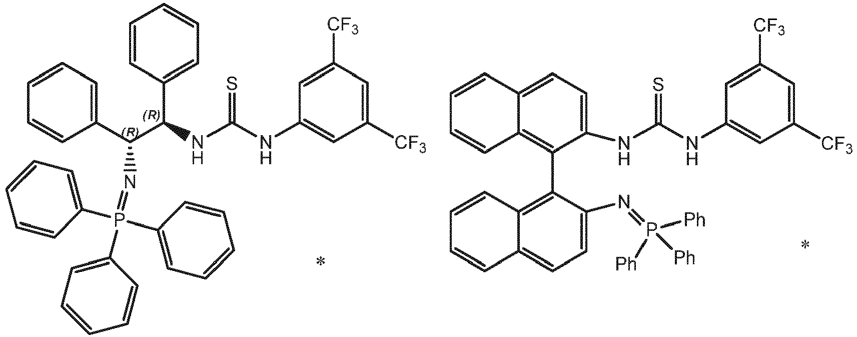

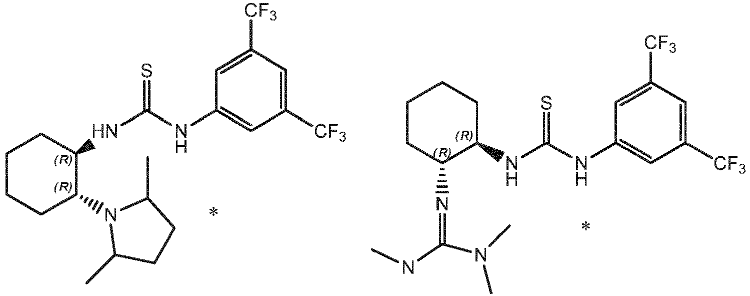

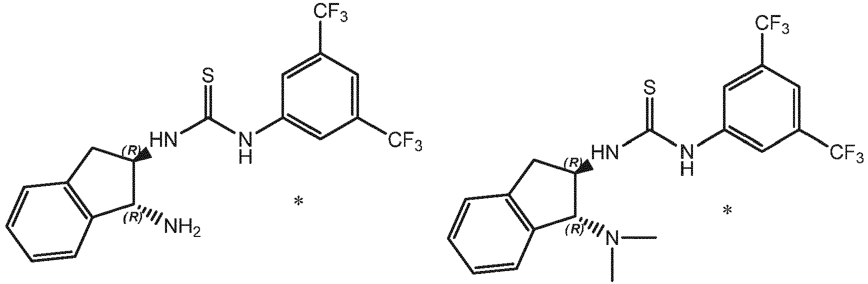

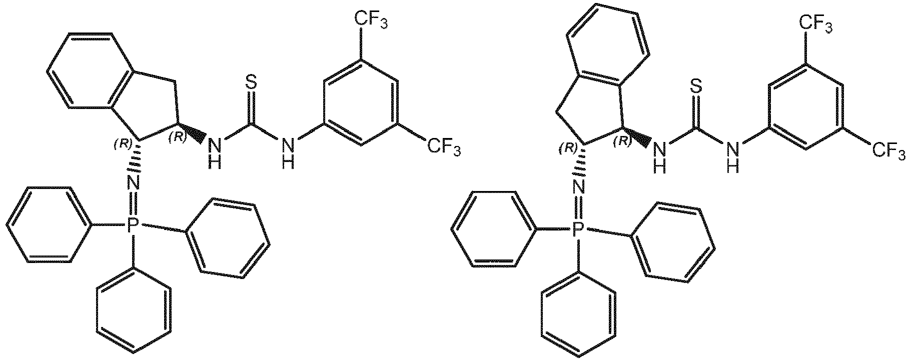

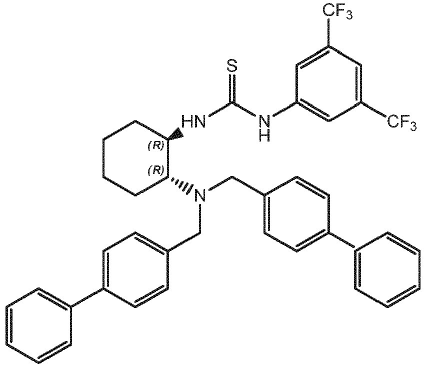

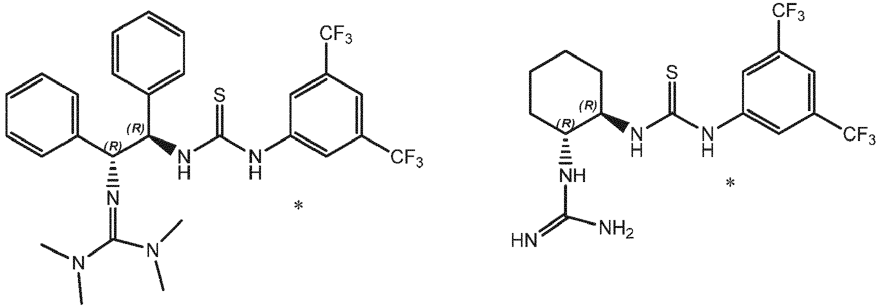

15. Use of a compound of any one of claims 1 to 3 in the manufacture of a compound of formula (10) 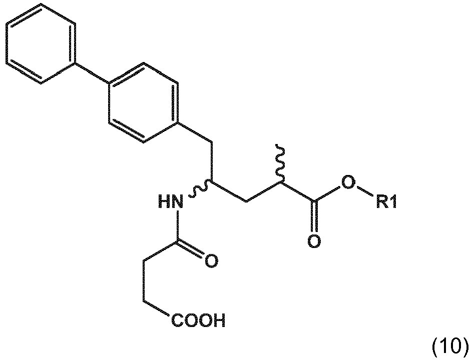 wherein R1 is hydrogen or C₁-C₆-alkyl.

## Patentansprüche

1. Verbindung der Formel (1) oder ein Salz davon 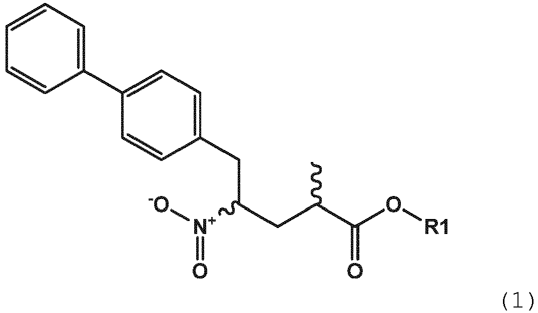 wobei R1 für Wasserstoff oder C₁-C₆-Alkyl steht.

2. Verbindung der Formel (2) oder ein Salz davon,

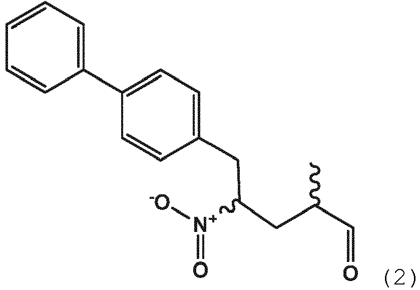

3. Verbindung der Formel (7),

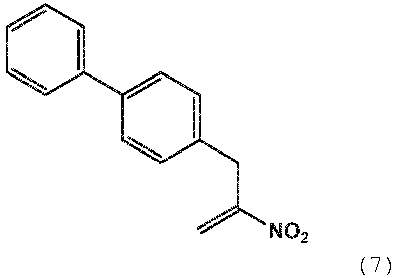

4. Verfahren zur Herstellung der Verbindung der Formel (1) oder eines Salzes davon, 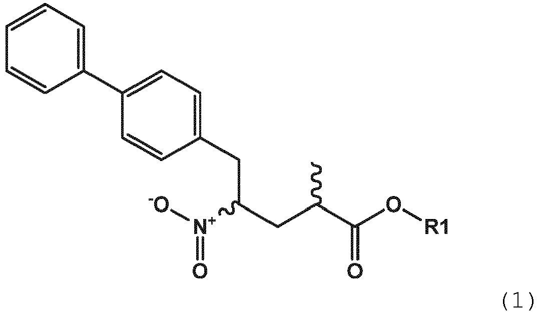 wobei R1 für Wasserstoff oder C₁-C₆-Alkyl steht,
bei dem man eine Verbindung der Formel (2) oder ein Salz davon 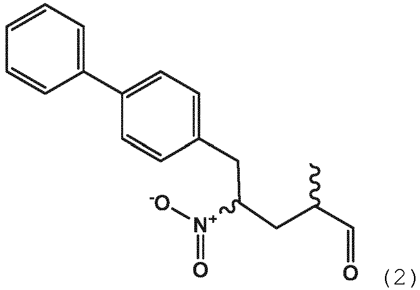 oxidiert und gegebenenfalls die erhaltene Verbindung der Formel (1) oder ein Salz davon, wobei R1 für Wasserstoff steht, gleichzeitig oder separat mit einem C₁-C₆-aliphatischen Alkohol zu einer Verbindung der Formel (1), wobei R1 für C₁-C₆-Alkyl steht, verestert.

5. Verfahren zur Herstellung einer Verbindung der Formel (2), 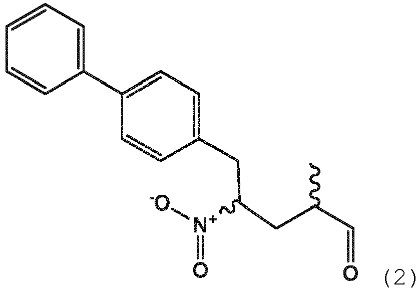 bei dem man
(i) eine Verbindung der Formel (3) 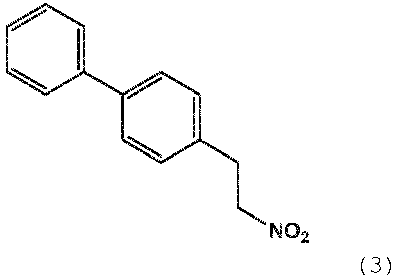 in Gegenwart eines Organokatalysators für eine Michael-Reaktion mit Methacrolein oder einem reaktiven Derivat davon umsetzt oder
(ii) eine Verbindung der Formel (7) 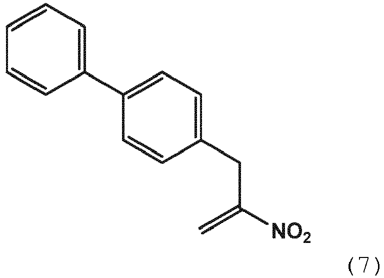 in Gegenwart eines Organokatalysators für eine Michael-Reaktion mit Propionaldehyd oder einem reaktiven Derivat davon umsetzt.

6. Verfahren nach Anspruch 5, bei dem es sich bei dem Organokatalysator um einen chiralen Prolinol- oder einen chiralen Thioharnstoff-Organokatalysator handelt.

7. Verfahren zur Herstellung einer Verbindung der Formel (7), 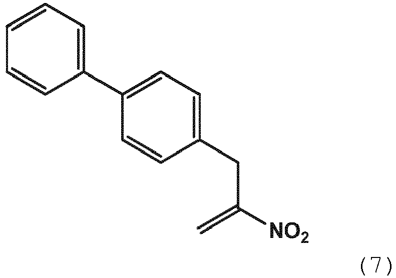 bei dem man eine Verbindung der Formel (3) 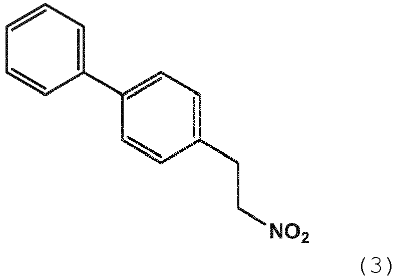 mit Formaldehyd oder einem reaktiven Derivat davon umsetzt.

8. Verfahren nach Anspruch 5 oder 6, bei dem die Verbindung der Formel (7) gemäß dem Verfahren von Anspruch 7 hergestellt wird.

9. Verfahren nach einem der Ansprüche 5 bis 8, bei dem die Verbindung der Formel (3) 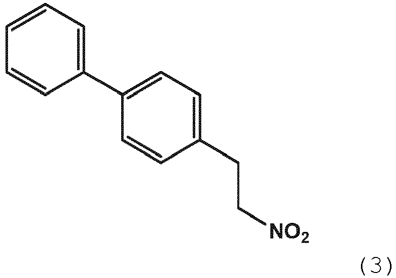 durch ein Verfahren erhalten wird, bei dem man
(a) eine Aldehydverbindung der Formel (6), 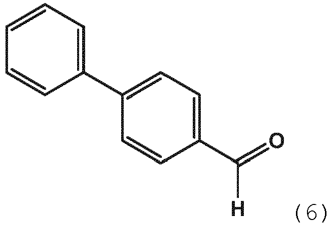 oder ein reaktives Derivat davon mit Nitromethan zu einer Verbindung der Formel (5) und/oder Formel (4) 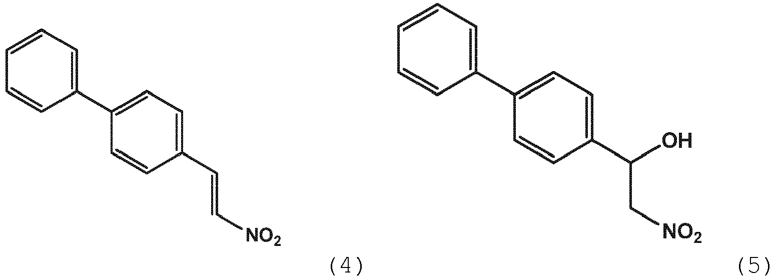 umsetzt,
(b) die erhaltene Verbindung der Formel (5) mit einem Dehydratisierungsmittel, vorzugsweise einem Säureanhydrid, zu der Verbindung der Formel (4) 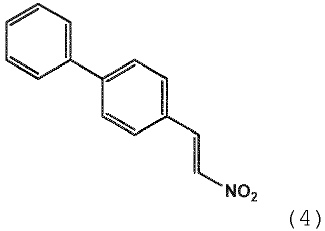 umsetzt und
(c) eine Verbindung der Formel (4) und/oder eine Verbindung der Formel (5) hydriert, vorzugsweise in Gegenwart eines komplexen Hydrids, das zur selektiven Reduktion der Doppelbindung befähigt ist.

10. Verfahren zur Herstellung einer Verbindung der Formel (1) nach Anspruch 4, bei dem die Ausgangsverbindung der Formel (2) durch ein Verfahren nach einem der Ansprüche 5 bis 6, 8 oder 9 erhalten wird.

11. Verfahren zur Herstellung einer Verbindung der Formel (8), 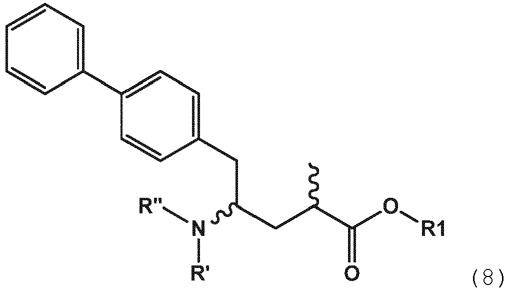 wobei R' und R" unabhängig voneinander für Wasserstoff oder eine Stickstoff-Schutzgruppe stehen und R1 für Wasserstoff oder C₁-C₆-Alkyl steht,
bei dem man eine Verbindung der Formel (1) oder ein Salz davon, 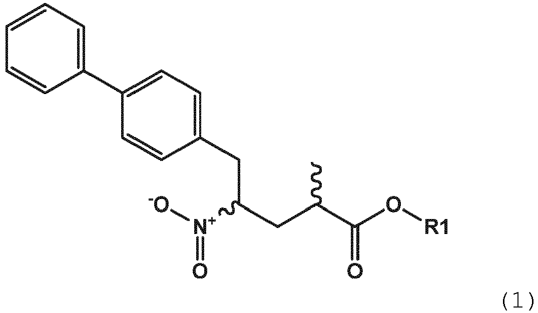 wobei R1 für Wasserstoff oder C₁-C₆-Alkyl steht, hydriert, gegebenenfalls mit oder gefolgt von der Einführung einer Stickstoff-Schutzgruppe.

12. Verfahren nach Anspruch 11, bei dem die Verbindung der Formel (1), vorzugsweise Formel (1-a), durch ein Verfahren nach Anspruch 4 oder 10 erhalten wird.

13. Verfahren nach Anspruch 11 oder 12, bei dem die erhaltene Verbindung der Formel (8) durch Umsetzung mit Bernsteinsäure oder einem Derivat davon weiter zu einer Verbindung der Formel (10) 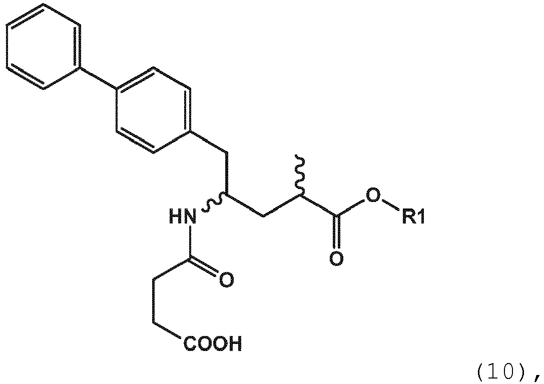 wobei R1 für Wasserstoff oder C₁-C₆-Alkyl steht, umgesetzt wird.

14. Organokatalysator, ausgewählt aus der Gruppe bestehend aus denjenigen mit den folgenden Formeln:

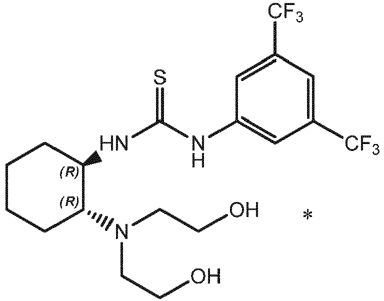

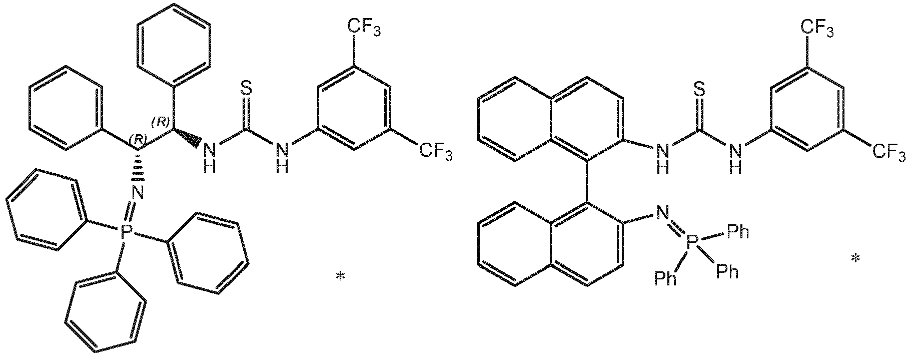

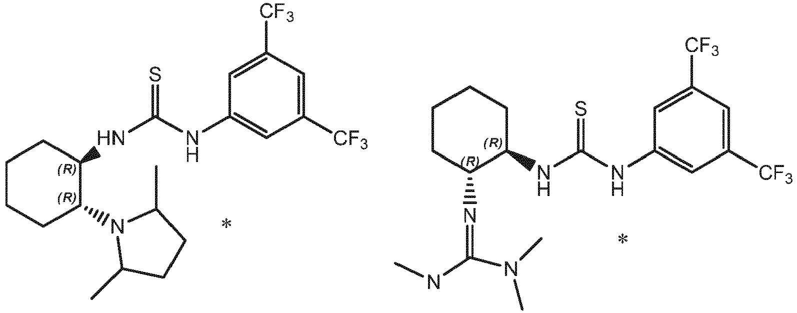

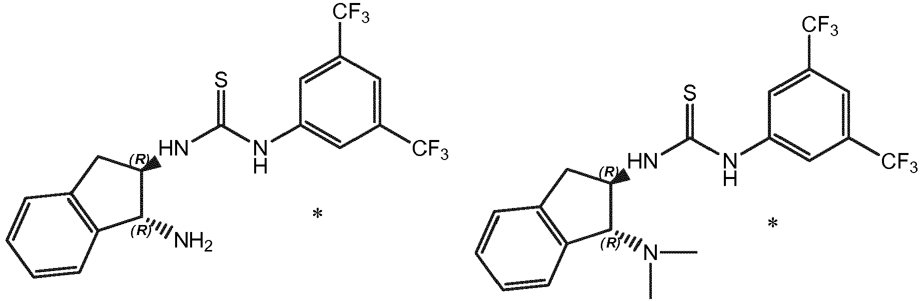

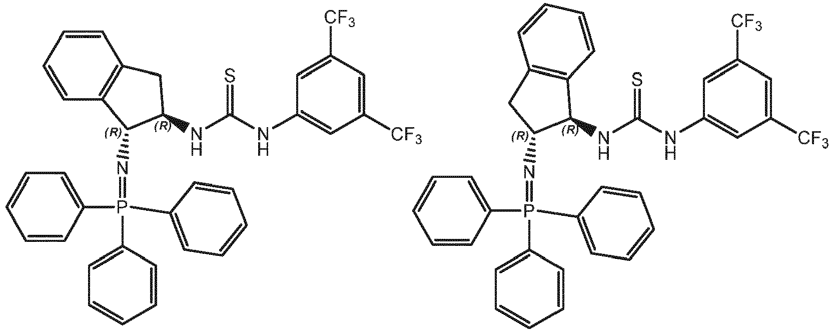

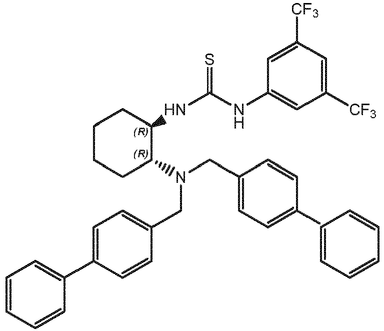

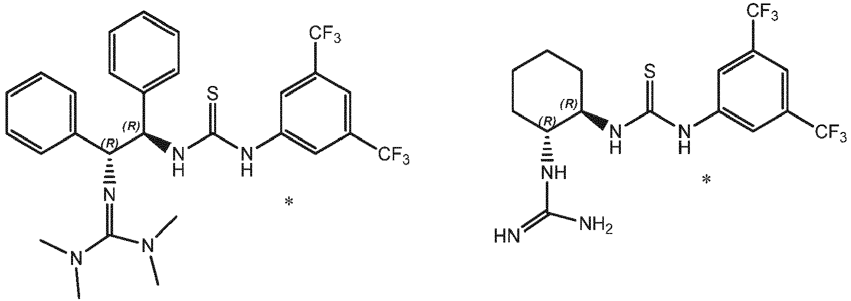

15. Verwendung einer Verbindung nach einem der Ansprüche 1 bis 3 bei der Herstellung einer Verbindung der Formel (10) 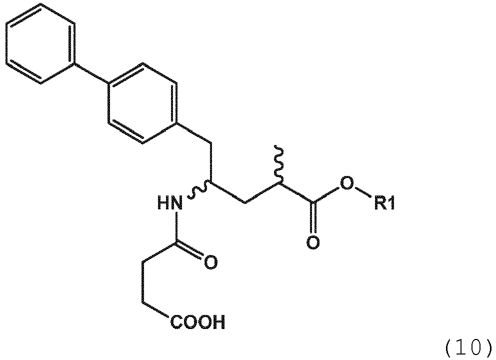 wobei R1 für Wasserstoff oder C₁-C₆-Alkyl steht.

## Revendications

1. Composé de formule (1), ou sel de celui-ci 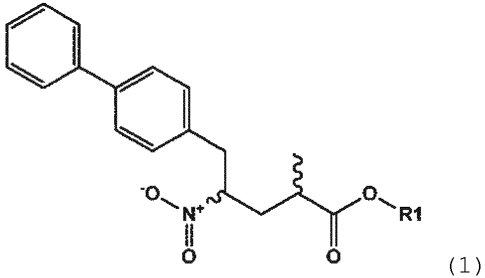 dans lequel R1 est hydrogène ou alkyle en C₁-C₆.

2. Composé de formule (2) ou sel de celui-ci,

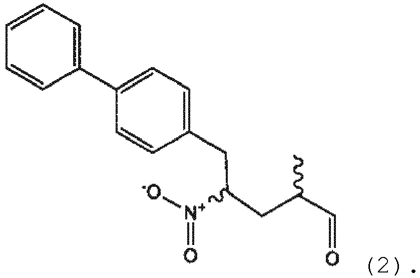

3. Composé de formule (7),

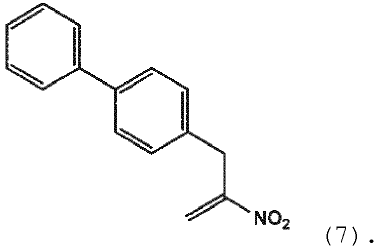

4. Procédé de fabrication du composé de formule (1), ou d'un sel de celui-ci, 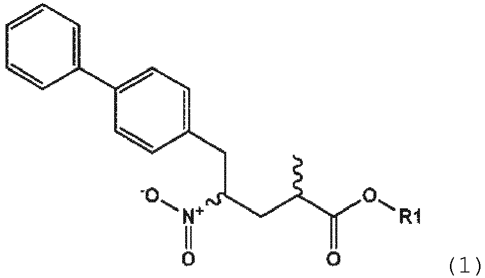 dans lequel R₁ est hydrogène ou alkyle en C₁-C₆,
ledit procédé comprenant l'oxydation d'un composé de formule (2) ou d'un sel de celui-ci 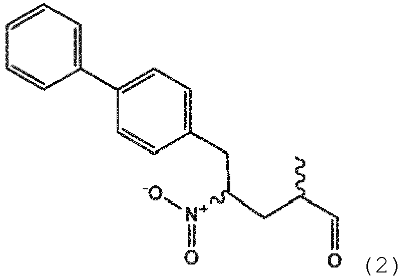 et comprenant facultativement l'étape d'estérification simultanément ou séparément du composé de formule (1) obtenu, ou d'un sel de celui-ci, dans lequel R1 est hydrogène, avec un alcool aliphatique en C₁-C₆, pour obtenir le composé de formule (1) dans lequel R1 est alkyle en C₁-C₆.

5. Procédé de fabrication d'un composé de formule (2), 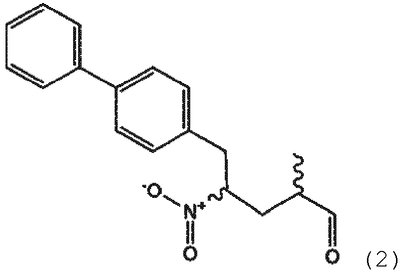 comprenant
(i) la réaction d'un composé de formule (3) 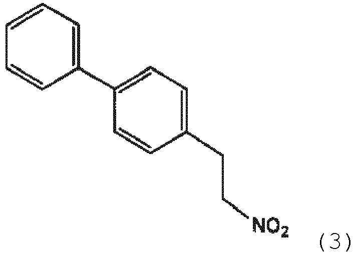 avec de la méthacroléine ou un dérivé réactif de celle-ci en présence d'un organocatalyseur pour une réaction de Michael, ou
(ii) la réaction d'un composé de formule (7), 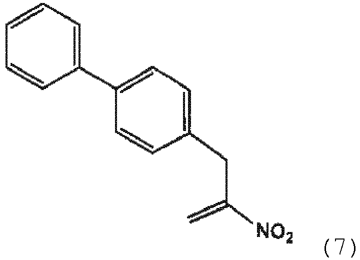 avec du propionaldéhyde ou un dérivé réactif de celui-ci en présence d'un organocatalyseur pour une réaction de Michael.

6. Procédé selon la revendication 5, dans lequel l'organocatalyseur est un organocatalyseur de prolinol chiral ou de thiourée chirale.

7. Procédé de fabrication d'un composé de formule (7), 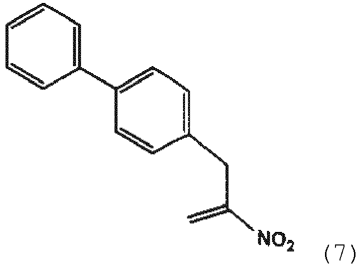 comprenant la réaction d'un composé de formule (3) 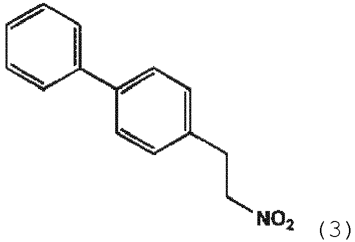 avec du formaldéhyde ou un dérivé réactif de celui-ci.

8. Procédé selon la revendication 5 ou 6, dans lequel le composé de formule (7) est fabriqué selon le procédé de la revendication 7.

9. Procédé selon l'une quelconque des revendications 5 à 8, dans lequel le composé de formule (3) 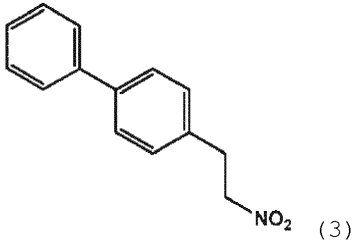 est obtenu par un procédé comprenant
(a) la réaction d'un composé aldéhyde de formule (6), 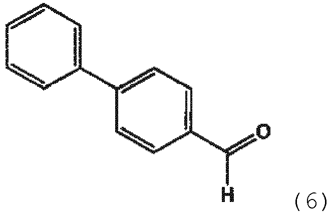 ou un dérivé réactif de celui-ci, avec du nitrométhane, pour obtenir un composé de formule (5), formule (4) ou les deux 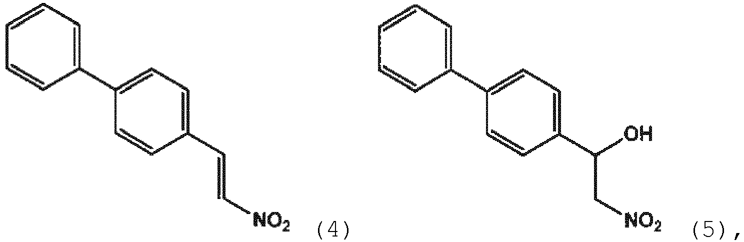
(b) la réaction du composé de formule (5) obtenu avec un agent déshydratant, de préférence un anhydride d'acide, pour obtenir le composé de formule (4) 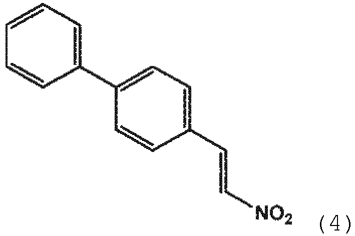 et
(c) l'hydrogénation d'un composé de formule (4) et/ou d'un composé de formule (5), de préférence en présence d'un hydrure complexe capable de réduire sélectivement la double liaison.

10. Procédé de fabrication d'un composé de formule (1) selon la revendication 4, dans lequel le composé de départ de formule (2) est obtenu par un procédé selon l'une quelconque des revendications 5 et 6, 8 ou 9.

11. Procédé de fabrication d'un composé de formule (8), 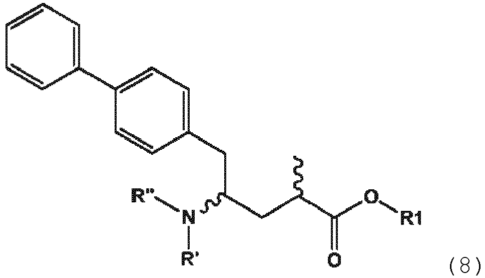 dans lequel R' et R" sont, indépendamment l'un de l'autre, hydrogène ou un groupe protecteur d'azote, et R1 est hydrogène ou alkyle en C₁-C₆,
comprenant l'hydrogénation d'un composé de formule (1), ou d'un sel de celui-ci, 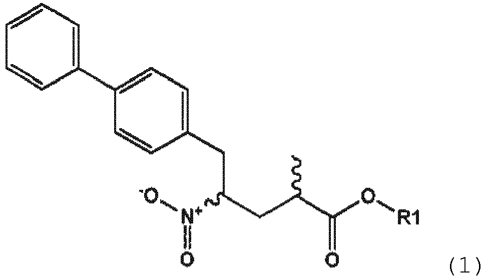 dans lequel R1 est hydrogène ou alkyle en C₁-C₆, comprenant facultativement ou suivi de l'introduction d'un groupe protecteur d'azote.

12. Procédé selon la revendication 11, dans lequel le composé de formule (1), de préférence de formule (1-a) est obtenu par un procédé selon la revendication 4 ou 10.

13. Procédé selon la revendication 11 ou 12, dans lequel le composé de formule (8) obtenu est mis à réagir en outre pour obtenir un composé de formule (10) 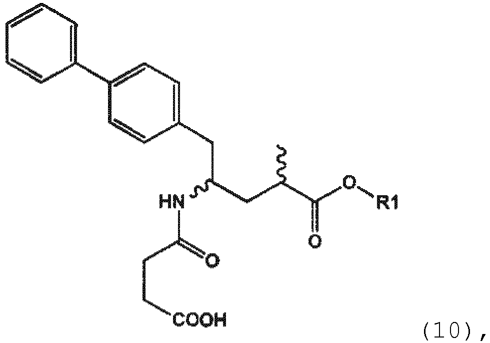 dans lequel R1 est hydrogène ou alkyle en C₁-C₆, par réaction avec l'acide succinique ou un dérivé de celui-ci.

14. Organocatalyseur choisi dans le groupe constitué de ceux ayant les formules suivantes :

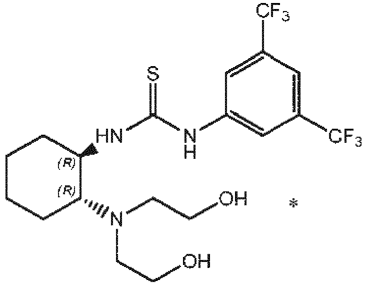

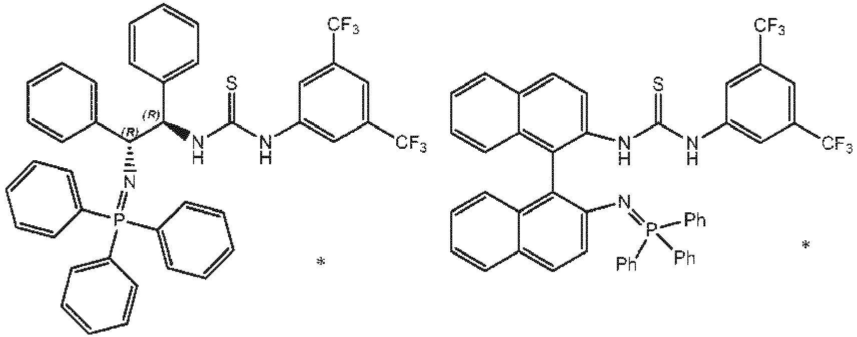

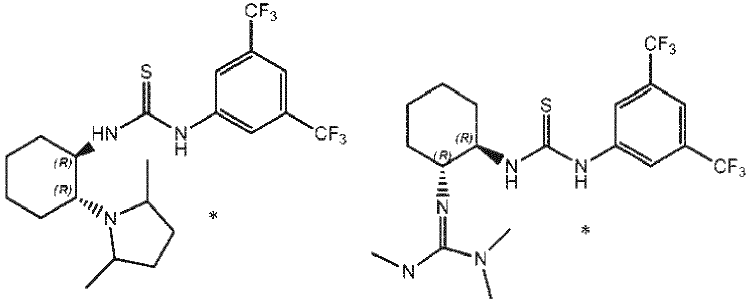

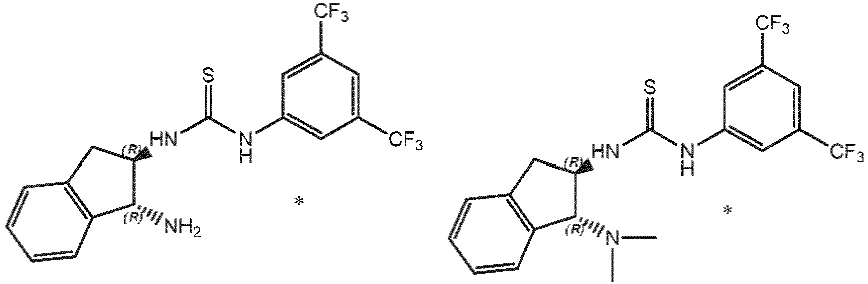

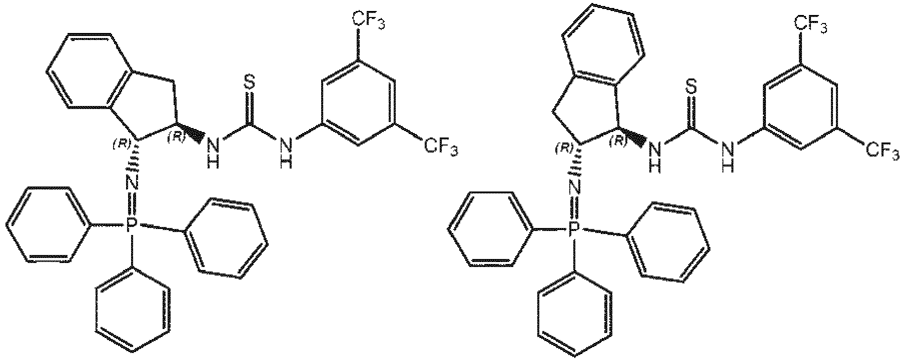

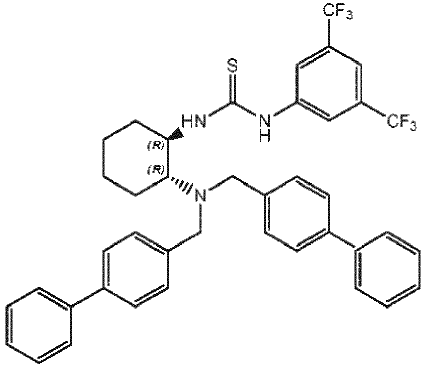

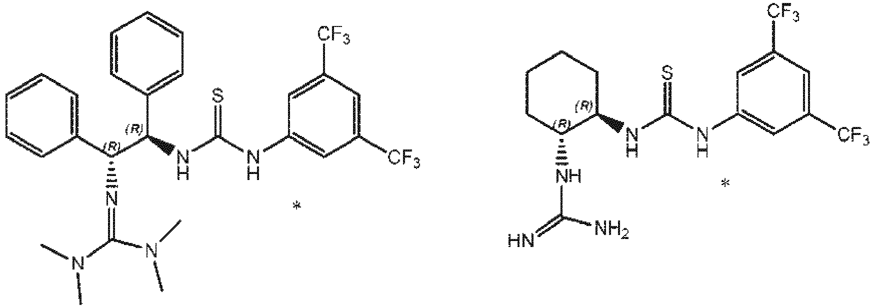

15. Utilisation d'un composé selon l'une quelconque des revendications 1 à 3 dans la fabrication d'un composé de formule (10) 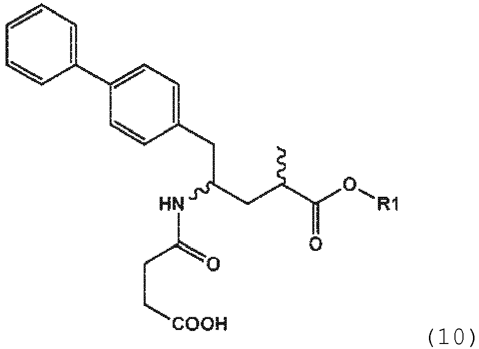 dans lequel R1 est hydrogène ou alkyle en C₁-C₆.
